# EUROPÄISCHE PATENTSCHRIFT

(11) **EP 2 386 576 B1**
(45) Veröffentlichungstag und Bekanntmachung des Hinweises auf die Patenterteilung: **13.08.2014**
(21) Anmeldenummer: 10012760.4
(22) Anmeldetag: 01.12.2003
(51) Int. Cl.: C07K 16/30, C12N 5/00, A61K 39/00, A61K 47/48, A61K 51/10

(54) **Tumorspezifische Erkennungsmoleküle**
Tumor-specific recognition molecules
Molécule de reconnaissance spécifique à la tumeur

(30) Priorität: 29.11.2002 DE 10256900
(43) Veröffentlichungstag der Anmeldung: 16.11.2011
(62) Teilanmeldung aus: 08158547.3
(73) Patentinhaber: Glycotope GmbH, 13125 Berlin (DE)
(72) Erfinder: Goletz, Steffen, 13125 Berlin (DE); Danielczyk, Antje, 16341 Panketal (DE); Stahn, Renate, 13125 Berlin (DE); Karsten, Uwe, 16341 Panketal (DE); Ravn, Peter, Cambridge CB1 3AE (GB); Astrup Christensen, Peter, 8920 Randers NV (DK)
(74) Vertreter: Roth, Carla

(56) Entgegenhaltungen:
- DE-A1- 4 329 004
- KARSTEN UWE ET AL: "A New Monoclonal Antibody (A78-G/A7) to the Thomsen-Friedenreich Pan-Tumor Antigen", HYBRIDOMA, Bd. 14, Nr. 1, 1995, Seiten 37-44, XP009034408, ISSN: 0272-457X
- PRICE M R ET AL: "SUMMARY REPORT ON THE ISOBM TD-4 WORKSHOP: ANALYSIS OF 56 MONOCLONAL ANTIBODIES AGAINST THE MUC1 MUCIN", TUMOR BIOLOGY, KARGER, BASEL, CH, Bd. 19, Nr. SUPPL 1, 1998, Seiten 1-20, XP002071245, ISSN: 1010-4283
- JESCHKE U ET AL: "Expression of the Thomsen-Friedenreich antigen and of its putative carrier protein mucin 1 in the human placenta and in trophoblast cells in vitro", HISTOCHEMISTRY AND CELL BIOLOGY, Bd. 117, Nr. 3, März 2002 (2002-03), Seiten 219-226, XP002290192, ISSN: 0948-6143
- SCHNEIDER F ET AL: "Overexpression of sialyltransferase CMP-sialic acid:Galbeta1,3GalNAc-R alpha6-Sialyltransferase is related to poor patient survival in human colorectal carcinomas", CANCER RESEARCH, AMERICAN ASSOCIATION FOR CANCER RESEARCH, BALTIMORE, MD, US, Bd. 61, Nr. 11, 1. Juni 2001 (2001-06-01), Seiten 4605-4611, XP002232470, ISSN: 0008-5472
- CIECHANOVER A ET AL: "The asialoglycoprotein receptor internalizes and recycles independently of the transferrin and insulin receptors", CELL, CELL PRESS, US, Bd. 32, Nr. 1, 1. Januar 1983 (1983-01-01) , Seiten 267-275, XP027462690, ISSN: 0092-8674, DOI: 10.1016/0092-8674(83)90517-2 [gefunden am 1983-01-01]

### SEQUENZPROTOKOLL

<110> Nemod Biotherapeutics GmbH & Co. KG
<120> Tumorspezifische Erkennungsmoleküle
<130> P1778 EP/1 S3
<140> Teilanmeldung basierend auf EP 03 78 8853.4
   <141> 2003-12-01
<150> DE 102 56 900.2
   <151> 2002-11-29
<160> 113
<170> PatentIn Ver. 2.1
<210> 1
   <211> 5
   <212> PRT
   <213> Künstliche Sequenz
<220>
   <223> Beschreibung der künstlichen Sequenz: CDR-Sequenz
<400> 1
   Asn Tyr Trp Leu Gly
    1               5
<210> 2
   <211> 17
   <212> PRT
   <213> Künstliche Sequenz
<220>
   <223> Beschreibung der künstlichen Sequenz: CDR-Sequenz
<400> 2
   Asp Ile Tyr Pro Gly Gly Gly Tyr Thr Asn Tyr Asn Glu Lys Phe Lys
    1               5                  10                  15
   Gly
<210> 3
   <211> 17
   <212> PRT
   <213> Künstliche Sequenz
<220>
   <223> Beschreibung der künstlichen Sequenz: CDR-Sequenz
<400> 3
   Asp Ile Tyr Pro Gly Gly Ser Tyr Thr Asn Tyr Asn Glu Lys Phe Lys
    1               5                  10                  15
   Gly
<210> 4
   <211> 10
   <212> PRT
   <213> Künstliche Sequenz
<220>
   <223> Beschreibung der künstlichen Sequenz: CDR-Sequenz
<400> 4
   Tyr Asp Ala Ala Gly Pro Trp Phe Ala Tyr
   1               5                   10
<210> 5
   <211> 10
   <212> PRT
   <213> Künstliche Sequenz
<220>
   <223> Beschreibung der künstlichen Sequenz: CDR-Sequenz
<400> 5
   Tyr Asp Ala Ala Gly Pro Gly Phe Ala Tyr
   1               5                   10
<210> 6
   <211> 8
   <212> PRT
   <213> Künstliche Sequenz
<220>
   <223> Beschreibung der künstlichen Sequenz: CDR-Sequenz
<400> 6
   Tyr Asp Asn His Tyr Phe Asp Tyr
   1               5
<210> 7
   <211> 16
   <212> PRT
   <213> Künstliche Sequenz
<220>
   <223> Beschreibung der künstlichen Sequenz: CDR-Sequenz
<400> 7
   Arg Ser Ser Gln Ser Ile Val His Ser Asn Gly Asn Thr Tyr Leu Glu
   1               5                   10                  15
<210> 8
   <211> 16
   <212> PRT
   <213> Künstliche Sequenz
<220>
   <223> Beschreibung der künstlichen Sequenz: CDR-Sequenz
<400> 8
   Arg Ser Ser Gln Ser Leu Leu His Ser Asn Gly Asn Thr Tyr Leu His
   1 5 10 15
<210> 9
   <211> 16
   <212> PRT
   <213> Künstliche Sequenz
<220>
   <223> Beschreibung der künstlichen Sequenz: CDR-Sequenz
<400> 9
   Lys Ser Ser Gln Ser Leu Leu His Ser Asp Gly Lys Thr Tyr Leu Tyr
   1 5 10 15
<210> 10
   <211> 7
   <212> PRT
   <213> Künstliche Sequenz
<220>
   <223> Beschreibung der künstlichen Sequenz: CDR-Sequenz
<400> 10
   Lys Val Ser Asn Arg Phe Ser
   1 5
<210> 11
   <211> 7
   <212> PRT
   <213> Künstliche Sequenz
<220>
   <223> Beschreibung der künstlichen Sequenz: CDR-Sequenz
<400> 11
   Glu Val Ser Ser Arg Phe Ser
   1 5
<210> 12
   <211> 9
   <212> PRT
   <213> Künstliche Sequenz
<220>
   <223> Beschreibung der künstlichen Sequenz: CDR-Sequenz
<400> 12
   Phe Gln Gly Ser His Val Pro Tyr Thr
   1 5
<210> 13
   <211> 9
   <212> PRT
   <213> Künstliche Sequenz
<220>
   <223> Beschreibung der künstlichen Sequenz: CDR-Sequenz
<400> 13
   Ser Gln Ser Thr His Val Pro Tyr Thr
   1 5
<210> 14
   <211> 5
   <212> PRT
   <213> Künstliche Sequenz
<220>
   <223> Beschreibung der künstlichen Sequenz: CDR-Sequenz (canonical structure variant)
<400> 14
   Asn Tyr Trp Ile Gly
   1 5
<210> 15
   <211> 5
   <212> PRT
   <213> Künstliche Sequenz
<220>
   <223> Beschreibung der künstlichen Sequenz: CDR-Sequenz (canonical structure variant)
<400> 15
   Asn Tyr Trp Met Gly
   1 5
<210> 16
   <211> 5
   <212> PRT
   <213> Künstliche Sequenz
<220>
   <223> Beschreibung der künstlichen Sequenz: CDR-Sequenz (canonical structure variant)
<400> 16
   Asn Tyr Trp Trp Gly
   1 5
<210> 17
   <211> 5
   <212> PRT
   <213> Künstliche Sequenz
<220>
   <223> Beschreibung der künstlichen Sequenz: CDR-Sequenz (canonical structure variant)
<400> 17
   Asn Tyr Trp Val Gly
   1 5
<210> 18
   <211> 17
   <212> PRT
   <213> Künstliche Sequenz
<220>
   <223> Beschreibung der künstlichen Sequenz: CDR-Sequenz (canonical structure variant)
<400> 18
   Asp Ile Tyr Pro Gly Gly Asp Tyr Thr Asn Tyr Asn Glu Lys Phe Lys
   1 5 10 15
   Gly
<210> 19
   <211> 17
   <212> PRT
   <213> Künstliche Sequenz
<220>
   <223> Beschreibung der künstlichen Sequenz: CDR-Sequenz (canonical structure variant)
<400> 19
   Asp Ile Tyr Pro Gly Gly Asn Tyr Thr Asn Tyr Asn Glu Lys Phe Lys
   1 5 10 15
   Gly
<210> 20
   <211> 17
   <212> PRT
   <213> Künstliche Sequenz
<220>
   <223> Beschreibung der künstlichen Sequenz: CDR-Sequenz (canonical structure variant)
<400> 20
   Asp Ile Tyr Thr Gly Gly Gly Tyr Thr Asn Tyr Asn Glu Lys Phe Lys
   1 5 10 15
   Gly
<210> 21
   <211> 17
   <212> PRT
   <213> Künstliche Sequenz
<220>
   <223> Beschreibung der künstlichen Sequenz: CDR-Sequenz (canonical structure variant)
<400> 21
   Asp Ile Tyr Thr Gly Gly Asp Tyr Thr Asn Tyr Asn Glu Lys Phe Lys
   1               5                   10                  15
   Gly
<210> 22
   <211> 17
   <212> PRT
   <213> Künstliche Sequenz
<220>
   <223> Beschreibung der künstlichen Sequenz: CDR-Sequenz (canonical structure variant)
<400> 22
   Asp Ile Tyr Thr Gly Gly Asn Tyr Thr Asn Tyr Asn Glu Lys Phe Lys
   1               5                   10                  15
   Gly
<210> 23
   <211> 17
   <212> PRT
   <213> Künstliche Sequenz
<220>
   <223> Beschreibung der künstlichen Sequenz: CDR-Sequenz (canonical structure variant)
<400> 23
   Asp Ile Tyr Thr Gly Gly Ser Tyr Thr Asn Tyr Asn Glu Lys Phe Lys
   1               5                   10                  15
   Gly
<210> 24
   <211> 17
   <212> PRT
   <213> Künstliche Sequenz
<220>
   <223> Beschreibung der künstlichen Sequenz: CDR-Sequenz (canonical structure variant)
<400> 24
   Asp Ile Tyr Ala Gly Gly Gly Tyr Thr Asn Tyr Asn Glu Lys Phe Lys
   1               5                   10                  15
   Gly
<210> 25
   <211> 17
   <212> PRT
   <213> Künstliche Sequenz
<220>
   <223> Beschreibung der künstlichen Sequenz: CDR-Sequenz (canonical structure variant)
<400> 25
   Asp Ile Tyr Ala Gly Gly Asp Tyr Thr Asn Tyr Asn Glu Lys Phe Lys
   1               5                   10                  15
   Gly
<210> 26
   <211> 17
   <212> PRT
   <213> Künstliche Sequenz
<220>
   <223> Beschreibung der künstlichen Sequenz: CDR-Sequenz (canonical structure variant)
<400> 26
   Asp Ile Tyr Ala Gly Gly Asp Tyr Thr Asn Tyr Asn Glu Lys Phe Lys
   1               5                   10                  15
   Gly
<210> 27
   <211> 17
   <212> PRT
   <213> Künstliche Sequenz
<220>
   <223> Beschreibung der künstlichen Sequenz: CDR-Sequenz (canonical structure variant)
<400> 27
   Asp Ile Tyr Ala Gly Gly Ser Tyr Thr Asn Tyr Asn Glu Lys Phe Lys
   1               5                   10                  15
   Gly
<210> 28
   <211> 16
   <212> PRT
   <213> Künstliche Sequenz
<220>
   <223> Beschreibung der künstlichen Sequenz: CDR-Sequenz (canonical structure variant)
<400> 28
   Arg Pro Ser Gln Ser Ile Val His Ser Asn Gly Asn Thr Tyr Leu Glu
   1               5                   10                  15
<210> 29
   <211> 16
   <212> PRT
   <213> Künstliche Sequenz
<220>
   <223> Beschreibung der künstlichen Sequenz: CDR-Sequenz (canonical structure variant)
<400> 29
   Arg Pro Ser Gln Ser Ile Val His Ser Asn Gly Asn Thr Tyr Leu Glu
     1               5                  10                  15
<210> 30
   <211> 16
   <212> PRT
   <213> Künstliche Sequenz
<220>
   <223> Beschreibung der künstlichen Sequenz: CDR-Sequenz (canonical structure variant)
<400> 30
   Arg Ser Ser Gln Ser Ile Val His Ser Asn Gly Asn Thr Tyr Phe Glu
     1               5                  10                  15
<210> 31
   <211> 16
   <212> PRT
   <213> Künstliche Sequenz
<220>
   <223> Beschreibung der künstlichen Sequenz: CDR-Sequenz (canonical structure variant)
<400> 31
   Arg Pro Ser Gln Ser Leu Val His Ser Asn Gly Asn Thr Tyr Leu Glu
     1               5                  10                  15
<210> 32
   <211> 16
   <212> PRT
   <213> Künstliche Sequenz
<220>
   <223> Beschreibung der künstlichen Sequenz: CDR-Sequenz (canonical structure variant)
<400> 32
   Arg Pro Ser Gln Ser Ile Val His Ser Asn Gly Asn Thr Tyr Phe Glu
     1               5                  10                  15
<210> 33
   <211> 16
   <212> PRT
   <213> Künstliche Sequenz
<220>
   <223> Beschreibung der künstlichen Sequenz: CDR-Sequenz (canonical structure variant)
<400> 33
   Arg Ser Ser Gln Ser Leu Val His Ser Asn Gly Asn Thr Tyr Phe Glu
     1               5                  10                  15
<210> 34
   <211> 16
   <212> PRT
   <213> Künstliche Sequenz
<220>
   <223> Beschreibung der künstlichen Sequenz: CDR-Sequenz (canonical structure variant)
<400> 34
   Arg Pro Ser Gln Ser Leu Leu His Ser Asn Gly Asn Thr Tyr Leu His
   1               5                   10                  15
<210> 35
   <211> 16
   <212> PRT
   <213> Künstliche Sequenz
<220>
   <223> Beschreibung der künstlichen Sequenz: CDR-Sequenz (canonical structure variant)
<400> 35
   Arg Ser Ser Gln Ser Ile Leu His Ser Asn Gly Asn Thr Tyr Leu His
   1               5                   10                  15
<210> 36
   <211> 16
   <212> PRT
   <213> Künstliche Sequenz
<220>
   <223> Beschreibung der künstlichen Sequenz: CDR-Sequenz (canonical structure variant)
<400> 36
   Arg Ser Ser Gln Ser Leu Leu His Ser Asn Gly Asn Thr Tyr Phe His
   1               5                   10                  15
<210> 37
   <211> 16
   <212> PRT
   <213> Künstliche Sequenz
<220>
   <223> Beschreibung der künstlichen Sequenz: CDR-Sequenz (canonical structure variant)
<400> 37
   Arg Pro Ser Gln Ser Ile Leu His Ser Asn Gly Asn Thr Tyr Leu His
   1               5                   10                  15
<210> 38
   <211> 16
   <212> PRT
   <213> Künstliche Sequenz
<220>
   <223> Beschreibung der künstlichen Sequenz: CDR-Sequenz (canonical structure variant)
<400> 38
   Arg Pro Ser Gln Ser Leu Leu His Ser Asn Gly Asn Thr Tyr Phe His
    1               5                  10                  15
<210> 39
   <211> 16
   <212> PRT
   <213> Künstliche Sequenz
<220>
   <223> Beschreibung der künstlichen Sequenz: CDR-Sequenz (canonical structure variant)
<400> 39
   Arg Ser Ser Gln Ser Ile Leu His Ser Asn Gly Asn Thr Tyr Phe His
    1               5                  10                  15
<210> 40
   <211> 16
   <212> PRT
   <213> Künstliche Sequenz
<220>
   <223> Beschreibung der künstlichen Sequenz: CDR-Sequenz (canonical structure variant)
<400> 40
   Lys Pro Ser Gln Ser Leu Leu His Ser Asp Gly Lys Thr Tyr Leu Tyr
    1               5                  10                  15
<210> 41
   <211> 16
   <212> PRT
   <213> Künstliche Sequenz
<220>
   <223> Beschreibung der künstlichen Sequenz: CDR-Sequenz (canonical structure variant)
<400> 41
   Lys Ser Ser Gln Ser Ile Leu His Ser Asp Gly Lys Thr Tyr Leu Tyr
    1               5                  10                  15
<210> 42
   <211> 16
   <212> PRT
   <213> Künstliche Sequenz
<220>
   <223> Beschreibung der künstlichen Sequenz: CDR-Sequenz (canonical structure variant)
<400> 42
   Lys Ser Ser Gln Ser Leu Leu His Ser Asp Gly Lys Thr Tyr Phe Tyr
    1               5                  10                  15
<210> 43
   <211> 16
   <212> PRT
   <213> Künstliche Sequenz
<220>
   <223> Beschreibung der künstlichen Sequenz: CDR-Sequenz (canonical structure variant)
<400> 43
   Lys Pro Ser Gln Ser Ile Leu His Ser Asp Gly Lys Thr Tyr Leu Tyr
   1               5                   10                  15
<210> 44
   <211> 16
   <212> PRT
   <213> Künstliche Sequenz
<220>
   <223> Beschreibung der künstlichen Sequenz: CDR-Sequenz (canonical structure variant)
<400> 44
   Lys Pro Ser Gln Ser Ile Leu His Ser Asp Gly Lys Thr Tyr Leu Tyr
   1               5                   10                  15
<210> 45
   <211> 16
   <212> PRT
   <213> Künstliche Sequenz
<220>
   <223> Beschreibung der künstlichen Sequenz: CDR-Sequenz (canonical structure variant)
<400> 45
   Lys Ser Ser Gln Ser Ile Leu His Ser Asp Gly Lys Thr Tyr Phe Tyr
   1               5                   10                  15
<210> 46
   <211> 119
   <212> PRT
   <213> Künstliche Sequenz
<220>
   <223> Beschreibung der künstlichen Sequenz: variable schwere Ketten VH
<400> 46
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser
           115
<210> 47
   <211> 117
   <212> PRT
   <213> Künstliche Sequenz
<220>
   <223> Beschreibung der künstlichen Sequenz: variable schwere Ketten VH
<400> 47
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Ser Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Tyr Asp Asn His Tyr Phe Asp Tyr Trp Gly Gln Gly Thr Thr
               100                 105                 110
   Leu Thr Val Ser Ser
           115
<210> 48
   <211> 119
   <212> PRT
   <213> Künstliche Sequenz
<220>
   <223> Beschreibung der künstlichen Sequenz: variable schwere Ketten VH
<400> 48
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Leu Thr Val Ser Ser
           115
<210> 49
   <211> 119
   <212> PRT
   <213> Künstliche Sequenz
<220>
   <223> Beschreibung der künstlichen Sequenz: variable schwere Ketten VH
<400> 49
   Glu Val Lys Leu Val Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                      80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Ser Val Thr Val Ser Ser
           115
<210> 50
   <211> 119
   <212> PRT
   <213> Künstliche Sequenz
<220>
   <223> Beschreibung der künstlichen Sequenz: variable schwere Ketten VH
<400> 50
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                      80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 51
   <211> 119
   <212> PRT
   <213> Künstliche Sequenz
<220>
   <223> Beschreibung der künstlichen Sequenz: variable schwere Ketten VH
<400> 51
   Glu Val Lys Leu Val Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 52
   <211> 119
   <212> PRT
   <213> Künstliche Sequenz
<220>
   <223> Beschreibung der künstlichen Sequenz: variable schwere Ketten VH
<400> 52
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ala
           115
<210> 53
   <211> 119
   <212> PRT
   <213> Künstliche Sequenz
<220>
   <223> Beschreibung der künstlichen Sequenz: variable schwere Ketten VH
<400> 53
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
                   100                 105                 110
   Thr Thr Val Thr Val Ser Ser
           115
<210> 54
   <211> 119
   <212> PRT
   <213> Künstliche Sequenz
<220>
   <223> Beschreibung der künstlichen Sequenz: variable schwere Ketten VH
<400> 54
   Gln Val Thr Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Ser Val Thr Val Ser Ser
           115
<210> 55
   <211> 119
   <212> PRT
   <213> Künstliche Sequenz
<220>
   <223> Beschreibung der künstlichen Sequenz: variable schwere Ketten VH
<400> 55
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
    65                 70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Ser Val Thr Val Ser Ser
           115
<210> 56
   <211> 119
   <212> PRT
   <213> Künstliche Sequenz
<220>
   <223> Beschreibung der künstlichen Sequenz: variable schwere Ketten VH
<400> 56
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
    65                 70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 57
   <211> 119
   <212> PRT
   <213> Künstliche Sequenz
<220>
   <223> Beschreibung der künstlichen Sequenz: variable schwere Ketten VH
<400> 57
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
                20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                      80
   Met Gln Leu Ser Ser Leu Thr Ser Glu Asp Ser Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 58
   <211> 119
   <212> PRT
   <213> Künstliche Sequenz
<220>
   <223> Beschreibung der künstlichen Sequenz: variable schwere Ketten VH
<400> 58
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
                20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                      80
   Met Gln Leu Ser Arg Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 59
   <211> 119
   <212> PRT
   <213> Künstliche Sequenz
<220>
   <223> Beschreibung der künstlichen Sequenz: variable schwere Ketten VH
<400> 59
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Glu Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 60
   <211> 119
   <212> PRT
   <213> Künstliche Sequenz
<220>
   <223> Beschreibung der künstlichen Sequenz: variable schwere Ketten VH
<400> 60
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Arg Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 61
   <211> 119
   <212> PRT
   <213> Künstliche Sequenz
<220>
   <223> Beschreibung der künstlichen Sequenz: variable schwere Ketten VH
<400> 61
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 62
   <211> 119
   <212> PRT
   <213> Künstliche Sequenz
<220>
   <223> Beschreibung der künstlichen Sequenz: variable schwere Ketten VH
<400> 62
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
                   100                 105                 110
   Thr Thr Val Thr Val Ser Ser
           115
<210> 63
   <211> 119
   <212> PRT
   <213> Künstliche Sequenz
<220>
   <223> Beschreibung der künstlichen Sequenz: variable schwere Ketten VH
<400> 63
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 64
   <211> 119
   <212> PRT
   <213> Künstliche Sequenz
<220>
   <223> Beschreibung der künstlichen Sequenz: variable schwere Ketten VH
<400> 64
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 65
   <211> 119
   <212> PRT
   <213> Künstliche Sequenz
<220>
   <223> Beschreibung der künstlichen Sequenz: variable schwere Ketten VH
<400> 65
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 66
   <211> 119
   <212> PRT
   <213> Künstliche Sequenz
<220>
   <223> Beschreibung der künstlichen Sequenz: variable schwere Ketten VH
<400> 66
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 67
   <211> 119
   <212> PRT
   <213> Künstliche Sequenz
<220>
   <223> Beschreibung der künstlichen Sequenz: variable schwere Ketten VH
<400> 67
   Gln Val Gln Leu Val Gln Ser Gly Ala Glu Val Lys Lys Pro Gly Ala
   1               5                   10                  15
   Ser Val Lys Val Pro Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 68
   <211> 119
   <212> PRT
   <213> Künstliche Sequenz
<220>
   <223> Beschreibung der künstlichen Sequenz: variable schwere Ketten VH
<400> 68
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 69
   <211> 119
   <212> PRT
   <213> Künstliche Sequenz
<220>
   <223> Beschreibung der künstlichen Sequenz: variable schwere Ketten VH
<400> 69
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 70
   <211> 119
   <212> PRT
   <213> Künstliche Sequenz
<220>
   <223> Beschreibung der künstlichen Sequenz: variable schwere Ketten VH
<400> 70
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 71
   <211> 119
   <212> PRT
   <213> Künstliche Sequenz
<220>
   <223> Beschreibung der künstlichen Sequenz: variable schwere Ketten VH
<400> 71
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Tyr Tyr Asp Ala Ala Gly Pro Trp Phe Ala Tyr Trp Gly Gln Gly
                   100                 105                 110
   Thr Thr Val Thr Val Ser Ser
           115
<210> 72
   <211> 119
   <212> PRT
   <213> Künstliche Sequenz
<220>
   <223> Beschreibung der künstlichen Sequenz: variable schwere Ketten VH
<400> 72
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 73
   <211> 119
   <212> PRT
   <213> Künstliche Sequenz
<220>
   <223> Beschreibung der künstlichen Sequenz: variable schwere Ketten VH
<400> 73
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 74
   <211> 119
   <212> PRT
   <213> Künstliche Sequenz
<220>
   <223> Beschreibung der künstlichen Sequenz: variable schwere Ketten VH
<400> 74
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Thr Ser Thr Ala Tyr
   65                  70                  75                  80
   Met Glu Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Ser
           115
<210> 75
   <211> 119
   <212> PRT
   <213> Künstliche Sequenz
<220>
   <223> Beschreibung der künstlichen Sequenz: variable schwere Ketten VH
<400> 75
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Arg Val Thr Ile Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 76
   <211> 119
   <212> PRT
   <213> Künstliche Sequenz
<220>
   <223> Beschreibung der künstlichen Sequenz: variable schwere Ketten VH
<400> 76
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Arg Val Thr Ile Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 77
   <211> 119
   <212> PRT
   <213> Künstliche Sequenz
<220>
   <223> Beschreibung der künstlichen Sequenz: variable schwere Ketten VH
<400> 77
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Lys Gln Arg Pro Gly Gln Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Arg Val Thr Ile Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 78
   <211> 119
   <212> PRT
   <213> Künstliche Sequenz
<220>
   <223> Beschreibung der künstlichen Sequenz: variable schwere Ketten VH
<400> 78
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Met
           35                  40                  45
   Gly Asp Ile Tyr Pro Gly Gly Gly Tyr Thr Asn Tyr Asn Glu Lys Phe
       50                  55                  60
   Lys Gly Arg Val Thr Ile Thr Ala Asp Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 79
   <211> 119
   <212> PRT
   <213> Künstliche Sequenz
<220>
   <223> Beschreibung der künstlichen Sequenz: variable schwere Ketten VH
<400> 79
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Trp Leu Gly Trp Val Arg Gln Ala Pro Gly Gln Gly Leu Glu Trp Met
           35                  40                  45
   Gly Asp Ile Tyr Pro Gly Gly Gly Tyr Thr Asn Tyr Asn Glu Lys Phe
       50                  55                  60
   Lys Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Tyr Tyr Asp Ala Ala Gly Pro Trp Phe Ala Tyr Trp Gly Gln Gly
               100                 105                 110
   Thr Leu Val Thr Val Ser Ser
           115
<210> 80
   <211> 114
   <212> PRT
   <213> Künstliche Sequenz
<220>
   <223> Beschreibung der künstlichen Sequenz: variable leichte Ketten
<400> 80
   Asp Ile Gln Met Thr Gln Thr Pro Leu Ser Leu Pro Val Ser Leu Gly
    1               5                  10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
                   100                 105                 110
   Arg Ala
<210> 81
   <211> 114
   <212> PRT
   <213> Künstliche Sequenz
<220>
   <223> Beschreibung der künstlichen Sequenz: variable leichte Ketten
<400> 81
   Asp Ile Val Ile Thr Gln Thr Pro Leu Ser Leu Pro Val Ser Leu Gly
    1               5                  10                  15
   Asp Gln Ala Ser Ile Ser Cys Arg Ser Ser Gln Ser Leu Leu His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Phe Cys Ser Gln Ser
                   85                  90                  95
   Thr His Val Pro Tyr Thr Phe Gly Gly Gly Thr Lys Leu Glu Ile Lys
               100                 105                 110
   Arg Ala
<210> 82
   <211> 114
   <212> PRT
   <213> Künstliche Sequenz
<220>
   <223> Beschreibung der künstlichen Sequenz: variable leichte Ketten
<400> 82
   Asp Ile Gln Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Leu Lys
               100                 105                 110
   Arg Ala
<210> 83
   <211> 114
   <212> PRT
   <213> Künstliche Sequenz
<220>
   <223> Beschreibung der künstlichen Sequenz: variable leichte Ketten
<400> 83
   Asp Val Leu Met Thr Gln Thr Pro Leu Ser Leu Pro Val Ser Leu Gly
    1               5                  10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
               100                 105                 110
   Arg Ala
<210> 84
   <211> 114
   <212> PRT
   <213> Künstliche Sequenz
<220>
   <223> Beschreibung der künstlichen Sequenz: variable leichte Ketten
<400> 84
   Asp Val Leu Met Thr Gln Thr Pro Leu Ser Leu Pro Val Ser Leu Gly
    1               5                  10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Leu Lys
               100                 105                 110
   Arg Ala
<210> 85
   <211> 114
   <212> PRT
   <213> Künstliche Sequenz
<220>
   <223> Beschreibung der künstlichen Sequenz: variable leichte Ketten
<400> 85
   Asp Ile Gln Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gly Gly Thr Lys Val Glu Ile Lys
               100                 105                 110
   Arg Ala
<210> 86
   <211> 114
   <212> PRT
   <213> Künstliche Sequenz
<220>
   <223> Beschreibung der künstlichen Sequenz: variable leichte Ketten
<400> 86
   Asp Ile Gln Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                      80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 87
   <211> 114
   <212> PRT
   <213> Künstliche Sequenz
<220>
   <223> Beschreibung der künstlichen Sequenz: variable leichte Ketten
<400> 87
   Asp Ile Gln Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                      80
   Ser Arg Val Glu Ala Glu Asp Val Gly Val Tyr Tyr Cys Phe Gln Gly
                   85                  90                  95
   Ser His Val Pro Tyr Thr Phe Gly Gly Gly Thr Lys Val Glu Ile Lys
               100                 105                 110
   Arg Ala
<210> 88
   <211> 114
   <212> FRT
   <213> Künstliche Sequenz
<220>
   <223> Beschreibung der künstlichen Sequenz: variable leichte Ketten
<400> 88
   Asp Ile Gln Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 89
   <211> 114
   <212> PRT
   <213> Künstliche Sequenz
<220>
   <223> Beschreibung der künstlichen Sequenz: variable leichte Ketten
<400> 89
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gly Gly Thr Lys Val Glu Ile Lys
   100 105 110
   Arg Ala
<210> 90
   <211> 114
   <212> PRT
   <213> Künstliche Sequenz
<220>
   <223> Beschreibung der künstlichen Sequenz: variable leichte Ketten
<400> 90
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 91
   <211> 114
   <212> PRT
   <213> Künstliche Sequenz
<220>
   <223> Beschreibung der künstlichen Sequenz: variable leichte Ketten
<400> 91
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
    65                 70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gly Gly Thr Lys Val Glu Ile Lys
               100                 105                 110
   Arg Ala
<210> 92
   <211> 114
   <212> PRT
   <213> Künstliche Sequenz
<220>
   <223> Beschreibung der künstlichen Sequenz: variable leichte Ketten
<400> 92
   Asp Ile Val Met Thr Gln Ser Pro Leu Ser Leu Pro Val Thr Pro Gly
     1               5                  10                  15
   Glu Pro Ala Ser Ile Ser Cys Arg Ser Ser Gln Ser Ile Val His Ser
                20                  25                  30
   Asn Gly Asn Thr Tyr Leu Glu Trp Tyr Leu Gln Lys Pro Gly Gln Ser
            35                  40                  45
   Pro Gln Leu Leu Ile Tyr Lys Val Ser Asn Arg Phe Ser Gly Val Pro
        50                  55                  60
   Asp Arg Phe Ser Gly Ser Gly Ser Gly Thr Asp Phe Thr Leu Lys Ile
    65                 70                  75                  80
   Ser Arg Val Glu Ala Glu Asp Val Gly Val Tyr Tyr Cys Phe Gln Gly
                   85                  90                  95
   Ser His Val Pro Tyr Thr Phe Gly Gln Gly Thr Lys Val Glu Ile Lys
               100                 105                 110
   Arg Ala
<210> 93
   <211> 114
   <212> PRT
   <213> Künstliche Sequenz
<220>
   <223> Beschreibung der künstlichen Sequenz: variable leichte Ketten
<400> 93
   Asp Ile Val Met Thr Gln Thr Pro Leu Ser Leu Pro Val Thr Pro Gly
     1               5                  10                  15
   Gln Pro Ala Ser Ile Ser Cys Arg Ser Ser Gln Ser Ile Val His Ser
                20                  25                  30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 94
   <211> 114
   <212> PRT
   <213> Künstliche Sequenz
<220>
   <223> Beschreibung der künstlichen Sequenz: variable leichte Ketten
<400> 94
   Asp Ile Val Met Thr Gln Thr Pro Leu Ser Leu Ser Val Thr Pro Gly
   1 5 10 15
   Gln Pro Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Phe Gln Gly
   85 90 95
   Ser His Val Pro Tyr Thr Phe Gly Gln Gly Thr Lys Val Glu Ile Lys
   100 105 110
   Arg Ala
<210> 95
   <211> 276
   <212> PRT
   <213> Künstliche Sequenz
<220>
   <223> Beschreibung der künstlichen Sequenz: single chain Fv Formate
<400> 95
   Gln Val Gln Leu Lys Glu Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Leu Thr Ala Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Tyr Tyr Asp Ala Ala Gly Pro Gly Phe Ala Tyr Trp Gly Gln Gly
   100 105 110
   Thr Thr Val Thr Val Ser Ser Ala Ser Ser Gly Gly Gly Gly Ser Gly
   115 120 125
   Gly Gly Gly Ser Gly Gly Ser Ala Arg Asp Ile Gln Met Thr Gln Thr
   130 135 140
   Pro Leu Ser Leu Pro Val Ser Leu Gly Asp Gln Ala Ser Ile Ser Cys
   145 150 155 160
   Arg Ser Ser Gln Ser Ile Val His Ser Asn Gly Asn Thr Tyr Leu Glu
   165 170 175
   Trp Tyr Leu Gln Lys Pro Gly Gln Ser Pro Lys Leu Leu Ile Tyr Lys
   180 185 190
   Val Ser Asn Arg Phe Ser Gly Val Pro Asp Arg Phe Ser Gly Ser Gly
   195 200 205
   Ser Gly Thr Asp Phe Thr Leu Lys Ile Ser Arg Val Glu Ala Glu Asp
   210 215 220
   Leu Gly Val Tyr Tyr Cys Phe Gln Gly Ser His Val Pro Tyr Thr Phe
   225 230 235 240
   Gly Gly Gly Thr Lys Leu Glu Ile Lys Arg Ala Ala Ala His His His
   245 250 255
   His His His Gly Ala Ala Glu Gln Lys Leu Ile Ser Glu Glu Asp Leu
   260 265 270
   Asn Gly Ala Ala
   275
<210> 96
   <211> 267
   <212> PRT
   <213> Künstliche Sequenz
<220>
   <223> Beschreibung der künstlichen Sequenz: single chain Fv Formate
<400> 96
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Gly Ser Gly Ser Ser Ala
           115                 120                 125
   Asp Ile Gln Met Thr Gln Thr Pro Leu Ser Leu Pro Val Ser Leu Gly
       130                 135                 140
   Asp Gln Ala Ser Ile Ser Cys Arg Ser Ser Gln Ser Ile Val His Ser
   145                 150                 155                 160
   Asn Gly Asn Thr Tyr Leu Glu Trp Tyr Leu Gln Lys Pro Gly Gln Ser
                   165                 170                 175
   Pro Lys Leu Leu Ile Tyr Lys Val Ser Asn Arg Phe Ser Gly Val Pro
               180                 185                 190
   Asp Arg Phe Ser Gly Ser Gly Ser Gly Thr Asp Phe Thr Leu Lys Ile
           195                 200                 205
   Ser Arg Val Glu Ala Glu Asp Leu Gly Val Tyr Tyr Cys Phe Gln Gly
       210                 215                 220
   Ser His Val Pro Tyr Thr Phe Gly Gly Gly Thr Lys Leu Glu Ile Lys
   225                 230                 235                 240
   Arg Ala Ala Ala His His His His His His Gly Ala Ala Glu Gln Lys
                   245                 250                 255
   Leu Ile Ser Glu Glu Asp Leu Asn Gly Ala Ala
               260                 265
<210> 97
   <211> 266
   <212> PRT
   <213> Künstliche Sequenz
<220>
   <223> Beschreibung der künstlichen Sequenz: single chain Fv Formate
<400> 97
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Gly Gly Ser Ser Ala Asp
           115                 120                 125
   Ile Gln Met Thr Gln Thr Pro Leu Ser Leu Pro Val Ser Leu Gly Asp
       130                 135                 140
   Gln Ala Ser Ile Ser Cys Arg Ser Ser Gln Ser Ile Val His Ser Asn
   145                 150                 155                 160
   Gly Asn Thr Tyr Leu Glu Trp Tyr Leu Gln Lys Pro Gly Gln Ser Pro
                   165                 170                 175
   Lys Leu Leu Ile Tyr Lys Val Ser Asn Arg Phe Ser Gly Val Pro Asp
               180                 185                 190
   Arg Phe Ser Gly Ser Gly Ser Gly Thr Asp Phe Thr Leu Lys Ile Ser
           195                 200                 205
   Arg Val Glu Ala Glu Asp Leu Gly Val Tyr Tyr Cys Phe Gln Gly Ser
       210                 215                 220
   His Val Pro Tyr Thr Phe Gly Gly Gly Thr Lys Leu Glu Ile Lys Arg
   225                 230                 235                 240
   Ala Ala Ala His His His His His His Gly Ala Ala Glu Gln Lys Leu
                   245                 250                 255
   Ile Ser Glu Glu Asp Leu Asn Gly Ala Ala
               260                 265
<210> 98
   <211> 265
   <212> PRT
   <213> Künstliche Sequenz
<220>
   <223> Beschreibung der künstlichen Sequenz: single chain Fv Formate
<400> 98
   Gln Val Gln Leu Lys Glu Ser Gly Ala Glu Leu Val Arg Pro Gly Thr
    1               5                  10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Gly Ser Ser Ala Asp Ile
           115                 120                 125
   Gln Met Thr Gln Thr Pro Leu Ser Leu Pro Val Ser Leu Gly Asp Gln
       130                 135                 140
   Ala Ser Ile Ser Cys Arg Ser Ser Gln Ser Ile Val His Ser Asn Gly
   145                 150                 155                 160
   Asn Thr Tyr Leu Glu Trp Tyr Leu Gln Lys Pro Gly Gln Ser Pro Lys
                   165                 170                 175
   Leu Leu Ile Tyr Lys Val Ser Asn Arg Phe Ser Gly Val Pro Asp Arg
               180                 185                 190
   Phe Ser Gly Ser Gly Ser Gly Thr Asp Phe Thr Leu Lys Ile Ser Arg
           195                 200                 205
   Val Glu Ala Glu Asp Leu Gly Val Tyr Tyr Cys Phe Gln Gly Ser His
       210                 215                 220
   Val Pro Tyr Thr Phe Gly Gly Gly Thr Lys Leu Glu Ile Lys Arg Ala
   225                 230                 235                 240
   Ala Ala His His His His His His Gly Ala Ala Glu Gln Lys Leu Ile
                   245                 250                 255
   Ser Glu Glu Asp Leu Asn Gly Ala Ala
               260                 265
<210> 99
   <211> 264
   <212> PRT
   <213> Künstliche Sequenz
<220>
   <223> Beschreibung der künstlichen Sequenz: single chain Fv Formate
<400> 99
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Ser Ser Ala Asp Ile Gln
           115                 120                 125
   Met Thr Gln Thr Pro Leu Ser Leu Pro Val Ser Leu Gly Asp Gln Ala
       130                 135                 140
   Ser Ile Ser Cys Arg Ser Ser Gln Ser Ile Val His Ser Asn Gly Asn
   145                 150                 155                 160
   Thr Tyr Leu Glu Trp Tyr Leu Gln Lys Pro Gly Gln Ser Pro Lys Leu
                   165                 170                 175
   Leu Ile Tyr Lys Val Ser Asn Arg Phe Ser Gly Val Pro Asp Arg Phe
               180                 185                 190
   Ser Gly Ser Gly Ser Gly Thr Asp Phe Thr Leu Lys Ile Ser Arg Val
           195                 200                 205
   Glu Ala Glu Asp Leu Gly Val Tyr Tyr Cys Phe Gln Gly Ser His Val
       210                 215                 220
   Pro Tyr Thr Phe Gly Gly Gly Thr Lys Leu Glu Ile Lys Arg Ala Ala
   225                 230                 235                 240
   Ala His His His His His His Gly Ala Ala Glu Gln Lys Leu Ile Ser
                   245                 250                 255
   Glu Glu Asp Leu Asn Gly Ala Ala
               260
<210> 100
   <211> 263
   <212> PRT
   <213> Künstliche Sequenz
<220>
   <223> Beschreibung der künstlichen Sequenz: single chain Fv Formate
<400> 100
   Gln Val Gln Leu Lys Glu Ser Gly Ala Glu Leu Val Arg Pro Gly Thr
    1               5                  10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Ser Ala Asp Ile Gln Met
           115                 120                 125
   Thr Gln Thr Pro Leu Ser Leu Pro Val Ser Leu Gly Asp Gln Ala Ser
       130                 135                 140
   Ile Ser Cys Arg Ser Ser Gln Ser Ile Val His Ser Asn Gly Asn Thr
   145                 150                 155                 160
   Tyr Leu Glu Trp Tyr Leu Gln Lys Pro Gly Gln Ser Pro Lys Leu Leu
                   165                 170                 175
   Ile Tyr Lys Val Ser Asn Arg Phe Ser Gly Val Pro Asp Arg Phe Ser
               180                 185                 190
   Gly Ser Gly Ser Gly Thr Asp Phe Thr Leu Lys Ile Ser Arg Val Glu
           195                 200                 205
   Ala Glu Asp Leu Gly Val Tyr Tyr Cys Phe Gln Gly Ser His Val Pro
       210                 215                 220
   Tyr Thr Phe Gly Gly Gly Thr Lys Leu Glu Ile Lys Arg Ala Ala Ala
   225                 230                 235                 240
   His His His His His His Gly Ala Ala Glu Gln Lys Leu Ile Ser Glu
                   245                 250                 255
   Glu Asp Leu Asn Gly Ala Ala
               260
<210> 101
   <211> 262
   <212> PRT
   <213> Künstliche Sequenz
<220>
   <223> Beschreibung der künstlichen Sequenz: single chain Fv Formate
<400> 101
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ser Ala Asp Ile Gln Met Thr
           115                 120                 125
   Gln Thr Pro Leu Ser Leu Pro Val Ser Leu Gly Asp Gln Ala Ser Ile
       130                 135                 140
   Ser Cys Arg Ser Ser Gln Ser Ile Val His Ser Asn Gly Asn Thr Tyr
   145                 150                 155                 160
   Leu Glu Trp Tyr Leu Gln Lys Pro Gly Gln Ser Pro Lys Leu Leu Ile
                   165                 170                 175
   Tyr Lys Val Ser Asn Arg Phe Ser Gly Val Pro Asp Arg Phe Ser Gly
               180                 185                 190
   Ser Gly Ser Gly Thr Asp Phe Thr Leu Lys Ile Ser Arg Val Glu Ala
           195                 200                 205
   Glu Asp Leu Gly Val Tyr Tyr Cys Phe Gln Gly Ser His Val Pro Tyr
       210                 215                 220
   Thr Phe Gly Gly Gly Thr Lys Leu Glu Ile Lys Arg Ala Ala Ala His
   225                 230                 235                 240
   His His His His His Gly Ala Ala Glu Gln Lys Leu Ile Ser Glu Glu
                   245                 250                 255
   Asp Leu Asn Gly Ala Ala
               260
<210> 102
   <211> 261
   <212> PRT
   <213> Künstliche Sequenz
<220>
   <223> Beschreibung der künstlichen Sequenz: single chain Fv Formate
<400> 102
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ser Ala Asp Ile Gln Met Thr Gln
           115                 120                 125
   Thr Pro Leu Ser Leu Pro Val Ser Leu Gly Asp Gln Ala Ser Ile Ser
       130                 135                 140
   Cys Arg Ser Ser Gln Ser Ile Val His Ser Asn Gly Asn Thr Tyr Leu
   145                 150                 155                 160
   Glu Trp Tyr Leu Gln Lys Pro Gly Gln Ser Pro Lys Leu Leu Ile Tyr
                   165                 170                 175
   Lys Val Ser Asn Arg Phe Ser Gly Val Pro Asp Arg Phe Ser Gly Ser
               180                 185                 190
   Gly Ser Gly Thr Asp Phe Thr Leu Lys Ile Ser Arg Val Glu Ala Glu
           195                 200                 205
   Asp Leu Gly Val Tyr Tyr Cys Phe Gln Gly Ser His Val Pro Tyr Thr
       210                 215                 220
   Phe Gly Gly Gly Thr Lys Leu Glu Ile Lys Arg Ala Ala Ala His His
   225                 230                 235                 240
   His His His His Gly Ala Ala Glu Gln Lys Leu Ile Ser Glu Glu Asp
                   245                 250                 255
   Leu Asn Gly Ala Ala
               260
<210> 103
   <211> 260
   <212> PRT
   <213> Künstliche Sequenz
<220>
   <223> Beschreibung der künstlichen Sequenz: single chain Fv Formate
<400> 103
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Ala Asp Ile Gln Met Thr Gln Thr
           115                 120                 125
   Pro Leu Ser Leu Pro Val Ser Leu Gly Asp Gln Ala Ser Ile Ser Cys
       130                 135                 140
   Arg Ser Ser Gln Ser Ile Val His Ser Asn Gly Asn Thr Tyr Leu Glu
   145                 150                 155                 160
   Trp Tyr Leu Gln Lys Pro Gly Gln Ser Pro Lys Leu Leu Ile Tyr Lys
                   165                 170                 175
   Val Ser Asn Arg Phe Ser Gly Val Pro Asp Arg Phe Ser Gly Ser Gly
               180                 185                 190
   Ser Gly Thr Asp Phe Thr Leu Lys Ile Ser Arg Val Glu Ala Glu Asp
           195                 200                 205
   Leu Gly Val Tyr Tyr Cys Phe Gln Gly Ser His Val Pro Tyr Thr Phe
       210                 215                 220
   Gly Gly Gly Thr Lys Leu Glu Ile Lys Arg Ala Ala Ala His His His
   225                 230                 235                 240
   His His His Gly Ala Ala Glu Gln Lys Leu Ile Ser Glu Glu Asp Leu
                   245                 250                 255
   Asn Gly Ala Ala
               260
<210> 104
   <211> 259
   <212> PRT
   <213> Künstliche Sequenz
<220>
   <223> Beschreibung der künstlichen Sequenz: single chain Fv Formate
<400> 104
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Ala Asp Ile Gln Met Thr Gln Thr Pro
           115                 120                 125
   Leu Ser Leu Pro Val Ser Leu Gly Asp Gln Ala Ser Ile Ser Cys Arg
       130                 135                 140
   Ser Ser Gln Ser Ile Val His Ser Asn Gly Asn Thr Tyr Leu Glu Trp
   145                 150                 155                 160
   Tyr Leu Gln Lys Pro Gly Gln Ser Pro Lys Leu Leu Ile Tyr Lys Val
                   165                 170                 175
   Ser Asn Arg Phe Ser Gly Val Pro Asp Arg Phe Ser Gly Ser Gly Ser
               180                 185                 190
   Gly Thr Asp Phe Thr Leu Lys Ile Ser Arg Val Glu Ala Glu Asp Leu
           195                 200                 205
   Gly Val Tyr Tyr Cys Phe Gln Gly Ser His Val Pro Tyr Thr Phe Gly
       210                 215                 220
   Gly Gly Thr Lys Leu Glu Ile Lys Arg Ala Ala Ala His His His His
   225                 230                 235                 240
   His His Gly Ala Ala Glu Gln Lys Leu Ile Ser Glu Glu Asp Leu Asn
                   245                 250                 255
   Gly Ala Ala
<210> 105
   <211> 258
   <212> PRT
   <213> Künstliche Sequenz
<220>
   <223> Beschreibung der künstlichen Sequenz: single chain Fv Formate
<400> 105
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Ser Asp Ile Gln Met Thr Gln Thr Pro Leu
           115                 120                 125
   Ser Leu Pro Val Ser Leu Gly Asp Gln Ala Ser Ile Ser Cys Arg Ser
       130                 135                 140
   Ser Gln Ser Ile Val His Ser Asn Gly Asn Thr Tyr Leu Glu Trp Tyr
   145                 150                 155                 160
   Leu Gln Lys Pro Gly Gln Ser Pro Lys Leu Leu Ile Tyr Lys Val Ser
                   165                 170                 175
   Asn Arg Phe Ser Gly Val Pro Asp Arg Phe Ser Gly Ser Gly Ser Gly
               180                 185                 190
   Thr Asp Phe Thr Leu Lys Ile Ser Arg Val Glu Ala Glu Asp Leu Gly
           195                 200                 205
   Val Tyr Tyr Cys Phe Gln Gly Ser His Val Pro Tyr Thr Phe Gly Gly
       210                 215                 220
   Gly Thr Lys Leu Glu Ile Lys Arg Ala Ala Ala His His His His His
   225                 230                 235                 240
   His Gly Ala Ala Glu Gln Lys Leu Ile Ser Glu Glu Asp Leu Asn Gly
                   245                 250                 255
   Ala Ala
<210> 106
   <211> 257
   <212> PRT
   <213> Künstliche Sequenz
<220>
   <223> Beschreibung der künstlichen Sequenz: single chain Fv Formate
<400> 106
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Gly Phe Ala Tyr Trp Gly Gln Gly
               100                 105                 110
   Thr Thr Val Thr Val Ser Asp Ile Gln Met Thr Gln Thr Pro Leu Ser
           115                 120                 125
   Leu Pro Val Ser Leu Gly Asp Gln Ala Ser Ile Ser Cys Arg Ser Ser
       130                 135                 140
   Gln Ser Ile Val His Ser Asn Gly Asn Thr Tyr Leu Glu Trp Tyr Leu
   145                 150                 155                 160
   Gln Lys Pro Gly Gln Ser Pro Lys Leu Leu Ile Tyr Lys Val Ser Asn
                   165                 170                 175
   Arg Phe Ser Gly Val Pro Asp Arg Phe Ser Gly Ser Gly Ser Gly Thr
               180                 185                 190
   Asp Phe Thr Leu Lys Ile Ser Arg Val Glu Ala Glu Asp Leu Gly Val
           195                 200                 205
   Tyr Tyr Cys Phe Gln Gly Ser His Val Pro Tyr Thr Phe Gly Gly Gly
       210                 215                 220
   Thr Lys Leu Glu Ile Lys Arg Ala Ala Ala His His His His His His
   225                 230                 235                 240
   Gly Ala Ala Glu Gln Lys Leu Ile Ser Glu Glu Asp Leu Asn Gly Ala
                   245                 250                 255
   Ala
<210> 107
   <211> 219
   <212> PRT
   <213> Künstliche Sequenz
<220>
   <223> Beschreibung der künstlichen Sequenz: muriner Antikörper
<400> 107
   Asp Ile Val Ile Thr Gln Thr Pro Leu Ser Leu Pro Val Ser Leu Gly
   1 5 10 15
   Asp Gln Ala Ser Ile Ser Cys Arg Ser Ser Gln Ser Leu Leu His Ser
   20 25 30
   Asn Gly Asn Thr Tyr Leu His Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Phe Cys Ser Gln Ser
   85 90 95
   Thr His Val Pro Tyr Thr Phe Gly Gly Gly Thr Lys Leu Glu Ile Lys
   100 105 110
   Arg Ala Asp Ala Ala Pro Thr Val Ser Ile Phe Pro Pro Ser Ser Glu
   115 120 125
   Gln Leu Thr Ser Gly Gly Ala Ser Val Val Cys Phe Leu Asn Asn Phe
   130 135 140
   Tyr Pro Lys Asp Ile Asn Val Lys Trp Lys Ile Asp Gly Ser Glu Arg
   145 150 155 160
   Gln Asn Gly Val Leu Asn Ser Trp Thr Asp Gln Asp Ser Lys Asp Ser
   165 170 175
   Thr Tyr Ser Met Ser Ser Thr Leu Thr Leu Thr Lys Asp Glu Tyr Glu
   180 185 190
   Arg His Asn Ser Tyr Thr Cys Glu Ala Thr His Lys Thr Ser Thr Ser
   195 200 205
   Pro Ile Val Lys Ser Phe Asn Arg Asn Glu Cys
   210 215
<210> 108
   <211> 219
   <212> PRT
   <213> Künstliche Sequenz
<220>
   <223> Beschreibung der künstlichen Sequenz: muriner Antikörper
<400> 108
   Asp Ile Gln Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
               100                 105                 110
   Arg Ala Asp Ala Ala Pro Thr Val Ser Ile Phe Pro Pro Ser Ser Glu
           115                 120                 125
   Gln Leu Thr Ser Gly Gly Ala Ser Val Val Cys Phe Leu Asn Asn Phe
       130                 135                 140
   Tyr Pro Lys Asp Ile Asn Val Lys Trp Lys Ile Asp Gly Ser Glu Arg
   145                 150                 155                 160
   Gln Asn Gly Val Leu Asn Ser Trp Thr Asp Gln Asp Ser Lys Asp Ser
                   165                 170                 175
   Thr Tyr Ser Met Ser Ser Thr Leu Thr Leu Thr Lys Asp Glu Tyr Glu
               180                 185                 190
   Arg His Asn Ser Tyr Thr Cys Glu Ala Thr His Lys Thr Ser Thr Ser
           195                 200                 205
   Pro Ile Val Lys Ser Phe Asn Arg Asn Glu Cys
       210                 215
<210> 109
   <211> 571
   <212> PRT
   <213> Künstliche Sequenz
<220>
   <223> Beschreibung der künstlichen Sequenz: muriner Antikörper
<400> 109
   Gln Val Gln Leu Lys Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Ser Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Tyr Asp Asn His Tyr Phe Asp Tyr Trp Gly Gln Gly Thr Thr
               100                 105                 110
   Leu Thr Val Ser Glu Ser Gln Ser Phe Pro Asn Val Phe Pro Leu Val
           115                 120                 125
   Ser Cys Glu Ser Pro Leu Ser Asp Lys Asn Leu Val Ala Met Gly Cys
       130                 135                 140
   Leu Ala Arg Asp Phe Leu Pro Ser Thr Ile Ser Phe Thr Trp Asn Tyr
   145                 150                 155                 160
   Gln Asn Asn Thr Glu Val Ile Gln Gly Ile Arg Thr Phe Pro Thr Leu
                   165                 170                 175
   Arg Thr Gly Gly Lys Tyr Leu Ala Thr Ser Gln Val Leu Leu Ser Pro
               180                 185                 190
   Lys Ser Ile Leu Glu Gly Ser Asp Glu Tyr Leu Val Cys Lys Ile His
           195                 200                 205
   Tyr Gly Gly Lys Asn Arg Asp Leu His Val Pro Ile Pro Ala Val Ala
       210                 215                 220
   Glu Met Asn Pro Asn Val Asn Val Phe Val Pro Pro Arg Asp Gly Phe
   225                 230                 235                 240
   Ser Gly Pro Ala Pro Arg Lys Ser Lys Leu Ile Cys Glu Ala Thr Asn
                   245                 250                 255
   Phe Thr Pro Lys Pro Ile Thr Val Ser Trp Leu Lys Asp Gly Lys Leu
               260                 265                 270
   Val Glu Ser Gly Phe Thr Thr Asp Pro Val Thr Ile Glu Asn Lys Gly
           275                 280                 285
   Ser Thr Pro Gln Thr Tyr Lys Val Ile Ser Thr Leu Thr Ile Ser Glu
       290                 295                 300
   Ile Asp Trp Leu Asn Leu Asn Val Tyr Thr Cys Arg Val Asp His Arg
   305                 310                 315                 320
   Gly Leu Thr Phe Leu Lys Asn Val Ser Ser Thr Cys Ala Ala Ser Pro
                   325                 330                 335
   Ser Thr Asp Ile Leu Thr Phe Thr Ile Pro Pro Ser Phe Ala Asp Ile
               340                 345                 350
   Phe Leu Ser Lys Ser Ala Asn Leu Thr Cys Leu Val Ser Asn Leu Ala
           355                 360                 365
   Thr Tyr Glu Thr Leu Asn Ile Ser Trp Ala Ser Gln Ser Gly Glu Pro
       370                 375                 380
   Leu Glu Thr Lys Ile Lys Ile Met Glu Ser His Pro Asn Gly Thr Phe
   385                 390                 395                 400
   Ser Ala Lys Gly Val Ala Ser Val Cys Val Glu Asp Trp Asn Asn Arg
                   405                 410                 415
   Lys Glu Phe Val Cys Thr Val Thr His Arg Asp Leu Pro Ser Pro Gln
               420                 425                 430
   Lys Lys Phe Ile Ser Lys Pro Asn Glu Val His Lys His Pro Pro Ala
           435                 440                 445
   Val Tyr Leu Leu Pro Pro Ala Arg Glu Gln Leu Asn Leu Arg Glu Ser
       450                 455                 460
   Ala Thr Val Thr Cys Leu Val Lys Gly Phe Ser Pro Ala Asp Ile Ser
   465                 470                 475                 480
   Val Gln Trp Leu Gln Arg Gly Gln Leu Leu Pro Gln Glu Lys Tyr Val
                   485                 490                 495
   Thr Ser Ala Pro Met Pro Glu Pro Gly Ala Pro Gly Phe Tyr Phe Thr
               500                 505                 510
   His Ser Ile Leu Thr Val Thr Glu Glu Glu Trp Asn Ser Gly Glu Thr
           515                 520                 525
   Tyr Thr Cys Val Val Gly His Glu Ala Leu Pro His Leu Val Thr Glu
       530                 535                 540
   Arg Thr Val Asp Lys Ser Thr Gly Lys Pro Thr Leu Tyr Asn Val Ser
   545                 550                 555                 560
   Leu Ile Met Ser Asp Thr Gly Gly Thr Cys Tyr
                   565                 570
<210> 110
   <211> 573
   <212> PRT
   <213> Künstliche Sequenz
<220>
   <223> Beschreibung der künstlichen Sequenz: muriner Antikörper
<400> 110
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Glu Ser Gln Ser Phe Pro Asn Val Phe Pro
           115                 120                 125
   Leu Val Ser Cys Glu Ser Pro Leu Ser Asp Lys Asn Leu Val Ala Met
       130                 135                 140
   Gly Cys Leu Ala Arg Asp Phe Leu Pro Ser Thr Ile Ser Phe Thr Trp
   145                 150                 155                 160
   Asn Tyr Gln Asn Asn Thr Glu Val Ile Gln Gly Ile Arg Thr Phe Pro
                   165                 170                 175
   Thr Leu Arg Thr Gly Gly Lys Tyr Leu Ala Thr Ser Gln Val Leu Leu
   180 185 190
   Ser Pro Lys Ser Ile Leu Glu Gly Ser Asp Glu Tyr Leu Val Cys Lys
   195 200 205
   Ile His Tyr Gly Gly Lys Asn Arg Asp Leu His Val Pro Ile Pro Ala
   210 215 220
   Val Ala Glu Met Asn Pro Asn Val Asn Val Phe Val Pro Pro Arg Asp
   225 230 235 240
   Gly Phe Ser Gly Pro Ala Pro Arg Lys Ser Lys Leu Ile Cys Glu Ala
   245 250 255
   Thr Asn Phe Thr Pro Lys Pro Ile Thr Val Ser Trp Leu Lys Asp Gly
   260 265 270
   Lys Leu Val Glu Ser Gly Phe Thr Thr Asp Pro Val Thr Ile Glu Asn
   275 280 285
   Lys Gly Ser Thr Pro Gln Thr Tyr Lys Val Ile Ser Thr Leu Thr Ile
   290 295 300
   Ser Glu Ile Asp Trp Leu Asn Leu Asn Val Tyr Thr Cys Arg Val Asp
   305 310 315 320
   His Arg Gly Leu Thr Phe Leu Lys Asn Val Ser Ser Thr Cys Ala Ala
   325 330 335
   Ser Pro Ser Thr Asp Ile Leu Thr Phe Thr Ile Pro Pro Ser Phe Ala
   340 345 350
   Asp Ile Phe Leu Ser Lys Ser Ala Asn Leu Thr Cys Leu Val Ser Asn
   355 360 365
   Leu Ala Thr Tyr Glu Thr Leu Asn Ile Ser Trp Ala Ser Gln Ser Gly
   370 375 380
   Glu Pro Leu Glu Thr Lys Ile Lys Ile Met Glu Ser His Pro Asn Gly
   385 390 395 400
   Thr Phe Ser Ala Lys Gly Val Ala Ser Val Cys Val Glu Asp Trp Asn
   405 410 415
   Asn Arg Lys Glu Phe Val Cys Thr Val Thr His Arg Asp Leu Pro Ser
   420 425 430
   Pro Gln Lys Lys Phe Ile Ser Lys Pro Asn Glu Val His Lys His Pro
   435 440 445
   Pro Ala Val Tyr Leu Leu Pro Pro Ala Arg Glu Gln Leu Asn Leu Arg
   450 455 460
   Glu Ser Ala Thr Val Thr Cys Leu Val Lys Gly Phe Ser Pro Ala Asp
   465 470 475 480
   Ile Ser Val Gln Trp Leu Gln Arg Gly Gln Leu Leu Pro Gln Glu Lys
   485 490 495
   Tyr Val Thr Ser Ala Pro Met Pro Glu Pro Gly Ala Pro Gly Phe Tyr
   500 505 510
   Phe Thr His Ser Ile Leu Thr Val Thr Glu Glu Glu Trp Asn Ser Gly
               515                 520                 525
   Glu Thr Tyr Thr Cys Val Val Gly His Glu Ala Leu Pro His Leu Val
       530                 535                 540
   Thr Glu Arg Thr Val Asp Lys Ser Thr Gly Lys Pro Thr Leu Tyr Asn
   545                 550                 555                 560
   Val Ser Leu Ile Met Ser Asp Thr Gly Gly Thr Cys Tyr
                   565                 570
<210> 111
   <211> 448
   <212> PRT
   <213> Künstliche Sequenz
<220>
   <223> Beschreibung der künstlichen Sequenz: chimärer Antikörper Maus/Mensch
<400> 111
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Gly Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 112
   <211> 571
   <212> PRT
   <213> Künstliche Sequenz
<220>
   <223> Beschreibung der künstlichen Sequenz: chimärer Antikörper Maus/Mensch
<400> 112
   Gln Val Gln Leu Lys Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Tyr Tyr Asp Ala Ala Gly Pro Trp Phe Ala Tyr Trp Gly Gln Gly
               100                 105                 110
   Thr Thr Val Thr Val Ser Gly Ser Ala Ser Ala Pro Thr Leu Phe Pro
           115                 120                 125
   Leu Val Ser Cys Glu Asn Ser Pro Ser Asp Thr Ser Ser Val Ala Val
       130                 135                 140
   Gly Cys Leu Ala Gln Asp Phe Leu Pro Asp Ser Ile Thr Leu Ser Trp
   145                 150                 155                 160
   Lys Tyr Lys Asn Asn Ser Asp Ile Ser Ser Thr Arg Gly Phe Pro Ser
                   165                 170                 175
   Val Leu Arg Gly Gly Lys Tyr Ala Ala Thr Ser Gln Val Leu Leu Pro
               180                 185                 190
   Ser Lys Asp Val Met Gln Gly Thr Asp Glu His Val Val Cys Lys Val
           195                 200                 205
   Gln His Pro Asn Gly Asn Lys Glu Lys Asn Val Pro Leu Pro Val Ile
       210                 215                 220
   Ala Glu Leu Pro Pro Lys Val Ser Val Phe Val Pro Pro Arg Asp Gly
   225                 230                 235                 240
   Phe Phe Gly Asn Pro Arg Lys Ser Lys Leu Ile Cys Gln Ala Thr Gly
                   245                 250                 255
   Phe Ser Pro Arg Gln Ile Gln Val Ser Trp Leu Arg Glu Gly Lys Gln
               260                 265                 270
   Val Gly Ser Gly Val Thr Thr Asp Gln Val Gln Ala Glu Ala Lys Glu
           275                 280                 285
   Ser Gly Pro Thr Thr Tyr Lys Val Thr Ser Thr Leu Thr Ile Lys Glu
       290                 295                 300
   Ser Asp Trp Leu Gly Gln Ser Met Phe Thr Cys Arg Val Asp His Arg
   305                 310                 315                 320
   Gly Leu Thr Phe Gln Gln Asn Ala Ser Ser Met Cys Val Pro Asp Gln
                   325                 330                 335
   Asp Thr Ala Ile Arg Val Phe Ala Ile Pro Pro Ser Phe Ala Ser Ile
               340                 345                 350
   Phe Leu Thr Lys Ser Thr Lys Leu Thr Cys Leu Val Thr Asp Leu Thr
           355                 360                 365
   Thr Tyr Asp Ser Val Thr Ile Ser Trp Thr Arg Gln Asn Gly Glu Ala
       370                 375                 380
   Val Lys Thr His Thr Asn Ile Ser Glu Ser His Pro Asn Ala Thr Phe
   385                 390                 395                 400
   Ser Ala Val Gly Glu Ala Ser Ile Cys Glu Asp Asp Trp Asn Ser Gly
                   405                 410                 415
   Glu Arg Phe Thr Cys Thr Val Thr His Thr Asp Leu Pro Ser Pro Leu
               420                 425                 430
   Lys Gln Thr Ile Ser Arg Pro Lys Gly Val Ala Leu His Arg Pro Asp
           435                 440                 445
   Val Tyr Leu Leu Pro Pro Ala Arg Glu Gln Leu Asn Leu Arg Glu Ser
       450                 455                 460
   Ala Thr Ile Thr Cys Leu Val Thr Gly Phe Ser Pro Ala Asp Val Phe
   465                 470                 475                 480
   Val Gln Trp Met Gln Arg Gly Gln Pro Leu Ser Pro Glu Lys Tyr Val
                   485                 490                 495
   Thr Ser Ala Pro Met Pro Glu Pro Gln Ala Pro Gly Arg Tyr Phe Ala
               500                 505                 510
   His Ser Ile Leu Thr Val Ser Glu Glu Glu Trp Asn Thr Gly Glu Thr
           515                 520                 525
   Tyr Thr Cys Val Val Ala His Glu Ala Leu Pro Asn Arg Val Thr Glu
       530                 535                 540
   Arg Thr Val Asp Lys Ser Thr Gly Lys Pro Thr Leu Tyr Asn Val Ser
   545                 550                 555                 560
   Leu Val Met Ser Asp Thr Ala Gly Thr Cys Tyr
                   565                 570
<210> 113
   <211> 219
   <212> PRT
   <213> Künstliche Sequenz
<220>
   <223> Beschreibung der künstlichen Sequenz: chimärer Antikörper Maus/Mensch
<400> 113
   Asp Ile Gln Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215

## Beschreibung

Die Erfindung betrifft Herstellungs- und Identifizierungsverfahren für Core-1 tragende Moleküle, Zellen, Viren und Bakterien.

Tumor- oder Krebserkrankungen sind Geschwuisterkrankuhgen, die eine örtlich umschriebene Zunahme des Gewebevolumens beschreiben. Im weiteren Sinne ist jede lokalisierte Anschwellung durch Ödeme, akute und/oder chronische Entzündungen, eine aneurysmatische Erweiterung oder auch eine entzündlich bedingte Organschwellung ein Tumor. Im engeren Sinne werden vor allem gewebllche Neubildungen wie Gewächse, Blastome und/oder Neoplaslen in Form eines spontanen, verschiedenartig enthemmten, autonomen und irreversiblen Überschußwachsturns von körpereigenem Gewebe, das in der Regel mit unterschiedlich ausgeprägtem Verlust spezifischer Zellen und Gewebefunktionen verbunden ist, als Tumorerkrankungen verstanden. Es ist möglich, Tumore nach ihrem biologischen Verhalten, aber auch nach einer histogenetischen Systematik bzw. nach klinischen oder pathologischen Befunden zu systematisieren.

Insbesondere im klinischen Bereich kann es erforderlich sein, Tumore möglichst frühzeitig und auch selektiv zu erkennen, da eine frühzeitige Erkennung und die dann folgende Behandlung bzw. Entfernung sicherstellt, daß die Geschwulst erfolgreich behan deit werden kann, ohne daß die befallenen Organstrukturen bzw. Genabschnitte deformiert werden, wobei weiterhin verhindert werden kann, daß sich Metastasen ausbilden. Auch bei den Folgeuntersuchungen nach einer Krebsbehandlung müssen kleinste Metastasen frühzeitig detektiert werden, um die weitere Nachbehandlung zu optimieren. Für weite Bereiche der Arbeitsmedizin ist es außerdem notwendig, zu bestimmen, ob ein Gewebe oder ein Organ eine potentielle Krebsanfälligkeit zeigt, ohne daß das Organ bzw. das Gewebe bereits selbst entartet bzw. transformiert ist.

Die älteste und zugleich einfachste und zum Teil auch noch heute mit Erfolg angewendete Methode, einen Tumor zu erkennen, ist das Tasten und Schauen. So ist z.B. das Mammakarzinom bzw. das Prostatakarzinom als Knoten ertastbar. Hinweise auf Hautkrebs sind durch auffällige Muttermale durch den Arzt oder den Patienten selbst optisch zu detektieren. Andere optische Verfahren sind beispielsweise die bildgebenden Methoden. Hier werden mit Hilfe von Apparaten Bilder vom Körper aufgenommen, auf denen ein Tumor erkennbar ist. Zu diesen Methoden zählen zum Beispiel Röntgenbestrahlung wie auch die Computer-Tomographie (CT). Bei diesen Verfahren wird der Körper mit energiereicher Strahlung durchleuchtet, wobei die entarteten Gewebestrukturen aufgrund der veränderten Durchlässigkeit für diese Strahlung im Vergleich zum gesunden Gewebe erkennbar sind. Häufig werden bei diesen Methoden Kontrastmittel verwendet, die in entsprechende Regionen gespritzt werden und die Absorption erhöhen. Außerdem ist die Krebsdiagnose mittels Ultraschall sowie durch die Verwendung radioaktiv markierter Antikörper möglich, wobei die tumortypischen Antigene an die zu untersuchenden Organe binden und so die Tumore innerhalb des bitdgebenden Verfahrens erkennbar machen. Neben den bildgebenden Methoden sind Laboruntersuchungen ein weiteres wichtiges Mittel zur Krebsfrüherkennung. Dabei werden Proben von Urin, Blut oder auch Gewebeproben auf Abnormitäten untersucht. Dies kann beispielsweise eine veränderte Zusammensetzung dieser Proben sein, aber auch das Auftreten von Substanzen, die normalerweise nicht oder nur in geringen Mengen vorkommen. Diese Substanzen werden allgemein als Tumormarker bezeichnet. Sie werden entweder vom Tumorgewebe selbst produziert oder als Reaktion des Körpers auf den Tumor gebildet. Als Tumormarker werden neben Substanzen auch zelluläre Veränderungen bezeichnet, deren qualitative oder quantitative Analyse eine Aussage Ober das Vorliegen, den Verlauf oder eine Prognose von bösartigen Erkrankungen ermöglicht. Tumormarker sind meist physiologisch vorkommende bzw. modifizierte Substanzen, die gegenüber physiologischen Bedingungen oder der normalen genotyplschen/phänotyplschen Ausprägung im Urin, Serum oder anderen Körperflüsslgkeiten in erhöhter oder erniedrigter Konzentration oder in oder auf Tumorzellen nachweisbar sind, wobei diese Substanzen vom Tumorgewebe synthetisiert und/oder sezemiert und folgend durch Tumorzerfall freigesetzt oder als Reaktion des Organismus auf einen Tumor gebildet werden. Es ist eine Vielzahl von Tumormarkern beschrieben worden, deren Einsatz insbesondere beim Dickdarmkrebs, Brustkrebs, Eierstockkrebs, Prostata- und Hodenkrebs und beim kleinzeiligen Lungenkarzinom als sinnvoll gift. Zu diesen Krebsmarkem gehört beispielsweise das CEA, CA 15-3, CA 125, Alpha-Fetoprotein, HCG, das prostataspezifische Antigen, die neuronspezifische Enolase, CA 19-9 und SCC.

Die genannten Marker zeigen durch eine Erhöhung im Serum oder in Geweben oder durch ihr Vorhandensein als modifizierte Proteine, Liplde und/oder Kohlenhydrate zum einen beispielsweise (I) entzündliche Erkrankungen, Darmpolypen, Virusentzündungen aber insbesondere auch (II) Zirrhosen, Entartungen, Tumore und Metastasen an. Ein Großteil dieser Marker besteht aus Molekülen, die Protein- als auch Kohlenhydratstrukturen, ggf. Lipide umfassen. Je geringer der Proteinantell und demgemäß je größer der Kohlenhydrat- oder Lipidartteil dieser Marker ist, um so schwieriger können diese beispielsweise mit Erkennungsmolokülen, wie z.B. Antikörpern, detektiert werden. Bisher wurden durch Immunisierung von Mäusen mit Hilfe der Hybridom-Technologle verschiedene Antikörper gegen Kohlenhydratstrukturen hergestellt

Die Krebsdiagnostik mit Erkennungsmolekülen weist mehrere Nachteile auf. So können bestimmte Tumormarker auch bei nichtkanzerogenen Krankheiten auftreten, wodurch die eingesetzten Erkennungsmoteküle eine positive Reaktion anzelgen; weiterhin bedeutet eine Nichtinteraktion der Erkennungsmoleküle nicht, daß keine Tumorkrankheit vorhanden ist. Ein weiterer Nachteil ist, daß die bekannten Erkennungssubstanzen in der Regel unspeztfisch sind. Das bedeutet, daß ein positiver Nachweis nur in seltenen Fällen auf eine bestimmte Art der Tumorerkrankung weist Ein weiterer, ganz entscheidender Nachteil der bekannten Erkennungsmoleküle ist außerdem, daß sie nur bedingt zur Verlaufskontralle der Entwicklung von Tumoren, beispielsweise nach einer Operation, verwendet werden können. Das heißt, die bekannten Tumormarker können in der Regel nicht zur Früherkennung oder zur Nachbehandlung, insbesondere nicht zur Prophylaxe eingesetzt werden.

Neben diesen allgemeinen Nachteilen treten bei Erkennungsmolekülen, die gegen Kohlenhydratstrukturen gerichtet sind, spezielle Nachteile auf. Die Immunisierung mit Kohlenhydratantigenen führt meist nur zu einer primären IgM-Antwort bzw. die Immunantwort bleibt vollständig aus, da viele Kohlenhydretstrukturen auch Autosntlgene sind. Weil Kohlenhydrate T-Cell-unabhängige Antigene sind, die nicht in der Lage sind, einen Klassenswitch und die damit verbundene Reifung durch somatische Mutationen hervorzurufen, bleibt die Antikörperantwort meist auf die IgM-Klasse beschränkt. Aufgrund der generell schwachen Wechselwirkung und der notwendigen Multivalenz ist es deshalb schwierig, Antikörper mit hoher Affinität herzustellen. Ein Problem bei Antikörpern gegen Kohlanhydratstrukturen sind nicht nur die geringe Affinität, sondern auch die Spezifität Besonders gegen kurze nichtgeladene Kohlenhydratstrukturen ist es äußerst schwierig, spezifische Antikörper herzustellen, wobei eine gewisse Spezifität in vielen Fällen auch nur dann erreicht wird, wenn die Kohtenhydratstruktur auf einem bestimmten Träger lokalisiert ist So erkennt beispielsweise der Antikörper JAA/F11. der gegen Galβ1 →GalNAc gerichtet ist, nicht nur dieses Antigen selbst, sondern auch GlcNAcβ1→6Galβ1→3-(GlcNAcβ1→6) GalNAc sowie, wenn auch mit geringerer Avidität, Galβ1-3GlcNAc. Auch die neueren Möglichkeiten der Gewinnung von Erkennungsmoleküien durch verschiedene Formen der kombinatorischen Techniken, wie beispielsweise der Phagendisplay-Technologie, lösen die genannten Nachteile nicht. Auch hier bleibt das Problem der schwachen Erkennungsmoiekül-Kohlenhydrat-Interaktion. Zu berücksichtigen ist hierbei insbesondere, daß die durch immunisierung milstgewonnenen primären IgM-Antikörperfürden therapeutischen Einsatz zu groß sind. Ein weiterer Nachteil derbekannten Erkennungsmoleküle gegen Tumormarker ist der, dass sieden Tumorerst erkennbar machen, wenn dieser eine kritische Größe bereits erreicht hat. Das heißt, frühe Stadien des Tumorwachstums können mit den bekannten Erkennungsmolekülen, die gegen Tumormarker gerichtet sind, nicht bestimmt werden.

Ein weiterer Nachteil der bekannten Erkennungssubstanzen ist, daß sie nicht 'funktional' eingesetzt werden können. Funktional bedeutet, daß die Erkennungsmoleküle nicht nurso an die Tumormarker binden, daß diese detektiert werden, sondern daß sie somit über marker mit der Tumorzelle wechselwirken, daß die Tumorzelle in ihrem Wachstum beeinträchtigt wird. Es ist möglich, daß die Erkannungemoleküle mit bestimmten Tumormarkem, die beispielsweise auf Tumorzelloberflächen immobilisiert sind, so spezifisch interagieren, daß der durch die Tumormarker charakterisierte Tumor therapeutisch behandelt wird. Diese funktional aktiven Erkennungsmoleküle sind zum einen in der Lage, tumorzellenassoziierten Tumormarker zu detektieren und glelchzeitig durch ihre Bindung an diese tumorspezifische Struktur die Tumorzelle an einem weiteren Wachstum oder an einer Ausbildung von Metastesen zu hindern. Die bekannten Erkennungosmoleküle sind nachteilhafterweise in nur wenigen Fällen in der Lage, das Tumorwachstum zu beeinflussen. In der Regel müssen daher zusätzlich Substanzen, die das Tumorwachstum einschränken bzw. Inhibieren, an den Antikörper gekoppelt werden, so daß dieser die "Fähre" dieser Substanz ist, aber nicht das Agens der Behandlung.

Ein Aspekt der vorliegenden Erfindung ist ein Verfahren zur Identifizierung und/oder Isolierung und/oder Gewinnung eines Core-1 tragenden Moleküls, einer Core-1 tragenden Zelle, eines Core-1 tragenden Virus, eines Core-1 tragenden Bakteriums, oder von Core-1 tragenden Teilen von Zellen durch Bindung an ein Core-1 spezifisches Erkennungsmolekül, wobei das Erkennungsmolekül eine Aminosäuresequenz ümfasst, die
(i) die Aminosäuresequenz SEQ ID NO. 1 oder eine zu SEQ ID NO. 1 mindestens 80% homologe Aminosäuresequenz, und
(ii) die Aminosäuresequenz SEQ ID NO. 2 oder 3 oder eine zu SEQ ID NO. 2 oder 3 mindestens 80% homologe Aminosäuresequenz, und
(iii) die Aminosäuresequenz SEQ ID NO. 4, 5 oder 6 oder eine zu SEQ ID NO. 4, 5 oder 6 mindestens 80% homologe Aminosäuresequenz
enthält und das Antigen Core-1 spezifisch bindet.

Die Definitionen der Begriffe, die im folgenden gemacht werden, treffen mutatis mutandis auf zuvor gemachte, diese und dee nachfolgenden Ausführungen zu.

Unter dem Begriff Erkennungsmolekül versteht man erfindungsgemäß ein Molekül, das, insbesondere unter stringenten Bedingungen, die Kohlenhydratstruktur Core-1 spezifisch bindet

Unter Core-1 versteht man erfindungsgemäß die Kohlenhydratstruktur Galβ1-3GalNAc, die als α-homer (Galβ1-3GalNAcα) oder β-Anomer (Galß1-GalNAcβ) vorilegen kann. Bevorzugt ist hier die α-anomere Variante. Die erfindungsgemäßen Erkennungsmoleküle können aber auch nur das alpha-Anomer Galβ1-3GalNAcα oder beide Anomere Galβ1-3GalNAcα und Gelβ1-SGalNAcβ in gleicher Weise binden.

Unter einer spezifischen Bindung gegen Core-1 versteht man erfindungsgemäß eine Bindung, die nur Core-1, bevorzugt das α-Anomer, erkennt oder die Cord-1 und Core-2 (Galß1 -3(GicNAcß1-6)GalNAcα) erkannt Die Erkennungsmoleküle zeigen dabei keine Kreuzreaktivität mit anderen Derivaten und Anomaren dieser Kohlenhydratstrukturen wie sie in Beispiel 7 aufgeführt sind. Die Erkennungsmoleküle interagleren nicht mit Galαl-3GalNAcα, Galα1-3GalNAcβ, GaNAcα, Neu5Acα2-3Galβ1-3GalNAcα, Galβ1-3(Neu5Acα2-6)GalAcα, GlcNAcβ1-2Galβ1-3GalNAcα, GlcNAca1-3Galβ1-3GalNAcα, GalNAcα1-3Galβ und 3'-O-Su-Galß1-3GalNAcα unter den in Beispiel 7 beschriebenen Bedingungen. Die Bestimmung erfolgt dabei insbesondere Ober Spezifitätstests mit definierten synthetischen Kohlenhydratstruktufen.

In einer bevorzugten Ausführungsform umfasst ein Erkennungsmolekül, das das Antigen Core-1 spezifisch bindet:
a) eine erste Aminosäuresequenz, die die Aminosäuresequenz SEQ ID Nr. 1 und die Aminosäuresequenz SEQ ID Nr. 2 oder 3 und die Aminosäuresequenz SEQ ID Nr. 4 oder 5 oder 6 enthält; und
b) eine zweite Aminosäuresequenz, die die Aminosäuresequenz SEQ ID Nr. 7 oder 8 oder 9 und die Aminosäuresequenz SEQ ID Nr. 10 oder 11 und die Aminosäuresequenz SEQ ID Nr. 12 oder 13 enthält

Die erste und zweite Aminosäuresequenz können dabei auf einem oder mehreren, dort bevorzugt zwei, Polypeptiden vorkommen.

Die Core-1 bindenden Erkennungsmoleküle sind dadurch charakterisiert, dass sie ein definiertes Set von einzelnen Aminosäuresequenzen beinhalten. Die Aminosäuresequenz dieser Erkennungsmoleküle enthält ein oder zwei Tripletts definierter Sequenzen. Diese Sequenzen stellen die Bindungsdomänen dar und definieren die Spezifität des Erkennungsmoleküls. Das 1-Triplett-Erkennungsmolekül enthält die Aminosäuresequenz SEQ ID NO. 1, die Aminosäuresequenz SEQ ID NO. 2 oder 3 und die Aminosäuresequenz SEQ ID NO. 4 oder 5 oder 6. Cord-1-spezifische Erkennungsmoleküle, die durch zwei Tripletts definiert sind, enthalten für das erste Triplett die Aminosäuresequenz SEQ ID NO. 1, die Aminosäuresequenz SEQ ID NO. 2 oder 3 und die Aminosäuresequenz SEQ ID NO. 4 oder 5 oder 6 und für das zwelte Triplett die Aminosäuresequenz SEQ ID NO. 7 oder 8 oder 9, die Aminosäuresequenz SEQ ID NO. 10 oder 11 und die Aminosäuresequenz SEQ ID NO. 12 oder 13. Dabei können das erste und zweite Triplett entweder auf einer oder mehreren Polypeptidketten vorkommen, die im letzteren Fall gemeinsam das bindende Erkennungsmolekül bilden. Im Weiteren werden diese Tripletts im Sinne der Erfindung als Triplettsequenz 1 für die erste umfasste Aminosäuresequenz und als Triplettsequenz 2 für die zweite umfasste Aminosäuresequenz, siehe Definition a) und b) der obigen Beschreibung, bezeichnet Das Erkennungsmolekül kann erfindungsgemäß ein Antikörper sein, insbesondere ein muriner, chimärer oder humaner IgG oder IgM, eine scFv-Struktur oder eine andere. Aminosäuren mit analogen physlkochemischen Eigenschaften im Sinne der Erfindung können in 6 verschiedene Gruppen zusammengefaßt werden und sind in Tabelle 1 dargestellt

**Tabelle 1: Aminosäuren mit analogen physikochemlschen Eigenschaften unberücksichtlgt der molekularen Größe. Elgenschaft oder Aminosäure**

| funktionelle Gruppe | |
|---|---|
| aliphatisch | Glycin |
| | Alanin |
| | Valin |
| | Leucin |
| | Isoleucin |
| Hydroxy-Gruppe | Serin |
| | Threonin |
| Carboxy-Gruppe | Asparaginsäure |
| | Glutaminsäure |
| Amid-Gruppe | Asparagin |
| | Glutamin |
| Amino-Gruppe | Lysin |
| | Arginin |
| aromatisch | Phenylalanin |
| | Tyrosin |
| | Tryptophan |

In einer weiter bevorzugten Ausführungsform der Erkennungsmoleküle, die Cora-1 spezifisch binden, ist mindestens eine Aminosäuresequenz der Aminosäuresequenzen SEQ ID NO. 1, 2, 3, 7, 8 und/oder 9 durch kanonische Strukturvarianten bzw. äquivalente Strukturen mit den Aminosäuresequenzen SEQ ID NO. 14 bis 45 ersetzt, wobei die SEQ ID NO. 1 durch eine Sequenz der Sequenzen SEQ ID NO. 14 bis 17 (CDRH1), die SEQ ID NO. 2 oder 3 durch eine Sequenz der Sequenzen SEQ ID NO. 18 bis 27 (CDR2) und die SEQ ID NO. 7 oder 8 oder 9 durch eine Sequenz der Sequenzen SEQ ID NO. 28 bis 45 (CDRL1) ersetzt ist

Der generelle Zusammenhang zwischen einer Aminosäuresequenz und der Tertiärstruktur der von diesen Sequenzen gebildeten Loops ist dem Fachmann bekannt und wurde ausführlich untersucht [Rooman et al.,1989; Martin, Thomton, 1996]. Ein spezielles Beispiel stellen die Immunglobuline dar. Durch Analyse der Loop-Konformationen der hypervariablen Regionen (complementarity determining regions, CORs) in der leichten und der schweren Kette von Antikörpermoleküien wurden sogenannte kanonische Klassen definiert [Chothia, Lesk,1987; Chothia et al., 1986, 1989, 1992; Wu, Cygler, 1993], Auf dieser Grundlage wurden die kanonischen Strukturvarienten SEQ ID NO. 14 bis 46 der SEQ ID NO 1, 2, 3, 7, 8 und 9 abgeleitet.

Die Aminosäuresequenzen SEQ ID NO. 1 bis 13 oder deren Modifikationen in einem Code-1 spezifischen Erkennungsmolekül im Sinne der Erfindung bilden räumliche Strukturen aus, beispielsweise so genannte Loops, die dadurch gekennzeichnet sind, dass sie eine definiertere Tertiärstruktur und/oder Quartärstruktur besitzen. Die Bindungsregion eines Erkennungesmoleküls mit dem Core-1 Antigen wird von Aminosäureresten gebildet, die von bis zu sechs variablen Loops an der Oberfläche des Moleküls bereitgestellt werden, und die spezifisch mitCore-1 interagieren.

In einer weiteren Ausführungsform umfassen die Erkennungsmoleküle mindestens eine der Aminosäuresequenzen der SEQ ID NO. 1 bis 13 oder deren oben beschriebene Varianten doppelt oder mehrfach, wobei diese Dopplungen auch als Varianten der gleichen Aminosäuresequenz vorkommen können. Alle die in diesem Abschnitt beschriebenen Erkannungsmoleküle erkennen vorteilhafterweise das Antigen Core-1 spezifisch. Im folgenden werden auch diese Erkennungsmoleküle, die streng genommen aufgrund des Vervielfältigen von Sequenzen keine Tripiettsequenzen tragen, trotzdem als Tripiettsequenz 1 oder Triplettsequenz 2 bezeichnet, um die Anschaulichkeit zu vereinfachen.

In einer weiteren Ausführungsform, umfassen die Erkennungsmoleküle, die das Core-1 Antigen spezifisch binden, Aminosäuresequenzen, die eine Homologie von mindestens 80%, bevorzugt 90% gegenüber den Sequenzen SEQ ID NO. 1 bis 13 aufweisen.

Die Erkennungsmoleküle im Sinne der Erfindung können weiterhin Gerüstsequenzen umfassen, die die umfassenden Aminosäuresequenzen Aminosäuresequenz SEQ ID NO. 1 und die Aminosäuresequenz SEQ ID NO. 2 oder 3 und die Aminosäuresequenz SEQ ID Nr. 4 oder 5 oder 6, oder deren oben beschriebene Varianten, voneinander trennen, und Gerüstsequenzen, die die Aminosäuresequenzen SEQ ID Nr. 7 oder 8 oder 9 und die Aminosäuresequenz SEQ ID Nr. 10 oder 11 und die Amlnasäuresequenz SEQ ID Nr. 12 oder 13, oder deren oben beschriebene Varianten, voneinander trennen. Die erste und zweite Aminosäuresequenz können dabei auf einem oder mehreren, bevorzugt zwei, Polypeptidketten vorkommen. Diese Gerüstsequenzen werden im Sinne der Erfindung als Spacer oder Frameworksequenzen bezeichnet und, können unterschiedliche Längen und Sequenzen haben. Dabei sind ebenfalls solche Erkennungsmoleküle ausdrücklich mit eingeschlossen, bei denen nicht alle Aminosäuresequenzen der SEQ ID Nr. 1 bis 13 oder deren oben beschriebenen Varianten durch Spacer getrennt werden. Darüber hinaus haben die Erkennungsmoleküle vorzugsweise weitere flankierende Aminosäuresequenzen, die im Sinne der Erfindung auch als Gerüstsequenzen bezeichnet werden.

Die Gerüstsequenzen haben insbesondere die Aufgabe, die beschriebenen Aminosäuresequenzen, die für dieCore-1 spezifische Bindung der Erkennungsmoleküle verantwortlich beziehungsweise beteiligt sind, in eine geeignete Anordnung und räumliche Struktur zu bringen, damit die Bindung an das Cord-1 erfolgen kann. Es kann vorgesehen sein, dass die Aminosäuresequenzen SEQ ID NO. 1 bis NO. 13 ohne mindestens eine zusätzliche Aminosäuresequenz als Gerüstsequenz das Core-1 Antigen im Sinne der Erfindung nicht spezifisch binden können. Darüber hinaus können die Gerüstsequenzen den Erkennungsmolekülen z.B. die notwendige biologische und chemische Stabilltät geben, damit die räumliche Struktur effektiv aufgebaut und für die Funktion und Anwendung in einer geeigneten funktioneilen Form, die die Core-1 Bindung beinhaltet, erhalten werden kann.

In einer bevorzugten Ausführungsform werden die Triplettsequenzen in bestehende Proteine durch Austausch von Aminosäuresequenzen und/oder durch Hinzufügung eingefügt, wobei die bestehenden Proteinsequenzen als Gerüstsequenzen im Sinne der Erfindung dienen, beziehungsweise Gerüstsequenzen aus geeigneten Proteinen entnommen sind. Dabei können diese Gerüstsequenzen beispielsweise durch Mutationen, Deletionen oder Insertionen verändert werden. Hierbei bedient man sich dem Fachmann an sich bekannter Methoden der Molekularbiologie, Biochemie und Protein-Engineering. Bevorzugte Proteine hierfür sind Proteine der Immunglobulin-Superfamilie, Protease-Inhibitoren, Lektine, Helix-Bündel-Proteine und Lipocaline, wie sie z.B. offenbart sind in: Nygren und Uhlen, 1997; Nuttall SD et al., 1999 und Skerra, 2000.

In einer weiter bevorzugten Ausführungsform sind die Gerüstsequenzen Antikörpergerüstsequenzen aus einer oder verschiedenen Spezies oder Aminosäuresequenzen, die die Consensussequenz der Gerüstseuenzen muriner, humaner und/oder Antikörper anderer Säuger nachahmen. Eine Consensussequenz ist eine ideallsierte Sequenz, in derrepräsentativ an jeder Position die am meisten vorkommende Aminosäure steht, wenn viele existierende Sequenzen, beispielsweise aus Antikörper-Datenbanken, miteinander verglichen werden. Dabei sind die hier bevorzugten Erkennungsmoleküle dadurch gekennzeichnet dass die Gerüstsequenzen für die erste Triplettsequenz 1 umfassend die Aminosäuresequenz SEQ ID NO. 1, die Aminosäuresequenz SEQ ID NO. 2 oder 3 und die Aminosäuresequenz SEQ ID NO. 4 oder 5 oder 6, oder deren oben beschriebene Varianten, Antikörpergerüstsequenzen der variablen schweren Kette VH, die in der Literatur auch als Framework-Sequenzen bezeichnet werden, sind und die Gerüstsequenzen für die Triplettsequenz 2 umfassend die Aminosäuresequenz SEQ ID NO. 7 oder 8 oder 9, die Aminosäuresequenz SEQ ID NO. 10 oder 11 und die Aminosäuresequenz SEQ ID NO. 12 oder 13, oder deren oben beschrieben Varianten, Antikörpergerüstsequenzen der variablen leichten Kette VL sind.

Bevorzugt sind weiterhin Antikörpergerüstsequenzen von Antikörpern aus Säugetieren, besonders bevorzugt sind Antikörpergerütsequenzen humanen und/oder murinen Ursprungs. Die Gerüstsequenzen können dabei aus Antikörpergerüstsequenzen verschiedener Spezies kombiniert werden. Diese Antikörpergerüstsequenzen sind dem Fachmann bekannt und in verschiedenen Datenbanken zugänglich wie der Kabat-Datenbank (immuno.bme.nwu.edu) oder der Datenbank des National Center for Biotechnology Information (www.ncbl.nlm.nih.gov). Ebenfalls können diese Antikörpergerüststrukturen durch weitere Aminosäuren veriängert, und/oder durch eine oder mehrere Mutationen, z.B. Deletionen und/oder Insertionen, verändert werden, wobei die spezifische Bindung an Core-1 erhalten bleibt.

Werden in einer bevorzugten Variante der Erfindung die Triplettsequenzen mit Antikörpergerüstsequenzen kombiniert, stellt das Erkennungsmolekül eine variable Kette eines Antikörpers oder eine davon abgeleitete Struktur dar.

Besonders bevorzugte Antikörpergerüstsequenzen als Gerüstsequenzen im Sinne der Erfindung sind für die variable schwere Kette die Aminosäuresequenzen entsprechend FRH1, FRH2, FRH3 und FHR4 in Tabelle 2 und für die variable leichte Kette die Aminosäuresequenzen entsprechend FRL1, FRL2, FRL3 und FRL4 in Tabelle 2, wobei die Aminosäuresequenzen der Triplettsequenzen 1 und 2 mit den SEQ ID NO. 1 bis 13 den entsprechenden CDR-Regionen der Antikörper entsprechen. Dabei setzen sich die variable schwere (VH) bzw. leichte (VL) Antikörperkette wie folgt zusammen: die VH: FRH1-CDRH1-FRH2-CDRH2-FRH3-CDRH3-FRH4 und die VL: FRL1-CDRL1-FRL2-CDRL2-FRL3-CDRL3-FRL4. Tabelle 2 erläutert die Positionen im Detail. Die Positionen der einzelnen Aminosäuren bzw. Aminosäuresequenzen entsprechen der Nummerierung von Aminosäuren in Antikörpermolekülen nach Kabat.

**Tabelle 2:**

| **Name** | **Positionsbereich** | **Pos.** | **Amimosäure bzw. Aminosäuresequenz** |
|---|---|---|---|
| FRH1 | 1 bis 30 | 1 | Q oder E |
| | | 2 | V |
| | | 3 | Q, K oder T |
| | | 4 | L |
| | | 5 | K oder V |
| | | 6 | E oder Q |
| | | 7 | s |
| | | 8 | G |
| | | 9 | A |
| | | 10 | E |
| | | 11 | L oder V |
| | | 12 | V oder K |
| | | 13 | R oder K |
| | | 14 | P |
| | | 15 | G |
| | | 16 | T oder A |
| | | 17 | S |
| | | 18 | V |
| | | 19 | K |
| | | 20 | I oder V |
| | | 21 | S oder P |
| | | 22 | C |
| | | 23 | K |
| | | 24 | A, V, S oderT |
| | | 25 | s |
| | | 26 | G |
| | | 27 | Y, F, S oder D |
| | | 28 | T |
| | | 29 | F, L oder I |
| | | 30 | T |
| CDRH1 | 31 bis 35 | | SEQ ID NO. 1 und Varianten |
| FRH2 | 36 bis 49 | 36 | W |
| | | 37 | V |
| | | 38 | K oder R |
| | | 39 | Q |
| | | 40 | R oder A |
| | | 41 | P |
| | | 42 | G |
| | | 43 | H oder Q |
| | | 44 | G |
| | | 45 | L |
| | | 46 | E |
| | | 47 | W oder R |
| | | 48 | I oder M |
| | | 49 | G |
| CDRH2 | 50 bis 65, wobei zusätzlich die Pos. 52a eingeführt ist | | SEQ ID NO. 2 oder 3 und Varianten |
| FRH3 | 66 bis 94 | 66 | K oder R |
| | | 67 | A oder V |
| | | 68 | T |
| | | 69 | L oder M |
| | | 70 | T |
| | | 71 | A, L oder T |
| | | 72 | D |
| | | 73 | T |
| | | 74 | S |
| | | 75 | S oder T |
| | | 76 | s |
| | | 77 | T |
| | | 78 | A |
| | | 79 | Y |
| | | 80 | M |
| | | 81 | Q oder E |
| | | 82 | L |
| | | 82a | S |
| | | 82b | S oder R |
| | | 82c | L |
| | | 83 | T oder R |
| | | 84 | S |
| | | 85 | E |
| | | 86 | D |
| | | 87 | S oder T |
| | | 88 | A |
| | | 89 | V |
| | | 90 | Y |
| | | 91 | F oder Y |
| | | 92 | C |
| | | 93 | A |
| | | 94 | Y, K oder R |
| CDRH3 | 95 bis 102, wobei zusätzlich die Pos. 100a und 100b eingeführt sind | | SEQ ID NO. 4, 5 oder 6 und Varianten |
| FRH4 | 103 bis 113 | 103 | W |
| | | 104 | G |
| | | 105 | Q |
| | | 106 | G |
| | | 107 | T |
| | | 108 | T, S oder L |
| | | 109 | V oder L |
| | | 110 | T |
| | | 111 | V |
| | | 112 | S |
| | | 113 | S oder A |

| **Name** | **Positionsbereich** | **Pos.** | **Aminosäure bzw. Aminosäuresequenz** |
|---|---|---|---|
| FRL1 | 1 bis 23 | 1 | D |
| | | 2 | I, V oder L |
| | | 3 | Q oder L |
| | | 4 | M |
| | | 5 | T |
| | | 6 | Q |
| | | 7 | T oder S |
| | | 8 | P |
| | | 9 | L |
| | | 10 | S |
| | | 11 | L |
| | | 12 | P |
| | | 13 | V |
| | | 14 | S oder T |
| | | 15 | L oder P |
| | | 16 | G |
| | | 17 | D oder E |
| | | 18 | Q oder P |
| | | 19 | A |
| | | 20 | s |
| | | 21 | I |
| | | 22 | S |
| | | 23 | C |
| CDRL1 | 22 bis 34, wobei zusätzlich die Pos. 27a, 27b, 27c, 27d und 27e eingeführt sind | | SEQ ID NO. 7, 8 oder 9 und Varianten |
| FRL2 | 35 bis 49 | 35 | W |
| | | 36 | Y |
| | | 37 | L |
| | | 38 | Q |
| | | 39 | K |
| | | 40 | P |
| | | 41 | G |
| | | 42 | Q |
| | | 43 | S |
| | | 44 | P |
| | | 45 | K oder Q |
| | | 46 | L |
| | | 47 | L |

| **Name** | **Positionsbereich** | **Pos.** | **Amimosäure bzw. Aminosäuresequenz** |
|---|---|---|---|
| | | 48 | I oder V |
| | | 49 | Y |
| CDRL2 | 50 bis 56 | | SEQ ID NO. 10 oder 11 und Varianten |
| FRL3 | 57 bis 88 | 57 | G |
| | | 58 | V |
| | | 59 | P |
| | | 60 | D |
| | | 61 | R |
| | | 62 | F |
| | | 63 | S |
| | | 64 | G |
| | | 65 | S |
| | | 66 | G |
| | | 67 | S |
| | | 68 | G |
| | | 69 | T |
| | | 70 | D |
| | | 71 | F |
| | | 72 | T |
| | | 73 | L |
| | 74 | 74 | K |
| | | 75 | I |
| | | 76 | S |
| | | 77 | R |
| | | 78 | V |
| | 79 | 79 | E |
| | | 80 | A |
| | | 81 | E |
| | | 82 | D |
| | | 83 | L oder V |
| | | 84 | G |
| | | 85 | V |
| | | 86 | Y |
| | | 87 | Y |
| | | 88 | C |
| CDRL3 | 89 bis 97 | | SEQ ID NO. 12 oder 13 und Varianten |
| FRL4 | 98 bis 108 | 98 | F |
| | | 99 | G |
| | | 100 | G oder Q |
| | | 101 | G |
| | | 102 | T |
| | | 103 | K |
| | | 104 | L |
| | | 105 | E |
| | | 106 | I oder L |
| | | 106a | K |
| | | 107 | R |
| | | 108 | A |

Die Aminosäuresequenzen. SEQ ID NO. 46 bis 79 entsprechen Aminosäuresequenzen mit bevorzugten Gerüstsequenzen für die variable schwere Kette. Die Aminosäuresequenzen SEQ ID NO. 80 bis 94 entsprechen Aminosäuresequenzen mit bevorzugten Gerüstsequenzen für die variable leichte Kette.

Die zu verwendenden Techniken und Methoden zur Herstellung dieser Sequenzen sind dem Fachmann bekannt, ebenso ist der Fachmann in der Lage, geeignete Gerüstsequenzen und/oder Mutationen auszuwählen.

Im Sinne der Erfindung können die Core-1 spezifischen Erkennungsmoleküle in verschiedenen Formaten vorliegen. Die grundlegende Struktur des Erkennungsmoleküls sind eine oder mehrere Polypeptidketten, die oben beschriebenen erfindungsgemäßen Triplettsequenz 1 oder Triplettsequenzen 1 und 2 und Gerüstsequenzen umfassen. Beispielsweise sind die Aminosäuresequenz der variablen schweren Kette mit den Gerüstsequenzen und den Triplettsequenzen 1 und die Aminosäuresequenz der variablen leichten Kette mit den Gerüstsequenzen und den Triplettsequenzen 2 nicht-kovalent oder kovalent miteinander verknüpft, und können auf einer oder mehreren Polypeptidketten liegen. Mehrere Polypeptidketten können kovalent, beispielsweise durch Disulfidbrücken, oder nicht-kovalent verbunden als Erkennungsmolekül vorliegen.

Zu den unterschiedlichen Formaten der Erkennungsmoleküle gehören insbesondere die Verknüpfung der Triplettsequenzen mit Aminosäuresequenzen, die Ober die oben beschriebenen Gerüstsequenzen hinausgehen. In einer bevorzugten Variante umfassen daher Erkennungsmoleküle neben den Triplettsequenzen und den Gerüstsequenzen weitere Zusatzsequenzen. Zusatzsequenzen sind insbesondere Aminosäuresequenzen, die primär nicht der räumlichen Anordnung der Triplettsequenzen wie in Form der Gerüstsequenzen dienen, diese jedoch vorteilhaft durch sekundäre oder tertiäre Wechselwirkungen beeinflussen können. Beispielsweise stabilisieren Zusatzsequenzen in Form von konstanten Domänen eines Antikörpers den Antikörper und bewirken eine Dimerisierung, wodurch es zu einer verbesserten Bindung des Antikörpers kommt, oder beispielsweise bewirkt eine Fusion eines scFv mit einer Domäne eines Bakteriophagenhüllproteins eine Aktivitätssteigerung der scFv-Bindung, wie sie z.B. in Jensen KB et al., 2002 offenbart ist.

In einer bevorzugten Ausführungsform umfassen die Erkennungsmoleküle Aminosäuresequenzen mit Gerüstsequenzen auf Antikörperbasis und neben den Triplettsequenzen weitere Zusatzsequenzen. Die Zusatzsequenzen haben insbesondere mindestens eine der folgenden Aufgaben:
a) Verknüpfung einer Triplettsequenz mit ihren entsprechend geeigneten Gerüstsequenzen mit mindestens einer weiteren Triplettsequenz mit ihren entsprechend geeigneten Gerüstsequenzen, um beispielsweise eine Bindungsfähigkeit zu erzeugen oder zu verbessern;
b) der Stabilisierung der Domänen, beispielsweise durch einen Linker zwischen zwei Proteindomänen oder Aminosäuresequenzen, die mit anderen der gleichen oder einer zweiten Kette in Wechselwirkung treten;
c) Effektorfunktionen für immunologische Aufgaben, beispielsweise durch Fusion mit Fc-Tell von Antikörpern, Chemokinen, Cytokinen, Wachstumsfaktoren oder Teilen davon, oder Antikörpern mit einer anderen Spezifität oder Fragmenten davon, zur Rekrutlerung von Zellen des Immunsystems, beispielsweise Makrophagen, oder Teilen des Komplementsystems;
d) Fusion mit Tags, beispielsweise Multimerisierungssequenzen -zum Beispiel *µ*-tall-Sequenz aus IgM oder Assoziationsdomäne aus p53 oder MBL -zurMultimerisierung der Core-1 bindenden Anteile für eine multivalente Bindung oder zur Aufreinigung der Erkennungsmoleküle, beispielsweise His-Tag oder zum Nachweis, beispielsweise myc-Tag oder zur Markierung oder Chelatisierung von Erkennungsmolekülen, beispielsweise durch Lysin-reiche Sequenzen.

Geeignete Strukturen sind dem Fachmann bekannt oder durch logische Schlussfolgerung aus dem Stand der Technik abzuleiten.

Dabei weiter bevorzugte Ausführungsformen sind Erkennungsmoleküle, die folgende Formate umfassen: single chain Antikörperfragment (scFv), Fv-Fragment, Fab-Fragment, F(ab)₂-Fragment, Multibody (Dia-, Tria-, Tetrabody), Immunglobulin der Isotypen IgG, IgM, IgA, IgE, IgD oder deren Subklassen, beispielsweise IgG1, oder von Immunglobulinen abgeleitete Erkennungsmoleküle, die mindestens eine konstante Domäne umfassen.

In einer bevorzugten Ausführungsform sind die Erkennungsmoleküle aus einer schweren und einer leichten Polypeptidkette zusammengesetzt, wobei die Aminosäuresequenzen derschweren und leichten Kette jeweils eine der oben beschriebenen Triplettstrukturen umfassen, die die CDR Regionen des Antikörpers darstellen, die entsprechenden Antikörpergerüstsequenzen, die die Frameworksequenzen der Antikörper darstellen, und Zusatzsequenzen, die mindestens eine der konstanten Domänen des Antikörperisotyps umfassen. Die beiden Ketten können miteinander kovalente Bindungen eingehen. Die konstanten Regionen und variablen Regionen können dabei Sequenzen von Antikörpern aus einer oder verschiedenen Spezies enthalten. Es können Teile von konstanten Domänen oder ganze konstante Domänen deletiert oder mutiert sein, beispielsweise um die Effektorfunktion der Zusatzsequenzen zu verändem, beispielsweise die Bindung an Fc-Rezeptoren zu verhindern oder zu verbessern. In einer bevorzugten Ausführungsform ist das Erkennungsmolekül ein muriner, chimärisierter, humanisierter, partiell humaner oder humaner Antikörper oder Antikörperfragment. Die Chimärisierung erfolgt beispielsweise durch Verknüpfung dervariablen Antikörperdomänen mit konstanten Antikörperdomänen oder Fragmenten der konstanten Domäne von Antikörpern verschiedener Spezies. Bevorzugt sind Sequenzen der konstanten Domänen humaner Antikörper.

Die Antikörpergarüstsequenzen können so gewählt werden, dass die Sequenzen weitestgehend homolog zu humanen Antikörpersequenzen sind. Die Wahl für den Speziesursprung der Gerüstsequenzen hängt auch von der Anwendung ab. So werden für eine therapeutische Anwendung in bestimmten Bereichen möglichst große Anteile an humanen Gerüstsequenzen bevorzugt, vor allem dann, wenn eine human anti-Maus Antikörperantwort (HAMA) vermieden werden soll. In anderen therapeutischen Bereichen ist ein Xenoanteil vorteilhaft, da er das immunsystem in einer zusätzlichen Weise stimuliert. Eine Kombination beider ist in einigen Fällen besonders geeignet, vor allem dann, wenn in einer Erstimmunisierung ein Xenoanteil vorteilhaft und bei späteren Anwendungen ein spezieskonformer und damit humaner Anteil vorteilhaft ist.

Bevorzugt ist eine Homologie zu humanen Consensussequenzen, wobei für die variable schwere Kette die HuHI und für die variable leichte Kette die HuKII bevorzugt wird. Besonders bevorzugt ist eine Homologie zu humanen Keimbahnsequenzen, die dem Fachmann bekannt sind und zum Beispiel Ober die V BASE Datenbank (www.mrc-cpe.cam.ac.uk) zugänglich sind.

Die zu verwendenden Techniken und Methoden zur Herstellung dieser Sequenzen sind dem Fachmann bekannt, ebenso ist der Fachmann in der Lage, geeignete humane Sequenzen auszuwählen und/oder möglicherweise notwendige Mutationen der Sequenzen durchzuführen.

In einer weiteren Ausführungsform sind zusätzlich die Triplettsequenzen, die im allgemeinen den Bindungs-Loops (CDR-Regionen) entsprechen und die bevorzugt starke Homologlen zu den entsprechenden Sequenzbereichen in der humanen Keimbahnsequenz haben, diesen schrittweise durch einfache Mutationen angeglichen, ohne die spezifische Bindung an Core-1 zu beeinträchtigen. Erkennungsmoleküle mit diesen Sequenzen werden hier als partiell humane Antikörper oder Antikörperfragmente bezeichnet Bevorzugte humanisierte Sequenzen stellen z.B. die Sequenzen SEQ ID NO. 56 bis 79 bzw. SEQ ID NO. 85 bis 94 dar.

In einer weiteren bevorzugten Ausführungsform werden bestimmte Aminosäuren der Antikörpergerüstsequenzen einer Spezies durch andere ausgetauscht, um normalerweise weniger immunogene Regionen zu generieren. Dies beinhaltet dem Fachmann an sich bekannte Technologien, beispielsweise Technologien der Humanisierung, beispielsweise CDR-Grafting, Resurfacing, Chain-Shuffling mit Mutationen und Deimmunisierung durch Mutation oder Deletion von humanen MHC Epitopen.

In einer bevorzugten Ausführungsform handelt es sich um ein vom IgM abgeleitetes Erkennungsmolekül mit den entsprechenden konstanten Domänen eines IgM, bevorzugt humanen Sequenzen. Im Sinne der Erfindnung setzten sich Immunglobuline aus der schweren Kette und der leichten Kette eines Antikörpers zusammen, wobei bevorzugt 2 leichte und 2 schwere Ketten eine Einheit darstellen. Immunglobuline des IgM Typs bestehen meist aus 5 solchen Einheiten, die zusätzlich zu Disulfidbrücken durch die J-Kette miteinander verknüpft sind.

In einer besonders bevorzugten Ausführungsform ist die J-Kette nicht vorhanden, wobei es ebenfalls zur Multimerisierung der Untereinheiten kommt, wobei hier hexa- und pentamere Strukturen vorliegen können.

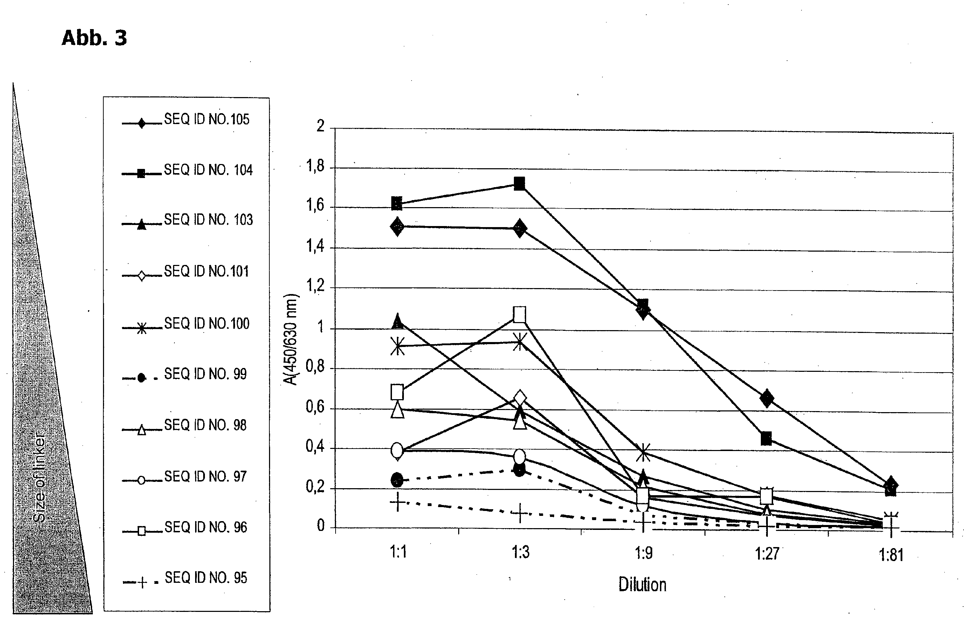

In einer bevorzugten Ausführungsform der Erkennungsmoleküle handelt es sich um Single chain Antikörperfragmente umfassend eine Triplettstruktur 1 mit entsprechenden oben beschriebenen Antikörpergerüstsequenzen, die die CDR Regionen des Antikörpers und Frameworksequenzen der variablen Domäne derschweren Kette von Antikörpern darstellen, und eine Triplettstruktur 2 mit den entsprechenden oben beschriebenen Antikörpergerüstsequenzen, die die CDR Regionen des Antikörpers und Frameworksequenzen der variablen Domäne der leichten Kette von Antikörpern darstellen, die kovalent miteinander in Form eines Fusionsproteins verknüpft sind. Hierbei sind die Sequenzen direkt oder durch einen Linker miteinander verknüpft. Bevorzugt sind hier scFv Formate ohne Linker oder mit einem Linker von 1 bis 9 Aminosäuren Länge. Diese scFv Antikörper bilden multimere Strukturen (beispielsweise Dia-, Tria-, Tetra-bodies), die im Sinne der Erfindung auch als Multibodies bezeichnet werden und zeigen aufgrund der Multivalenz höhere Avidität zum Core-1 Antigen, Es wurden Core-1 spezifische Erkennungsmoleküle im scFv Format mit verschiedenen Linkerlänge konstruiert (SEQ ID NO. 95 bis 106) und ihre Bindungscharakteristik im ELISA untersucht. Eine schrittweise Linkerverkürzung führte zu einer Erhöhung der Bindung an Asialoglykophorin, einem Core-1 tragenden Glykoprotein, wie in Abbildung 3 dargestellt. Die besten Bindungseigenschaften zeigten hierbei die Varianten mit der SEQ ID NO. 104 und 105. Diese multivalenten Konstrukte im Dia/triabody Format sind besonders bevorzugte Ausführungsformen der Erfindung und sind aufgrund verbesserter pharmakokinetischer Eigenschaften für die Tumortherapie von Vorteil.

In einer weiter bevorzugten Ausführungsform sind die Erkennungsmoleküle fusioniert, chemisch gekoppelt, kovalent oder nicht-kovalent assoziiert mit (i) Immunglobulindomänen verschiedener Spezies, (ii) Enzymmolekülen, (iii) Interaktionsdomänen, (iv) Signalsequenzen, (v) Fluoreszenzfarbstoffen, (vi) Toxinen, (vii) katalytischen Antikörpern, (viii) einem odermehreren Antikörpern oder Antikörperfragmenten mitanderer Spezifität, (ix) zytolytischen Komponenten, (x) Immunmodulatoren, (xi) Immuneffektoren, (xii) MHC-Klasse I oder Klasse II Antigenen, (xiii) Chelatoren zur radioaktiven Markierung, (xiv) Radiolsotopen, (xv) Liposomen, (xvi) Transmembrandomänen, (xvii) Viren und/oder Zellen. Außerdem können die Erkennungsmoleküle insbesondere mit einem Tag fusioniert sein, die die Detektion des Erkennungsmoleküls und deren Aufreinigung ermöglichen, wie zum Beispiel ein Myc-Tag oder ein His-Tag. Technologien zur Herstellung dieser Konstrukte sind dem Fachmann bekannt, ebenso ist der Fachmann in der Lage, geeignete Sequenzen und Komponenten auszuwählen und mit den erfindungsgemäßen Erkennungsmolekülen in geeigneter Weise zu verbinden.

In einer weiteren bevorzugten Ausführungsform sind die beschriebenen Erkennungsmoleküle auf Antikörper- oder Antikörperfragment-Basis mit Peptiden oder Proteinen, die nicht von Immunglobulinen abgeleitet sind, fusioniert. Bespielsweise wird die Multimerisierungsdomäne eines Nicht-Immunglobulinmoleküls mit einem scFv fusioniert, insbesondere das C-terminale Ende der alpha-Kette des C4 Bindungsproteins, wie es bei Tonye Libyh M. et al., 1997 beschrieben ist, und somit ein multivalentes Erkennungsmolekül konstruiert.

In einer weiteren Ausführungsform wird ein scFv mit einer Transmembrandomäne eines Nicht-Immunglobulinmoleküls fusioniert, beispielsweise mit derTransmembrandomäne des c-erb B2, des h-PDGFR, des humanen Transferrinrezeptors oder des humanen Asialoglykoprotein-Rezeptors (Liao et al., 2000), und somit die Expression von Bindungsmolekülen auf der Oberfläche von Zellen ermöglicht

Eine weitere bevorzugte Ausführungsform der Erfindung umfasst Erkennungsmoleküle, die weiterhin Aminosäuresequenzen umfassen, die spezifisch an Makrophagen oder andere Immuneffektorzellen binden. Beispielsweise umfassen die Erkennungsmoleküle weiterhin eine Antikörperbindungsstelle gegen CD64, wodurch es in Form eines bispezifischen Antikörpers beziehungsweise Antikörperfragments (Diabodies) zur Bindung von Makrophagen an Core-1 positive Tumorzellen kommt, was zu deren Bekämpfung und/oder Zerstörung führt.

Eine bevorzugte Ausführungsform betrifft radioaktiv markierte Core-1 spezifische Erkennungsmoleküle. Eine bevorzugte Form sind Erkennungsmoleküle auf der Basis von Antikörpern oder Antikörperfragmenten. Eine weitere bevorzugte Ausführungsform sind radioaktiv markierte Erkennungsmoleküle im single chain Format (einschließlich als Dia-, Tria-, Tetrabodies). Weitere bevorzugte Formen sind radioaktiv markierte single chain Antikörperfragmente und ganze Immunglobuline, beispielsweise chimäre oder humanisierte IgG oder IgM Antikörper oder humanisierte Antikörperfragmente. Die Erfindung ist selbstverständlich nicht auf diese Antikörper, die radioaktive Markierung und diese Formate der Antikörper beschränkt.

Antikörperfragmente wie die bevorzugten multivalenten scFv Fragmente insbesondere ohne oder mit sehr kurzem Linker bieten gegenüber intakten monoklonalen Antikörpern einen Vorteil für das Targeting von soliden Tumoren. Bei intakten Antikörpern, die in Blodistributionsstudien eine spezifische Anreicherung im Tumorareal zeigen, fällt bei genauer Untersuchung des Tumors eine inhomogene Antikörperverteilung mit vornehmlicher Anreicherung im Randbereich auf. Zentral gelegene Tumorantelle werden aufgrund von Tumornekrosen, Inhomogener Antigenverteilung sowie einem erhöhten interstitiellen Gewebedruck mit diesen Antikörperkonstrukten nicht erreicht. Kleinere Antikörperfragmente zeigen dagegen eine schnelle Tumormarkierung, dringen tiefer in den Tumor ein und werden gleichzeitig relativ schnell aus der Blutbahn entfernt. Die Dissoziationskonstante von monovalenten Antikörperfragmenten wie Fabs oder scFv ist allerdings oftmals zu niedrig, was in einer kurzen Verwelldauer an den Tumorzellen resultiert. Deshalb bieten multivalente Antikörperkonstrukte wie Multibodies (Diabodies, Trial/Tetrabodies), F(ab')₂ und andere Minibodies (multi-valente Antikörperkonstrukte bestehend aus der Bindungsdomäne und einer Multimerisierungssequenz, beispielsweise scFv und CH3 Domäne eines IgG) in der Tumortherapie viele Vorteile. Multivalenten Konstrukte im Dia/Triabody Format sind bevorzugte Ausführungsformen der Erfindung und sind aufgrund verbesserter pharmakokinetischer Eigenschaften für die Tumortherapie von Vorteil und wurden zur Verwendung in der Tumortherapie weiter entwickelt. Sie können als Vehikel für die spezifische Anreicherung von zum Beispiel zytotoxischen Substanzen wie Chemotherapeutika oder Radionuklide im Tumor verwendet werden. Durch geeignete Radionuklidwahl können Tumorzellen Ober eine Distanz von mehreren Zelldurchmessem abgetötet werden, wodurch auch Antigen-negative Tumorzellen in einem Tumorareal erfasst unddie schlechte Penetration der Antikörper in solide Tumoren zumindest teilweise ausgeglichen werden können.

Eine besonders bevorzugte Ausführungsform sind radioaktiv-markierten Multibodies -insbesondere wie unter Beispiel 9 näher ausgeführt -, die besonders vorteilhafte pharmakokinetischen Eigenschaften vereinen mit einer in der Kombination gegenüber ganzen Immunglobulinen und scFv verbesserten Tumorretention, Tumorpenetration, Serumhalbwertszeit und Serum zu Tumor-Vertellungsverhältnis. Weitere Vorteile sind die hohe Avidität und die bakterielle Expression, die es erlaubt, kostengünstig diese Erkennungsmoleküle herzustellen. Damit eignet sich dieses besondere Format der Erkennungsmoleküle vorteilhafterweise bevorzugt für die Behandlung von kleinen Primärtumoren, Metastasen und minimal residual Erkrankungen.

Eine bevorzugte Ausführungsform sind nicht radioaktiv markierte Erkennungsmoleküle. Eine bevorzugte Form hierbei sind Erkennungsmoleküle auf der Basis von Antikörpern oder Antikörperfragmenten.

Eine besonders bevorzugte Ausführungsform sind chimäre und humanisierte Immunglobuline auf der Basis von IgM Molekülen zur inhibition der Lebermetastasierung und zur Bekämpfung residualer Tumorzellen.

Weitere bevorzugte Ausführungsformen sind toxin- oder Zytostatika-gekoppelte chimärisierte oder humanisierte IgG und IgM basierte Erkennungsmoleküle und im Besonderen Multibodies (Dia- Tria-, Tetrabodies) mit besonders vorteilhaften pharmakokinetischen Eigenschaften wie oben ausgeführt

Eine weitere bevorzugte Ausführungsform sind Liposomen, die beispielsweise mit Toxinen oder Zytostatika beladen sind, und die auf ihrer Oberfläche Erkennungsmoleküle tragen.

Der Fachmann ist in der Lage, geeignete Radioisotope, Toxine und Zytostatika auszuwählen. Geeignete Techniken, Verfahren, Dosierungen und Formulierungen sind dem Fachmann bekannte

Eine weitere bevorzugte Ausführungsform sind Effektorzellen das Immunsystems, auf deren Oberfläche Erkennungsmoleküle gebunden sind, die die Effektorzellen zu Core-1 tragenden Tumorzellen dirigieren/adressieren- und dadurch deren Bekämpfung und/oder Zerstören vermitteln. Bevorzugte Effektorzellen sind Makrophagen, dendritische Zellen und NK-Zellen, die aus dem Patienten gewonnen werden und ex vivo mit den Erkennungemolekülen gekoppelt werden. Weiter bevorzugt sind Zelllinien dieser Zelltypen. Die Kopplung erfolgt beispielsweise durch bispezifische Erkennungsmoleküle, die neben den Core-1 spezifischen Anteilen weiterhin Aminosäuren umfassen, die eine Bindung an die Effektorzellen vermitteln. Beispielsweise sind dies bispezifische Antikörper, Komplementanteile oder konstante Domänen von Antikörpern.

Eine weiter bevorzugte Ausführungsform sind hierbei Makrophagen aus dem Patienten, die nach Gewinnung mit einem bispeziflschen Antikörper beispielsweise in Form ganzer Antikörper, bevorzugt chemisch gekoppelter Fab-Fragmente oderweiter bevorzugt Diabodies, die zum einen CD64 erkennen und zum anderen Core-1 spezifisch sind. Diese Makrophagen, die Ober die CD64 Spezifität die bispezifischen Erkennungsmoleküle tragen, werden dem Patienten in einer geeigneten Formulierung wieder zugeführt, um den Core-1 positiven Tumorzu bekämpfen. Die hierzu verwendeten Techniken und die geeigneten Verfahren. Dosierungen und Formulierungen sind dem Fachmann bekannt. Eine weiter bevorzugte Ausführungsform sind Makrophagen aus dem Patienten, die nach Gewinnung mit einem Core-1 spezifischen Antikörper oder Antiköperfragment, die den konstanten Teil eines Antikörpers umfassen, der an Makrophagen Ober die an sich bekannten Fc-Rezeptoren bindet. Dabei können die Erkennungsmoleküle entweder als ganze Antikörper, bevorzugt chimäre oder humanisierte IgG oder IgM, oder als Antikörperfragment, beispielsweise scFv, Fab oder Multibodies in Form eines Fusionsproteins oder chemisch gekoppelt mit dem dem Fachmann bekannten Teil der konstanten Domäne von Antikörpern, an die Makrophagen binden. Diese die Erkennungsmoleküle tragenden Makrophagen werden dem Patienten in einer geeigneten Formulierung wieder zugeführt, um den Core-1 positiven Tumorzu bekämpfen. Die hierzu verwendeten Techniken und die geeigneten Verfahren, Dosierungen und Formulierungen sind dem Fachmann bekannt.

Eine weiter bevorzugte Ausführungsform sind Zelllinien oder Zellen aus dem Körper wie die oben beschriebenen Effektorzellen, die mit Molekülen transfiziert werden, die Core-1 spezifische Erkennungsmoleküle und weiterhin Elemente umfassen, die eine Expression und eine Verankerung in der Membran bewirken, beispielsweise transmembrane Domäne, und die Aktivierung der Effektorzellen bei Kontakt mit einer Core-1 tragenden Tumorzelle vermitteln. Die entsprechenden Elemente sind dem Fachmann bekannt. Beispielsweise wird eine dendritische Zelllinie mit einem Vektor transfiziert, der ein Erkennungsmolekül umfasst, das ein scFv oder Multibody und eine Transmembrandomäne und eine aktivierende Domäne umfasst. In einem anderen Beispiel werden dazu Makrophagen viral transfiziert. Diese die Erkennungsmoleküle tragenden Effektorzellen werden einem Patienten in einer geeigneten Formulierung zugeführt um den Core-1 positiven Tumor zu bekämpfen. Die hierzu verwendeten Techniken und die geeigneten Verfahren, Dosierungen und Formulierungen sind dem Fachmann bekannt.

Nukleinsäuremoleküle, die ein oder mehrere genetische Sequenzen umfassen, können mindestens eines der oben beschriebenen Erkennungsmoleküle und/oder Konstrukte kodieren. Aufgrund des degenerierten genetischen Codes können diese Nukleinsäuremolaküle sehr unterschledliche Sequenzen haben. Die Wahl der Codons ist ebenfalls von der Zelle abhängig, die für die Herstellung des Erkennungsmoleküls verwendet wird, da in unterschiedlichen Zellen aus unterschiedlichen Organismen häufig unterschiedliche Codons bevorzugt werden und die Expressionsrate stark beeinflusst werden kann, beispielsweise sind die in eukaryontischen Genen bevorzugt verwendeten Codons AGA und AGG für Arginin in Bakterien nur selten vertreten. Hier treten die Codons CGC und CGU deutlich häufiger auf. Das Nükleinsäuremolekül ist in bevorzugten Ausführungsformen eine genomische DNA, eine cDNA und/oder eine RNA. Die Kriterien zur Wahl geeigneter Codons und die Herstellung eines geeigneten Nukleinsäuremoleküls sind dem Fachmann bekannt.

Vektoren können zur Expression der Erkennungemoleküle insbesondere in Zellen eingesetzt werden. Unter einem Vektor versteht man im Sinne der Erfindung ein Nukleinsäuremolekül, das zur Expression des Erkennungsmoleküls dient und das eine Nukleinsäuresequenz, die ein odermehrere genetische Sequenzen umfasst, die mindestens eines der oben beschriebenen Erkennungemoleküle kodieren, und insbesondere mindestens einen Promotor umfasst, der die Expression des Erkennungsmoleküls bewirkt. Vektoren können dabei selbstverständlich weitere Elemente umfassen, die dem Fachmann bekannt sind und die beispielsweise der Vermehrung von Vektoren zur Herstellung in geeigneten Zellen und zur Klonierung dienen. Die Nukleinsäuresequenzen können auf einem oder mehreren Vektoren vorliegen, beispielsweise wird in einer bevorzugten Ausführungsform die schwere Kette eines Immunglobulins durch einen und die leichte Kette durch einen anderen Vektor kodiert. In einer weiteren bevorzugten Ausführungsform der Erfindung sind die variable Domäne der leichten Kette und die variable Domäne der schweren Kette auf dem gleichen Vektor unter einem Promotor als Fusionsprotein kodiert. Außerdem können im Sinne der Erfindung Nukleinsäuresequenzen, die Teile eines Erkennungsmoleküle kodieren, durch unterschiedliche dem Fachmann bekannte Promotoren exprimiert werden. In einer weiteren Ausführungsform können die unterschiedlichen Nukleinsäuresequenzen auf einem gemeinsamen Vektor liegen. Dabei kann jede Sequenz durch einen eigenen, gleichen oder unterschiedlichen, Promotor exprimiert werden oder die Sequenzen können in einem bleistronischen Vektor unter einem Promotor vorliegen. Bevorzugt werden durch die unterschiedlichen Promotoren unterschiedliche Expressionsraten der Teile der Erkennungsmoleküle erreicht, die eine Bildung des gesamten Erkennungsmoleküls gegenüber einer gleichen Expressionsrate der verschiedenen Teile verbessert Weiterhin bevorzugt werden Promotoren verwendet, die Induzierbar sind, um eine Expression des Erkennungsmoleküls zu verbessern. Besonders bevorzugt umfassen die Vektoren weiterhin andere dem Fachmann bekannte regulatorische Elemente, beispielsweise Enhancer, die die Expression des Erkennungsmoleküls oder Teile davon verstärken, beispielsweise derCMV Enhancer oder Immunglobulin-Enhancer-Sequenzen. Bevorzugt umfassen die Nukleinsäuremoleküle und Vektoren zusätzlich Nukleinsäuresequenzen, die als Signalsequenzen zur Sekretion des Erkennungsmoleküls oder Tellen davon dienen, die dem Fachmann an sich bekannt sind, beispielsweise PelB, OmpA oder MalE für prokaryontische Zellsysteme bzw. das Signalpeptid des T-Zellrezeptors, der Immunglobulinketten, des t-PA oder EPO für eukaryontische Zellsysteme [Boel et al., 2000; Herrera et al., 2000]. Dies erleichtert vorteilhafterweise die Reinigung und/oder verbessert die Ausbeute der Erkannungsmoleküle. Die Verfahren zur Herstellung der oben beschriebenen Nukleinsäuren und Vektoren, geeigneter Promotoren, Enhancer und Vektorkonstrukte sowie die Kriterien zu deren Wahl sind dem Fachmann bekannt und werden in den Beispielen näher erläutert.

In einer besonderen Ausführungsform umfasst der Vektor weiterhin Nukleinsäuresequenzen, die für virale Proteine kodieren. Als eine besondere Form eines Vektors wird dabei der Virus selbst bezeichnet, dessen genetisches Material eine Nukleinsäuresequenz umfasst, die für ein Erkennungsmolekül kodiert. In einer bevorzugten Form ist das Erkennungsmolekül ein Fusionsprotein mit einem Vlrushüli-protein oder Teilen davon, das es ermöglicht, dass nicht nur das genetische Material die Nukleinsäuresequenz des Erkennungsmoleküls umfasst sondern auch das Erkennungsmolekül selbst auf der Oberfläche des Virus bindungsaktiv vorliegt, beispielsweise ein scFv Erkennungsmolekül als Fusionsprotein mit einem Hüllprotein von für gentherapeutische Anwendungen geeigneten Adenoviren, Poxviren oder Vacciniaviren. Dies vermittelt die Adressierung des Virus zu einer Core-1 exprimierenden Tumorzelle, wodurch es zur Expression des Erkennungsmoleküls in der Tumorzelle kommt Dies kann zur Expression des Erkennungsmoleküls in vivo im Organismus oder in vitro in der Zellkultur verwendet werden. Bevorzugt werden dabei bekannte Systeme verwendet, die einen Helfervirus zur Replikation verwenden, um beispielsweise die Sicherheit eines diesen Vektor umfassenden gentherapeutischen Verfahrens zu gewährleisten. Die Verfahren zur Herstellung der beschriebenen viralen Vektoren, zur Infektion und Expression der Erkennungsmoleküle sind dem Fachmann bekannt.

In einer weiteren besonderen Ausführungsform umfasst der Vektor ein Fusionsprotein aus einem Erkennungsmolekül und einem Protein oder Peptid, das spezifisch an ein Virus bindet. Die gewonnenen Erkennungsmoleküle können so mit Vorteil zur Adressierung des Virus an eine Core-1 exprimierende Zelle verwendet werden. So kann z.B. der Transfer des genetischen Materials Ober Infektionen vermittelt werden, wodurch es ermöglicht wird, spezifische Moleküle, die durch das genetische Material des Virus kodiert werden, in den Zellen in vivo im Organismus in Form einer Gentherapie oder in vitro in der Zellkultur zu exprimieren.

Weiterhin betrifft die Erfindung ein Verfahren zur Gewinnung der Erkennungsmoleküle umfassend das Einbringen von ein oder mehreren Vektoren, die ein oder mehrere Nukleinsäuremoleküle enthalten, in eine geeignete Wirtszelle, die Kultivierung dieser Wirtszelle unter geeigneten Bedingungen und die Bereitstellung von ein oder mehreren Erkennungsmolekülen aus den Zellen oder dem Kulturmedium. Unter dem Begriff "Einbringen von Vektoren" versteht man im Sinne der Erfindung dem Fachmann an sich bekannte Technologien mit denen der Vektor in eine Wirtszelle gebracht wird, beispielsweise Elektroporation, Transfektion unter Verwendung kationischer Lipide oder infektion und dort translent oder stabil verbleibt. Unter dem Begriff "Bereitstellung von einem oder mehreren Erkennungsmolekülen" versteht man im Sinne der Erfindung dem Fachmann an sich bekannte Technologien mit denen die während des Kultivierungs prozesses exprimierten Erkennungsmoleküle aus dem Kulturüberstand und/oder Zellen gewonnen werden, beispielsweise unterschiedliche proteinchemische Reinigungsschritte beispielsweise Fraktionierung, Konzentrierung, Fällungen, und/oder Chromatographie. Die in dem Verfahren zu verwendenden Techniken und Methoden sind dem Fachmann bekannt, ebenso ist der Fachmann in der Lage, geeignete Wirtszellen und Kultivierungsbedingungen sowie Methoden zur Bereitstellung aus den Zellen und/oder dem Kulturüberstand auszuwählen. Hierbei wählt der Fachmann beispielsweise, wie bereits oben ausgeführt, Nukleinsäuresequenzen mit geeigneten Codons und Promotorsequenzen abgestimmt auf die Wirtszelle, um eine möglichst starke Expression von aktiven Erkennungsmolekülen zu gewinnen. In einer bevorzugten Ausführungsform verwendet der Fachmann bespielsweise affinitätschromatographische Schritte, beispielsweise Chromatographie an Protein A oder Protein G oder Protein L oder beipielsweise Metallionen-Affinitätschromatographie über einen zusätzlich eingefügten His-Tag. In den Beispielen ist dies beispielhaft näher ausgeführt.

Der Begriff "Gewinnung" umfasst neben den zuvor explizit genannten Schritten auch zusätzliche Schritte, wie etwa Vorbehandlungen des Ausgangsstoffes oder Weiterbehandlungen des Endproduktes. Vorbehandlungsverfahren sind an sich dem Fachmann bekannt. Weiterbehandlungsverfahren umfassen neben den oben beschrieben Bereitstellungsverfahren beispielsweise auch die endgültige Zusammensetzungen und/oder Formulierung des mit dem Herstellungsverfahren gewonnenen Erkennungsmoleküls in geeigneten Verwendungs- und/oder Darreichungsformen. Die Art der Verwendungs- oder Darreichungsform, z.B. Lösung, Lyophilisat oder Tablette, hängt hierbei von der beabsichtigten Verwendung ab. Dem Fachmann ist hierbei bekannt, welche Darreichungsform sich für welchen Verwendungszweck eignet. Je nach Darreichungsform kann das durch das Verfahren hergestellte Erkennungsmolekül zusammen mit Hilfs-, Träger- oder weiteren Wirkstoffen vorliegen. Hilfsstoffe sind hierbei vorzugsweise Adjuvantien, weitere Wirkstoffe, vorzugsweise immunstimulatorische Moleküle, wie Interleukine. Das mit dem Verfahren hergestellte Erkennungsmolekül kann auch in Weiterbehandlungsschritten chemisch modifiziert werden. Vorzugsweise wird das Erkennungsmolekül hierbei mit einem oder mehreren weiteren Molekülen in geeigneter Weise, d.h. durch chemische oder physikalische Interaktion, verbunden. Als weitere Moleküle im Sinne der Erfindung dienen bevorzugt anderen Proteine oder Peptide, die mit dem durch das Verfahren hergestellten Erkennungsmolekül kovalent oder nicht-kovalent verknüpft werden, beispielsweise um bispezifische Erkennungsmoleküle herzustellen, indem ein Erkennungsmolekül, das spezifisch das Core-1 Antigen erkennt, mit einem zweiten Molekül verknüpft wird, das beispielsweise eine immuneffektorzeite (beispielsweise Makrophage, NK-Zellen, Dendritische Zellen) spezifisch bindet oder beispielsweise eine Verknüpfung mit Interleukinen (beispielsweise IL-2, IL-7, IL-12, IL-15), Chemokinen oder Wachstumsfaktoren, wodurch Ober die Wirkung dieser Moleküle über die Bindung des Erkennungsmoleküls Immuneffektoren an die Core-1 positiven Tumorzellen dirigiert werden und diese beilspielsweise bekämpfen und/oder zerstören. Diese weiteren Moleküle oder Teile davon können wie bereits weiter oben beschrieben auch Teil des Erkennungsmoleküls selbst sein und werden in diesem Fall nicht über die hier beschriebenen chemischen oder physikalischen Methoden nach der Expression des Erkennungsmoleküls verknüpft. Unter "immuneffektoren" verstehtman im Sinne der Erfindung solche Komponenten der Erfindung die direkt oder indirekt eine Bekämpfung und/oder Zerstörung von Core-1 positiven Tumorzellen bewirken können, beispielsweise Immuneffektorzellen, wie beispielsweise Makrophagen, NK-Zellen, Dendritische Zellen, oder Effektormoleküle, wie beispielsweise Proteine oder Peptide des Komplementsystems. Als weitere Moleküle im Rahmen des erfündungsgemäßen Verfahrens sind besonders Substanzen geeignet, die eine therapeutische oder diagnostische Wirkung entfalten, beispielsweise Radioisotope oder Toxine. Diese Substanzen werden Ober an sich bekannte Verfahren mit den Erkennungsmolekülen verknüpft, beispielsweise werden Radioisotope entweder direkt eingelagert (beispielsweise lod) oder über einen kovalent gekoppelten Chelator (beispielsweise Yttrium, Indium, Bismut) gebunden. Die Schritte des Weiterbehandlungsverfahrens sind dem Fachmann bekannt

Die zur Expression der Erkennungsmoleküle verwendeten Zellen können prokaryontische oder eukaryontische Zellen sein, beispielsweise Bakterien-, Hefe- (bevorzugt S.cerevisiae oder P.pastoris), Insekten-(D.melanogaster), Pflanzen-, Säugerzellen (bevorzugt Hamster-, Maus- oder humane Zelllinien) oder Organismen wie transgene Tiere und Pflanzen. Vorzugsweise werden zur Expression der Erkennungemoleküle in einem prokaryontischen System E.coli und zur Expression in einem eukaryontischen System die Säugerzeillinien NS0, SP2/0, CHO-K1, CHOdhfr-, COS-1, COS-7, HER293, K562, Namalwa oder Percy 6 verwendet.

Mit Hilfe von Wirtszellen, die durch das oben beschriebene Verfahren hergestellt wurden, können Erkennungsmoleküle hergestellt werden. Selbstverständlich können die Wirtszellen Tell eines Klons sein oder ihn selber darstellen. Organismen können diese Wirtszellen umfassen. Die zu verwendenden Techniken und Methoden zur Herstellung dieser Organismen sind dem Fachmann bekannt.

Zusammensetzungen für therapeutische, prophylaktische oder diagnostische Zwecke können mindestens ein Erkennungsmolekül in einer geeigneten, insbesondere einer pharmazeutisch geeigneten Form oder Zusammensetzung umfassen. Die pharmazeutische Zusammensetzung umfaßt insbesondere zusätzliche Stoffe und Substanzen, beispielsweise medizinische und/oder pharmazeutisch-technische Hilfsstoffe. Im Sinne der Erfindung gelten als Arzneimittel sowohl solche pharmazeutischen Zusammensetzungen, die für therapeutische und prophylaktische Zwecke verwendet werden, als auch solche pharmazeutischen Zusammensetzungen, die in vivo als Diagnostikum eingesetzt werden. In einer weiteren bevorzugten Ausführungsform handelt es sich um Zusammensetzungen für die ex vivo Diagnostik die zusätzliche Stoffe und Substanzen enthalten können. Diese Ausführungsform ist unter der Beschreibung für die Diagnostika näher ausgerührt

"Arzneimittel oder pharmazeutische Zusammensetzungen", die vorliegend synonym verwendet werden, sind erfindungsgemäß Stoffe und Zubereitungen aus Stoffen, die dazu bestimmt sind, durch Anwendung am oder im menschlichen Körper Krankheiten, Leiden, Körperschäden oder krankhafte Beschwerden zu hellen, zu lindem oder zu verhüten. Medizinische Hilfsstoffe sind erfindungsgemäß solche Stoffe, die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Beispiele für pharmazeutisch verträgliche Träger sind nachstehend aufgeführt. Die Arzneimittelformullerung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen z.B. Phosphat-gepufferte Kochsalzlösungen, Wasser, Emulsionen wie z.B. Öl/Wasser-Emulsionen, verschiedene Arten von Detergenzien, sterile Lösungen, etc. Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, z.B. in einem Bereich von 1 µg bis 10 g an Erkennungsmolekülen pro Tag und Patient Bevorzugt werden dabei Dosen von 1 mg bis 1 g. Die Verabreichung kann auf verschiedenen Wegen erfolgen, beispielsweise intravenös, intraperitoneal, intrarektal, intragastrointestinal, intranodal, intramuskulär, lokal, beispielsweise in den Tumor, aber auch subkutan, intradermal oder auf der Haut oder Ober die Schleimhäute. Die Verabreichung von Nukleinsäuren kann auch in Form von Gen-Therapie geschehen, beispielsweise Ober weiter oben beschriebene virale Vektoren. Die Art der Dosierung und des Verabreichungsweges kann vom behandelnden Arzt entsprechend den klinischen Faktoren bestimmt werden. Es ist dem Fachmann bekannt, dass die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Die pharmazeutischen Zusammensetzungen oder das Arzneimittel umfasst insbesondere eine pharmakologische Substanz, die ein oder mehrere Erkennungsmoleküle oder/und diese kodierende Nukleinsäuremoloküle in einer geeigneten Lösung oder Verabreichungsform enthält. Diese können entweder alleine mit den entsprechenden unter Arzneimitteln oder pharmazeutischen Zusammensetzungen beschriebenen Hilfsstoffen oder in Kombination mit einem oder mehreren Adjuvantien, beispielsweise QS-21, GPI-0100 oder andere Saponine, Wasser-Öl Emulsionen wie beispielsweise Montanide Adjuvantien, Polylysin, Polyargininverbindungen, DNA-Verbindungen wie beispielsweise CpG, Detox, bakterielle Vakzine wie beispielsweise Thyphusvakzine oder BCG-Vakzine, und/oder einem anderen geeigneten Stoff zur Wirkungsverstärkung verabreicht werden; vorzugsweise immunstimulatorische Moleküle, wie interleukine, beispielsweise IL-2, IL-12, IL-4 und/oder Wachstumsfaktoren, beispielsweise GM-CSF. Diese werden in bekannten Methoden mit den erfindurgagemäßen Erkennungsmolekülen gemischt und in einer geeigneten Formulierung und Dosierung verabreicht Formulierungen, Dosierungen und geeignete Komponenten sind dem Fachmann bekannt.

Die pharmazeutische Zusammensetzung oder das Arzneimittel kann selbstverständlich auch eine Kombination von 2 oder mehreren der pharmazeutischen Zusammensetzungen oder Arzneimittel sein, sowie eine Kombination mit anderen Arzneimitteln, Tumorvakzinen oder Tumorbehandlungen, wie beispielsweise Antikörpertherapien, Chemotherapien oder Radiotherapien, die auf eine geeignete Weise zeitlich gemeinsam oder getrennt verabreicht beziehungsweise angewandt werden. Herstellung der Arzneimittel oder pharmazeutischen Zusammensetzungen erfolgt nach an sich bekannten Methoden.

Die Arzneimittel oder pharmazeutischen Zusammensetzungen können insbesondere zur Behandlung von Core-1 positiven Tumorerkrankungen eingesetzt werden, wie beispielsweise Mammakarzinome, Zervikalkarzinome, Ovarialkarzinome, Colonkarzinome, Gastrointestinalkarzinome, Pankreaskarziome, Lungenkarzinome, Prostatakarzinome. Zu diesen Tumorerkrankungen können auch Core-1 und/oder Core-2 positive Tumorerkrankungen gehören. Die Behandlung geht beispielsweise gegen Primärtumoren, minimal residuale Tumorerkrankungen, Relapses und/oder Metastasen. Die Behandlung der Tumoren kann auch als adjunvante Behandlung erfolgen. Die Verwendung der Arzneimittel kann auch zur Prophylaxe von Core-1 positiven Tumorerkrankungen erfolgen. Die prophylaktische Anwendung zielt beispielsweise auf eine Prophylaxe des Tumors sowie von Metastasen. Die Tumormittel werden in einer geeigneten Form nach bekannten Methoden verabreicht. Eine bevorzugte Variante ist die Injektion beziehungsweise Verabreichung der Arzneimittel intravenös, lokal in Körperkavitäten, beispielsweise intraperitoneal, intrarektal, intragastrointestinal, lokal beispielsweise direkt in den Tumor, Organe oder Lymphgefäße (intranodal), aber auch subkutan, intradermal oder auf der Haut, intramuskulär. Verabreichungsarten können bevorzugterweise auch kombiniert werden, wobei sie an verschiedenen Behandlungstagen oder an einem Behandlungstag verabreicht werden können. Dabei können auch 2 oder mehrere der Arzneimittel oder pharmazeutischen Zusammensetzungen kombiniert werden oder eine oder mehrere Arzneimittel mit ein oder mehreren Arzneimitteln oder Tumorbehandlungenr wie beispielsweise Antikörpertherapien, Chemotherapien oder Radiotherapien, die zeitlich gemeinsam oder getrennt verabreicht beziehungsweise angewandt werden.

Ein Verfahren zur Herstellung eines Arzneimittels oder einer pharmazeutischen Zusammensetzung kann die Schritte der Herstellung von Erkennungsmolekülen und weiterhin den Schritt der Formulierung der Erkennungsmoleküle in pharmazeutisch verträglicher Form umfassen. Die hierfür bevorzugten Erkennungsmoleküle sind weiteroben als Ausführungsformen zur Behandlung von Tumorerkrankungen und Prophylaxe, sowie weiter unten unter in vivo Diagnostika näher beschrieben.

Die Erkennungsmoleküle und durch das Verfahren hergestellten Stoffe und Zusammensetzungen können demgemäß bevorzugt zur Prophylaxe, Diagnose, Verlaufskontrolle und/oder Behandlung von Tumorerkrankungen verwendet werden. Die Verwendung der Erkennungsmoleküle, der Vektoren und/oder des Arzneimittels oder der pharmazeutischen Zusammensetzung zur Prophylaxe und/oder Behandlung von Krebserkrankungen, einschließlich Tumoren und Metastasen ist weiterhin bevorzugt.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplestischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom, die Karzinoid-Herzerkrankung; das Karzinom (z.B. Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nichtlkleinzelliges Bronchialkarzinom, Haferzellen-Karzinom, papilläres Karzinom, szirrhöses Karzinom, bronchioloalveoläres Karzinom, Bronchial-Karzinom, Plattenepithelkarzinom und Transitionalzell-Karzinom); histiocytische Funktionsstörung; Leukämie (z.B. in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendothellose, Chondroblastom; Chondrom, Chondrosarkom; Fibrom; Fibrosarkom; Rlesenzell-Tumore; Histiocytom; Lipom; Liposarkam; Leukosarkom; Mesotheliom; Myxom; Myxosarkom; Osteom; Osteosarkom; Ewing-Sarkom; Syno-vlom; Adenofribrom; Adenolymphom; Karzinosarkom, Chordom, Craniopharynglom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholanglom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoll-ZellTumor, Thekazeiltumor, Lelomyom; Lelomyosarkom; Myoblastom; Myom; Myosarkom; Rhabdomyom; Rhabdomyosarkom; Ependynom; Ganglioneurom, Gliom; Medulloblastom, Meninglom; Neurliemmom; Neuroblastom; Neuroepitheliom, Neurofibrom, Neurom, Paragangliom, nicht-chromaffines Paragangliom, Anglokeratom, anglolymphoide Hyperplasie mit Eosinophille; scleroslerendes Anglom; Anglomatose; Glomanglom; Hemangloendotheliom; Hemanglom; Hemangloperlcytom, Hemanglosarkom; Lymphanglorn, Lymphangiomyom, Lymphangiosarkom; Pinealom; Cystosarkom phyliodes; Hemanglosarkom; Lymphanglosarkom; Myxosarkom, Ovarialkarzinom; Sarkom (z.B. Ewing-Sarkom, experimentell, Kaposi-Sarkom und Mastzell-Sarkom); Neoplasmen (z.B. Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halbes, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe von Krebserkrankungen oder Tumorerkrankungen, die Zellen umfassen, die das Core-1 in der erfindungsgemäßen Definition umfassen, ausgewählt aus der Gruppe: Tumoren des Hels-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Spelcheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Elleiters (Tuba Faloppil), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamille, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantationsbezogene Malignitäten, metastasierte Tumoren umfassend Gehirnmetastasen, Lungenmetastasen, Lebemetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzeilkarzinome und/oder Lebermetastasen.

Die Erkennungsmoleküle können bei der Behandlung oder Prophylaxe von Tumorerkrankungen direkt eingesetzt werden oder mit zusätzlichen Effektorstrukturen gekoppelt werden. Unter "Effektorstrukturen" versteht man erfindungsgemäß solche chemischen oder biochemischen Verbindnungen, Moleküle oder Atome, die direkt oder indirekt eine Abtötung oder Schädigung, einschließlich beispielsweise Wachstumsverlangsamung oder Wachstumsinhibition von Tumorzellen bewirken. Hierzu gehören beispielsweise Radioisotope, Toxine, Cytostatika und andere Effektormoleküle wie beispielsweise Cytokine und Chemokine oder andere Strukturen, die selbst Effektoren darstellen oder an die Effektormoleküle gekoppelt werden, beispielsweise mit Toxinen oder Cytostatika beladene Uposomen, die Erkennungsmoleküle tragen. Beim letzteren Beispiel der Liposomen sind insbesondere auch solche Effektorstrukturen gemeint, die neben dem Erkennungsmolekül für die Tumorspezifität auch solche Moleküle tragen, die für eine Aufnahme der Effektorstrukturen oder Teile davon in die Zellen verantwortlich sind, wie beispielsweise Antikörper gegen Rezeptoren, die eine Rezeptor-vermittelte Endozytose bewirken. Vorzugsweise umfassen die Erkennungsmoleküle in diesen Fällen eine Transmembrandomäne, die ihnen eine Insertion in die Liposomenmembran erlaubt oder in einer anderen bevorzugten Ausführungsform werden die Erkennungsmoleküle chemisch auf die Liposomenoberfläche gekoppelt. Die hierfür verwendeten Techniken sind dem Fachmann bekannt, einschließlich der Herstellung der Liposomen. Auch die Verbindung der Erkennungsmoleküle mit den anderen Effektorstrukturen erfolgt nach an sich bekannten Methoden. Die Kopplungen können dabei wie bereits oben aufgeführt beispielsweise direkt durch kovalente oder nicht-kovalente Beladung erfolgen, durch chemische Kopplung, wobei ein zusätzliches chemisches oder biologisches Molekül notwendig sein kann, beispielsweise ein Chelator oder ein Linker, oder in Form von Fusionsproteinen oder -peptiden durch Fusion. Eingesetzt werden die Erkennungsmoleküle bei der Behandlung von Tumorerkrankungen mit Core-1 tragenden Tumoren, und/oder für eine Untergruppe an Erkennungsmolekülen, die weiter oben Ober ihre Spezifität für Core-1 und Core-2 beschrieben sind, Core-2 und/oder Core-1 tragenden Tumorzellen oder zur Prophylaxe, die beispielsweise die Ausbildung von primären Tumoren oder Metastasen verhindert Bevorzugtes Ziel ist dabei die Behandlung der minimalen residualen Erkrankung undvon Metastasen. Eine weitere bevorzugte Anwendung ist dabei die inhibition der Lebermetastasierung von Core-1 und/oder Core-2 positiven Tumorzellen. Die Erkennungsmoleküle werden dabei in einer geeigneten Formulierung einmalig oder wiederholt in geeigneten zeitlichen Abständen und Dosen verabreicht.

Im Folgenden und davor versteht man im Sinne der Erfindung unter dem Core-1 Antigen auch Core-1 und/ oder Core-2 und unter Core-1 positiven Zellen oder Tumorzellen und/oder -geweben auch Core-1 und/oder Core-2 positive Zellen oder Tumorzellen und/oder -gewebe.

In einer bevorzugten Ausführungsform werden die oben beschriebenen radioaktiven Erkennungsmoleküle mit einer Applikation von nicht markierten Core-1 spezifischen Erkennungsmolekülen kombiniert. Dies dient der Verbesserung des Hintergrundes und damit einer spezifischeren Bindung an den Tumor, indem potentielle Core-1 tragende Moleküle im Blut abgesättigt werden. Bevorzugt werden dabei IgM abgleitete Erkennungsmoleküle verwendet, beispielsweise der in den Beispielen beschriebene cigM oder die humanisierte Form davon, da diese vor allem an Core-1 Antigen im Blut binden und damit den Hintergrund und die Serumbelastung mit Radioaktivität emiedrigen und das relative Tumortargeting erhöhen, während aufgrund der Größe der Moleküle ein Eindringen in Gewebe und Tumoren limitiert ist. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungemoleküle erstellen.

Bevorzugt ist weiterhin die Verwendung von viralen Vektoren zur gentherapeutischen Anwendung, bei der insbesondere die Oberfläche der Viren Erkennungsmoleküle tragen.

Die Erfindung betrifft weiterhin Verfahren unter Verwendung der Erkennungsmoleküle, die es erlauben, aus einem großen Pool unterschiedlicher Moleküle Core-1 tragende Moleküle zu identifizieren und/ oder zu gewinnen, die für eine Anwendung in der Tumorbehandlung, Tumorprophylaxe und Tumordiagnose vorteilhaft verwendet werden können. Unter Core-1 tragenden Morekülen versteht man erfindungsgemäß Moleküle, die Core-1 und/oder Core-2 Strukturen tragen und spezifisch von den Erkennungemolekülen gebunden werden. Erfindungsgemäß sind Core-1 tragende Moleküle Glykoproteine, Glykopeptide und/oder Glylcolipide sowie auch Zellen oder andere Trägersubstanzen, wie beispielsweise Viren, Bakterien, Teile von Zellen, wie beispielsweise Exosomen oder Zelllysate, oder Liposomen, die ein oder mehrere Core-1 Strukturen enthalten. Die Core-1 tragenden Moleküle können aus Zellen oder Zelllinien, aus Kulturüberständen, aus Tumorgewebe, Tumorzellen oder Körperflüssigkeiten, wie Blut, Blutserum, Lymphe, Urin, Spinalflüssigkeit oder Sperma angereichert oder isoliert werden.

Die zuvor eingeführten Definitionen der Begriffe sind für die Begriffe in den nachfolgend beschriebenen Verfahren mutatis mutandis anwendbar.

Core-1 tragende Moleküle werden in einem erfindungsgemäßen Verfahren durch Bindung an die oben beschriebenen Core-1 spezifischen Erkennungsmoleküle identifiziert und/oder isoliert und gewonnen. Nach dem erfindungsgemäßen Verfahren können die oben beschriebenen Core-1 tragenden Moleküle aus Körperflüssigkeiten oderaus Überständen von Zellkulturen durch eine Affinitätschromatographie gewonnen werden. Dabei können weitere Reinigungs- und/oder Konzentrierungsschritte nach an sich bekannten Methoden mit einem oder mehreren Affinitätschromatographieschritten kombiniert werden. Ebenso können tumorassoziierte Core-1 tragende Moleküle aus Tumorzellen, Tumorgeweben oder Tumorzelllinien gewonnen werden, indem ein geeigneter Schritt nach an sich bekannten Methoden vorgeschaltet wird, der es erlaubt, die zellassoziierten Core-1 tragenden Moleküle für die Affinitätsreznigung zugänglich zu machen, beispielsweise durch Solubilisierung mit geeigneten Detergenzien oder durch Abspaltung durch Proteolyse oder durch Zelllyse.

In einem weiteren erfindungsgemäßen Verfahren werden Core-1 tragende Moleküle oder Zellen aus Geweben gewonnen. Hierzu wird das Gewebe nach an sich bekannten Methoden aufgeschlossen, um die Core-1 tragenden Moleküle oder Zellen zugänglich zu machen, beispielsweise durch proteolytische oder mechanische Methoden. Dem Fachmann sind diese Verfahren bekannt.

Wie oben ausgeführt werden auch Core-1 positive Zellen oder Zelllinien unter Verwendung der Core-1 spezifischen Erkennungsmoleküle isoliert oder angereichert und von solchen Zellen, die keine oder geringe Mengen an Core-1 Strukturen tragen, getrennt. Unter dem Begriff "Isolierung oder Anreicherung der Zellen" sind alle Maßnahmen zur Separierung von Zellen zu verstehen, die durch das Tragen von Core-1 Strukturen einen Komplex mit den Erkennungsmolekülen gebildet haben. Dem Fachmann sind diese Verfahren bekannt. Vorzugsweise wird hierfür die FACS oder die MACS Methode eingesetzt. Beispielsweise erfolgt die Anreicherung durch Bindung von Erkennungsmolekülen an die Core-1 Struktur auf der Zelloberfläche und anschließender Selektion der so markierten Zellen durch Bindung an Trägermateriallen, die spezifisch mit dem Erkennungsmolekül interagieren, beispielsweise anti-Maus IgM Antikörper gekoppelt an Magnetbeads (MACS-Sortierung). Außerdem können die Core-1 spezifischen Erkennungsmoleküle selbst kovalent an einen Träger gekoppelt sein. Ein weiteres Beispiel ist die Gewinnung mit Hilfe eines FACS-Sorters, der Zellen, die die Erkennungsmoleküle tragen, die fluoreszenzmarkiert wurden, sortiert. Beide Methoden sind dem Fachmann bekannt Diese so angereicherten Core-1 positiven Zellen können für die Herstellung von Vakzinen verwendet werden, beispielsweise zur Beladung von Dendritischen Zellen oder direkt als Tumorzelllysat in einer Vakzinen Zusammensetzung. Die vorhergehende Anreicherung von Core-1 positiven Zellen soll zu einer höheren Tumorspezifität der Vakzinierung führen. Dem Fachmann sind diese Verfahren bekannt

Verfahren zur Herstellung eines Diagnostikums können die Schritte des Verfahrens zur Herstellung der erfindungsgemäßen Core-1 spezifischen Erkennungsmoleküle und weiterhin den Schritt der Formulierung der Erkennungsmoleküle in einer diagnostisch verwendbaren Form umfassen.

Unter dem Begriff "Diagnostikum" sind erfindungsgemäß Stoffe und Zubereitungen aus Stoffen definiert, die dazu bestimmt sind, durch Anwendung am oder im menschlichen Körper oder Teilen davon Krankheiten, Leiden, Körperschäden oder krankhafte Beschwerden zu erkennen. Als Teile des menschlichen Körpers sind vorzugsweise Körperflüssigkeiten, wie Blut, Blutserum, Lymphe, Urin, Spinalflüssigkeit oder Sperma, oder Gewebebiopsien oder-proben zu verstehen.

Die Formulierung des Diagnostikums umfasst vorzugsweise die Modifikation der hergestellten Erkennungsmoleküle mit Substanzen, die einen Nachweis des Antigens Core-1, in bestimmten Ausführungsformen, die von der Felnspezifität des Erkennungsmoleküls abhängen, definitionsgemäß auch das Antigen Core-2, erlauben. Geeignete Substanzen sind im Stand der Technik bekannt Ausgehend von der Wahl der Substanz ist der Fachmann in der Lage, geeignete Maßnahmen zur Formulierung des Diagnostikums einzusetzen.

Für die Diagnostik können erfindungsgemäß auch Substanzen nach an sich bekannten Methoden an die Erkennungsmoleküle gekoppelt werden, die einen Nachweis der Core-1 Antigene und/oder deren Trägermoleküle und/ oder -zellen erleichtern, beispielsweise durch Biotinylierung, Fluoreszenzmarkierung, radioaktive Markierung oder Enzymkopplung der Erkennungsmoleküle.

In einem weiteren Verfahren zur Tumordiagnostik und Prognose werden Erkennungsmoleküle verwendet, die Core-1 Antigene und/oder deren Trägermoleküle im Serum von Menschen erkennen. Die Bestimmung erfolgt bevorzugt qualitativ, quantitativ und/oder in zeitlich relativen Quantitäten nach an sich bekannten Methoden. Die gleichen Verfahren werden auch zur Verlaufskontrolle von Tumorerkrankungen und zur Kontrolle von Behandlungsverläufen einschließlich des Monitorings von Immunantworten und zur Kontrolle und Dosierung von Tumorbehandlungen eingesetzt Die in den Verfahren verwendeten Methoden sind an sich bekannt, beispielsweise ELISA, Westerblot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), Immuncytochemie und Immunhistochemie.

In den bevorzugten Verfahren zur Tumordiagnostik und Prognose werden Core-1 spezifische Erkennungsmoleküle in an sich bekannten Verfahren eingesetzt, um das Antigen Core-1 im Serum oder in Gewebspräparaten nachzuweisen. Dabei wird das Antigen Core-1 auf Trägermolekülen, in immunkomplexen auf Trägermolekülen vorliegendes Core-1, und/oder auf Zellen gebundenes Core-1 nachgewiesen und das Vorhandensein des Core-1 Antigens und/oder der Core-1 tragenden Moleküle qualitativ, quantitativ und/oder in relativen Quantitäten nach an sich bekannten Methoden bestimmt. Die gleichen Verfahren werden auch zur Verlaufskontrolle von Tumorerkrankungen und zur Kontrolle von Behandlungsverläufen eingesetzt. Die in den Verfahren verwendeten Methoden sind an sich bekannt, beispielsweise ELISA, Western-Blot, FACS (Fluoreszenzaktivierte Zellsortierung), MACS (Magnetvermittelte Zellsortierung), ADCC (Antikörpervermittelte Zellzytotoxizität), CDC (Komplement vermittelte Zytotoxizität), immuncytochemie und immunhistochemie.

Eine bevorzugte Ausführungsform ist ein Gewebsschneiltest, bei dem in einem immunhistologischen Verfahren die Gewebsproben mit fluoreszenzmarkierten Erkennungsmolekülen gefärbt werden. In einem weiter bevorzugten Verfahren wird das Erkennungsmolekül, bevorzugt ein Antikörper des isotyps IgM, mit einem weiteren Antikörper, der spezifisch das Antigen MUC1 erkennt, bevorzugt Isotop IgG1, kombiniert. Der Vorteil dabei ist, dass beispielsweise für die Diagnostik von gastrointestinalen Karzinomen (z.B. Colorektale Karzinome und Magenkarzinome) diese in einem frühen Stadium erkannt und gleichzeitig eine Prognose bezüglich des Krankheitsverlaufes und/oder der Lebermetastasierungsgefahr gegeben werden kann, wobei ein höheres Niveau an Core-1 Antigen eine schlechtere Verlaufsprognose und eine um das mehrfach höhere Wahrscheinlichkeit der Lebermetastasierung bedeutet. In einer weiter bevorzugten Ausführungsform sind die Antikörper und Erkennungsmoleküle direkt mit unterschiedlichen Fluoreszenzfarbstoffen, beispielsweise Cy3 und Cy5 oder Cy3 und FITC, markiert. In einer Ausführungsform, in der eine Signalverstärkung vorteilhaft ist, werden die Antikörper und/oder Erkennungsmoleküle durch markierte sekundäre Antikörper oder das Blotin-Streptavidin verstärkt. Dabei ist es vorteilhaft, unterschiedliche Isotypen und/oder Speziessequenzen im konstanten Teil der Antikörper zu verwenden. Die hierbei verwendeten Technologien und Methoden, beispielsweise der Markierung und der Immunhistologie, sowohl die Wahl der geeigneten Formate der Erkennungsmoleküle sind dem Fachmann bekannt. Das beschriebene diagnostische Verfahren ist nicht auf gastrointestinale Tumoren beschränkt, sondern anwendbar für alle das Antigen Core-1 tragende Tumorerkrankungen.

In einer weiter bevorzugten Ausführungsform wird ein serologischerTest durchgeführt, bei dem als Verfahren ein Sandwich-ELISA verwendet wird. Dieser besteht aus einem Fängeranilkörper, der Trägermoleküle des Core-1 Antigens aus dem Serum an eine feste Phase bindet, und einem Nachweisantikörper, hierunter fallen auch andere Erkennungemoleküle, die das Core-1 Antigen erkennen. Damit kann unterschieden werden, welches Trägermolekül das Core-1 trägt. In einer bevorzugten Form kann damit auf den Ursprung des Primärtumors Rückschlüsse gezogen werden. Als Fängerantikörper können verschiedene Antikörper dienen, die Glykoproteine erkennen, die O-Glykoslierungen tragen. Eine bevorzugte Ausführungsform verwendet Antikörper, gegen das epitheliale Muzin MUC1 als Fängerantikörper, das häufig ein Träger des Core-1 im Tumorfall ist. In einer weiteren Ausführungsfom werden alle Antigene im Blut bestimmt, die das Core-1 Antigen tragen. Dies ist dadurch möglich, dass das Core-1 Antigen in der Regel in mehreren Kopien pro Trägermolekül vorkommt. Hierbei wird ein Core-1 spezifisches Erkennungsmolekül als Fängerantikörper verwendet und ein markiertes Core-1 spezifisches Erkennungsmolekül als Nachweisantikörper, wobei die Erkennungsmoleküle keine Antikörpersein müssen. In einer bevorzugten Ausführungsform wird ein IgM als Erkennungsmolekül mindestens als Fänger- oder Nachwelsantikörper verwendet. In einer weiter bevorzugten Ausführungsform wird der Nachweisantikörper mit Biotin markiert und das System Ober Streptavidin in Kombination mit einem geeigneten Nachweisverfahren nachgewiesen. Ein geeignetes Nachweisverfahren sind beispielweise POD Markierungen oder Fluoreszenzmarkierungen des Streptavidins.

In einer weiter bevorzugten Ausführungsform werden für einen serologischen Tumortest die Bestimmung des Core-1 Antigens, wie zuvor beschrieben, mit der Bestimmung anderer serologischer Tumormarker kombiniert, beispielsweise PSA, CEA oder AFP. Eine hierbei bevorzugte Ausführungsform ist die Bestimmung des MUC1 und des Core-1 Antigens. In einer bevorzugten Ausführungsform wird hierbei das MUC1 mit Hilfe eines MUC1 spezifischen Antikörpers aus dem Serum an eine feste Phase immobilisiert und mit einem zweiten anti-MUC1 spezifischen Antikörper, bevorzugt solche, die die DTR-Region in einer glykosylierten Form verbessert erkennen, als Nachweisantikörper nachgewiesen und das Core-1 Antigen auf dem mit Hilfe eines anti-MUC1 Fängerantikörpers immobilisierten MUC1 mit einem Erkennungsmolekül nachgewiesen. Dieser diagnostische Test verbindet eine Früherkennung mit einer prognostischen Aussage über den Krankheitsverlauf und/oder der Lebermetastasierungswahrscheinlichkeit. Die hierbei verwendeten Technologien, beispielsweise der Markierung und der Serologie, einschließlich dar Nachweismethoden, sind dem Fachmann bekannt. Die beschriebenen diagnostischen Verfahren sind nicht auf gastrointestinale Tumoren beschränkt, sondern anwendbar für alle das Antigen Core-1 tragende Tumoren. Die beschriebenen serologischen Tests dienen der Diagnose, des Monitorings des Verlaufs derTumorerkrankung und der Prognose von Core-1 Antigen-positiven Tumoren.

In einem weiteren Verfahren werden die Cora-1-spezifischen Erkennungsmoleküle zu einer in vivo Diagnostik verwendet Hierfür werden die Erkennungsmoleküle mit geeigneten an sich bekannten Verfahren markiert und somit für an sich bekanntebildgebende Verfahren am Menschen zugänglich gemacht, beispielsweise Radioimmundiagnostik, PET-Scan Verfahren oder Immunofluoreszenzendoskopie, beispielweise durch Kopplung und/oder Beladung mit entsprechenden Molekülen, beispielsweise radioaktiven Isotope, beispielsweise das Indium, oder Fluoreszenzfarbstoffe, beispielsweise dem Cy3, Cy2, Cy5 oder FITC. In einer bevorzugten Ausführungsform werden Multibodies mit einem geeigneten Chelator (beispielsweise DOTA oder DTPA) kovalent gekoppelt und mit Indium-111 beladen und zur in vivo Diagnostik eingesetzt. Diese werden in einer bevorzugten Ausführungsform Intravenös in einer für das Individuum geeigneten Dosis verabreicht und die Lokalisation des Core-1 Antigens und eines potentiellen Tumors nach an sich bekannten Verfahren gemessen. Die hierfürverwendeten Verfahren und Technologien, einschließlich der bildgebenden Verfahren, sind dem Fachmann bekannt, ebenfalls kann der Fachmann eine geeignete Dosis und Formulierungen erstellen.

In einer weiteren bevorzugten Ausführungsform werden Immunglobuline, bevorzugt IgM und IgG, wie oben beschrieben und in den Beispielen näher ausgeführt, radioaktiv-markiert, beispielsweise mit Indium-111, und lokal in den Tumor oder in den Tumor versorgende oder entsorgende Blutgefäße gegeben. Dies dient in einer Ausführungsform zur Bestimmung der Größe des Tumors und in einer weiteren Ausführungsform der Bestimmung befallener Lymphknoten. Die hierfür verwendeten Verfahren und Technologien sind dem Fachmann bekannt, ebenfalls kann der Fachmann eine geeignete Dosis und Formulierungen erstellen.

In einer weiteren Ausführungsform werden die radioaktiv-markierten Erkennungsmoleküle auch über andere Applikationsrouten verabreicht Dabei bevorzugte Routen sind intraperitoneal, intranodal oder intrarectal bzw. intragastrointestinal. Intraperitoneal ist dabei besonders vorteilhaft zur Bestimmung von Tumoren, die Ober das Peritoneum zugänglich sind und/oder in dieses metastasieren, beispielsweise Ovarialkarzimome und bestimmte gastrointestinale Karzinome, Intrarectale bzw. intragastrointestinale Verabreichung ist vorteilhaft für bestimmte gastrointestinale Tumoren und deren Lokalisation und Größenbestimmung. Intranodal kann in bestimmten Fällen dafür verwendet werden einzelne Lymphknoten direkt zu infiltrieren.

In einer bevorzugten Ausführungsform werden die oben beschriebenen radioaktiven Erkennungsmoleküle für in vivo Diagnostika mit einer Applikation von nicht markierten Core-1 spezifischen Erkennungsmolekülen kombiniert. Dies dient der Verbesserung des Hintergrundes. Bevorzugt werden dabei IgM abgeleitete Erkennungsmoleküle verwendet, da diese vor allem an Core-1 Antigen im Blut binden und damit den Hintergrund deutlich erniedrigen, während aufgrund der Größe der Moleküle ein Eindringen in Gewebe und Tumoren limitiert ist. Die hierfür verwendeten Verfahren und Technologien sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen.

In einer weiteren bevorzugten Ausführungsform werden Erkennungsmoleküle, bevorzugt Immunglobuline, Multibodies oder Antikörperfragmente, weiter bevorzugt IgM, IgG und Multibodies, mit einem Fluoreszenzfarbstoff markiert und in vivo verabreicht. Bevorzugte Applikationsrouten sind hierbel intrarectal, intragastrointestinal, intraperitoneal, Intravenös und in zuführende oder abführende Blutgefäße. Eine besonders bevorzugte Ausführungeform dient der Lokalisation gastrointestinaler Karzinome, die durch eine Fluoreszenzendoskople nach Applikation der fluoreszenzmarkierten Erkennungsmoleküle durchgeführt wird. In einer weiter bevorzugten Ausführungsform wird ein Erkennungsmolekül mit mindestens einem Antikörper gegen ein weiteres Tumorantigen kombiniert, bevorzugt anti-MUC1 Antikörper. Bevorzugt werden dabei unterschiedliche Fluoreszenzfarbstoffe verwendet, die eine Unterscheidung der Erkennungsmoleküle und Antikörper erlauben, womit eine prognostische Aussage mit einer Früherkennung und einer größeren Anzahl an Fällen kombiniert wird. Bevorzugte Fluoreszenzfarbstoffe sind solche mit geringer Hintergrundifluoreszenz, die dem Fachmann bekannt sind. Die hierfürverwendeten Verfahren und Technologien, einschließlich der bildgebenden Verfahren, beispielsweise der Fluoreszenz-Endoskopie, sind dem Fachmann bekannt, ebenfalls kann der Fachmann eine geeignete Dosis, Formulierungen, Applikationsroute und Zeitpunkt der Gabe der nicht-markierten Erkennungsmoleküle erstellen

Die beschriebene Technologie weist mehrere Vorteile auf: Die Core-1 spezifischen Erkennungsmoleküle erkennen Karzinomatten spezifisch, wodurch sie mit Vorteil bei vielen Tumorpatienten verschiedener Indikation zu einer Diagnose und/oder Therapie verwendet werden können. Darüber hinaus binden die Erkennungsmoleküle vorteilhafterweise praktisch nicht auf normalen Geweben. Dies ist gegenüber den bekannten Tumormarkem ein besonderer Vorteil und eine herausragende Eigenschaft der Erkennungsmoleküle. Vorteilhaft ist weiterhin, daß die Erkennungsmoleküle das Core-1 Antigen Träger-unabhängig erkennen. Ein besonderer Vorteil der Erkennungsmoleküle ist die hohe Spezifität für das Tumorgewebe. Dies ist insbesondere in der hohen Spezifität für definite Kohlenhydrat-Antigene gegründet. Bei einer unspezifischen Erkennung anderer Kohlenhydratstrukturen würde sich nämlich die Gefahr der unspezifischen Erkennung von Nicht-Tumorgewebe erhöhen. Weiterhin weisen die Erkennungsmoleküle eine hohe Afffnität auf. Hierdurch ist insbesondere die Möglichkeit gegeben, geringervalente Fragmente zu konstruieren, wie IgG und Multibodies. Die Möglichkeit dieser verschiedenen Formate ist vorteilhaft für die Entwicklung von Therapeutika. Die Core-1 und/oder Core-2 Struktur an der Zelloberfläche erhöht die Wahrscheinlichkeit der Ausbildung von Metastasen, beispielsweise von Lebermetastasen; durch die Blockierung der Core-1 und/oder Core-2 Struktur mit Erkennungsmolekülen wird die Metastasenbildung reduziert bzw. inhibiert.

Im folgenden soll die Erfindung anhand von Beispielen näher erläutert werden, ohne auf diese Beispiele beschränkt zu sein.

### Beispiele

### 1. Herstellung von Core-1 spezifischen Multibodies mit kurzen Linkern

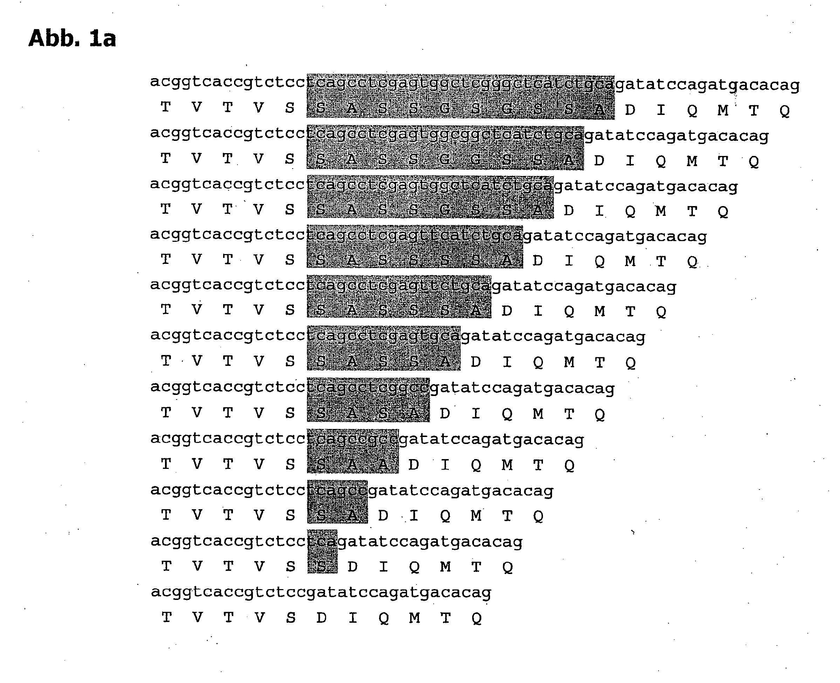

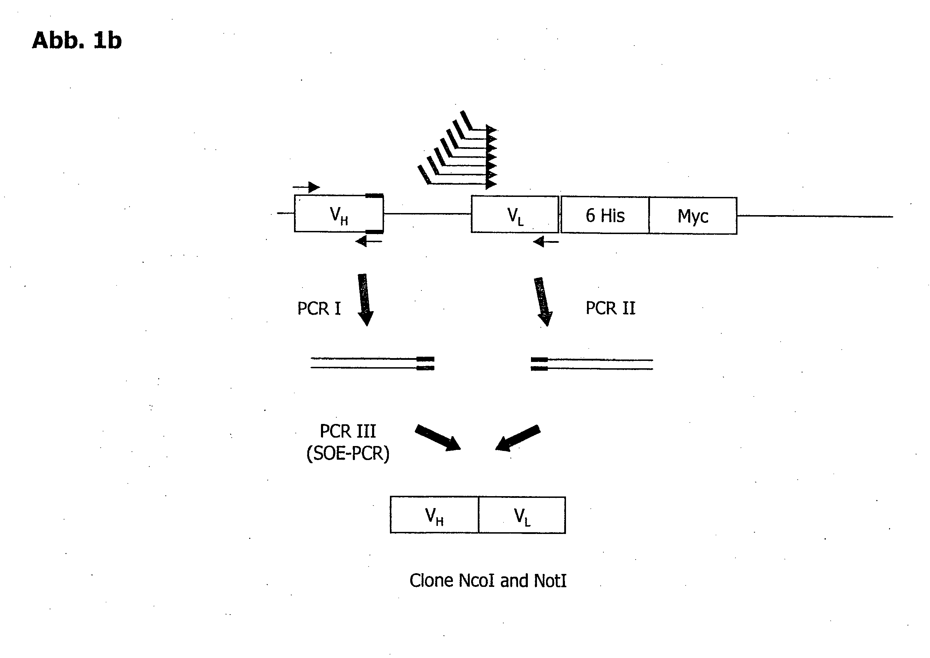

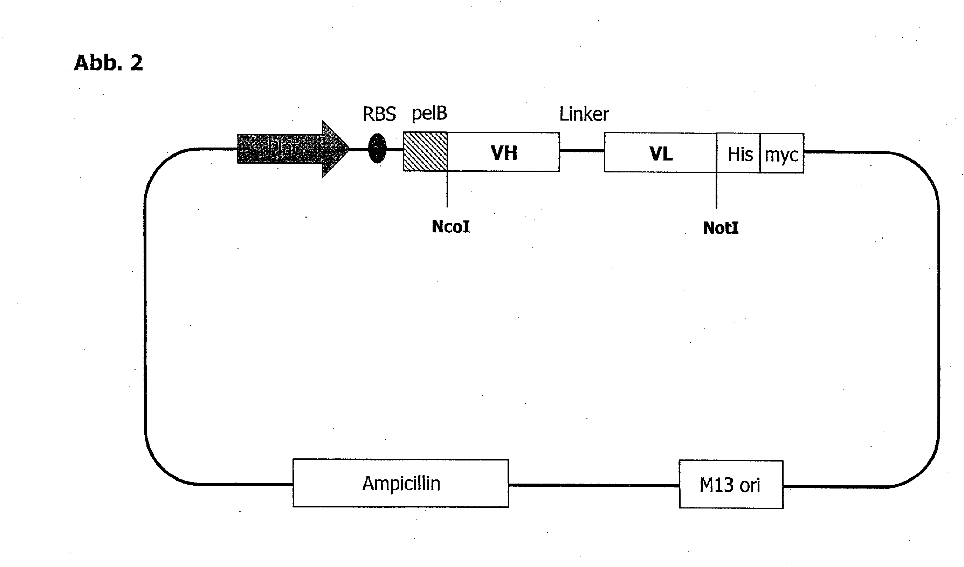

Multibodies mit den Sequenzen SEQ ID NO. 96 bis 106 wurden durch Verkürzung oder Deletion des Unkers zwischen der V_{H} und der V_{L} des single chain Antikörpers mit der Sequenz SEQ ID NO. 95 gebildet (bb. 1 a) . Hierfür wurden die V_{H} und die V_{L} mit spezifischen Primem so amplifiziert, daß 22 Nukleotide am 3'-Ende der V_{H} und am 5'-Ende der V_{L} einen komplementären Bereich ausbilden (Abb. 1b, PCR I und PCR II), und anschließend die beiden PCR-Fragmente nach Aufreinigung in einer SOE-PCR mit einen der verknöpft (Abb. 1b, PCR III). Zum Schluß wurde das PCR-Fragment über NcoI/NotI in einen prokaryontischen Expressionsvektor kloniert Dieser Vektor enthält den lacZ Promotor, eine Ribosomenbindungsstelle (RBS). das M13 origin, die pelB Signalsequenz zur Sekretion ins Periplasma, ein Ampicillin-Resistenzgen und eine Klonierungskassette, um an das Terminale Ende des scFV mit ein Hexa-Histidin-Tag zur effizienten Aufreinigung und ein c-myc-Tag zu koppeln (Abb. 2).

### 2. Bakterielle Expression und Aufreinigung der Core-1 spezifischen Multibodies

Die Antikörperfragmente aus Beispiel 1 wurden in *Escherichia coli* exprimiert und aufgereinigt. Hierfür wurde das entsprechende Plasmid durch Elektroporation in elektrokompetente *E.coli* transformiert und über Nacht in 2xTY Medium (10 g Hefeextrakt, 16 g Trypton, 5 g NaCl per L) mit 100 µg/mL Ampicillin kultiviert. Diese Kultur wurde 1:100 mit 2xTY Medium, dem 100 µg/ml Ampicillin und 0,5% Glukose zugesetzt wurde, verdünnt und bei 37°C inkubiert, bis eine OD_{600 nm} von ca. 0, 6 erreicht war. Dann wurde der Kultur zur Induktion 1 mM IPTG zugesetzt und diese bei 25°C weitere 5 b inkubiert. Die Bakterien wurden durch Zentrifugation bei 4000xg für 20 min geerntet, das Zelipellet in TES Puffer (30 mM Tris-HCl, pH 8.0, 20% Saccharose, 1 mM EDTA) resuspendiert und 20 min auf Eis inkubiert Anschließend wurden 5 mM MgSO₄ zugefügt und die Suspension für weitere 20 min auf Eis inkubiert. Durch Zentrifugation bei 4000 x g für 60 min wurde dann die Periplasmafraktion gewonnen und über Nacht bei 4°C gegen Bindungspuffer (50 mM Phosphatpuffer, pH 8.0, 300 mM NaCl, 10 mM imidazol) dialysiert. Die in der Periplasmafraktion enthaltenen Antikörperfragmente wurden unter Verwendung des C-terminalen His-Tags durch Metallionen-Affinitäts-chromatographie (HI-Trap Chelating HP, Amersham Pharmacia Biotech) aufgereinigt. Dafür wurde die dialysierte Fraktion auf die vorher mit Bindungspuffer äquilibrierte Säule gegeben und mit Waschpuffer (50 mM Phosphatpuffer, pH 8.0, 300 mM NaCl, 30 mM Imidazol die nicht bindenden Proteine von der Säule gewaschen. Anschließend wurden die Antikörperfragmente mit Elutionspuffer (50 mM Phosphatpuffer, pH 8.0,300 mM NaCl, 300 mM imidazol) eluiert. Dieses Aufreinigungsprotokoll wurde für alle Core-1 spezifischen Antikörperfragmente mit Hexa-Histidin-Tag verwendet, beispielsweise den humanisierten single chain Antikörpern aus Beispiel 6.

### 3. Analyse der Core-1 spezifischen Multibodies im scFv Format mit unterschiedlicher Linkerlänge im ELISA

Multibodies mit den Aminosäuresequenzen SEQ ID NO. 95, 96, 97, 98, 99, 100, 101, 103, 104 und 105 wurden wie oben beschrieben in E.coll exprimiert und die Periplasmafraktionen gewonnen. Als Antigen für den ELISA Test wurde Asialoglykophorin (Sigma), ein Core-1 tragendes Glykoprotein, eingesetzt. Aus der Stammlösungen (1 mg in 1 ml BI-dest. H₂O), die portioniert bei -20°C aufbewahrt werden, wurde eine Verdünnung von 5 µg/ml in PBS hergestellt Davon wurden 50µl/well in eine Mikrotiterplatte (NUNCLON-TC Microwell 96 F) pipettiert und die Testplatte über Nacht bei 4°C inkubiert. Am nächsten Tag wurde die Testplatte mit PBS/0,2% Tween 3x gewaschen. Anschließend wurden mit 2% BSA in PBS unspezifische Bindungsstellen blockiert und 50 µl der jeweiligen mit PBS/1% BSA in verschiedenen Verdünnungsschritten verdünnten Fraktionen aufgetragen und 2 h bei 37°C inkubiert. Nach drei Waschschritten mit PBS/0,2% Tween wurden zum Nachweis der spezifisch gebundenen Antikörperkonstrukte als Zweit-Antikörper Peroxidase-gekoppelterantl-His Tag-Antikörper eingesetzt. Zum Nachweis des gebundenen sekundären Antikörpers erfolgte eine Farbreaktion mit TMB (3,3',5,5'-Tetramethylbenzidine). Nach 15 Minuten wurde die Reaktion durch Zugabe von 2,5N H₂SO₄ abgestoppt Die Messung erfolgte mit einem Mikrotiterplattenphotometer mit Filter 450nm im Dual-mode gegen einen Referenzfilter 630nm. Das Ergebnis ist in Abbildung 3 dargestellt. Eine schrittweise Linkerverkürzung führt zu einer Erhöhung der Bindung an Asialoglykophorin. Die besten Bindungseigenschaften zeigen die Varianten mit der SEQ ID NO. 104 und 105. Diese multivalenten Konstrukte im Dia/Triabody Format sind bevorzugte Ausführungsformen und sind aufgrund verbesserter pharmakokinetischer Eigenschaften für die Tumortherapie von Vorteil.

### 4. Klonierung der Vektoren zur Expression chimärer Core-1 spezifischer IgG und IgM Antikörper

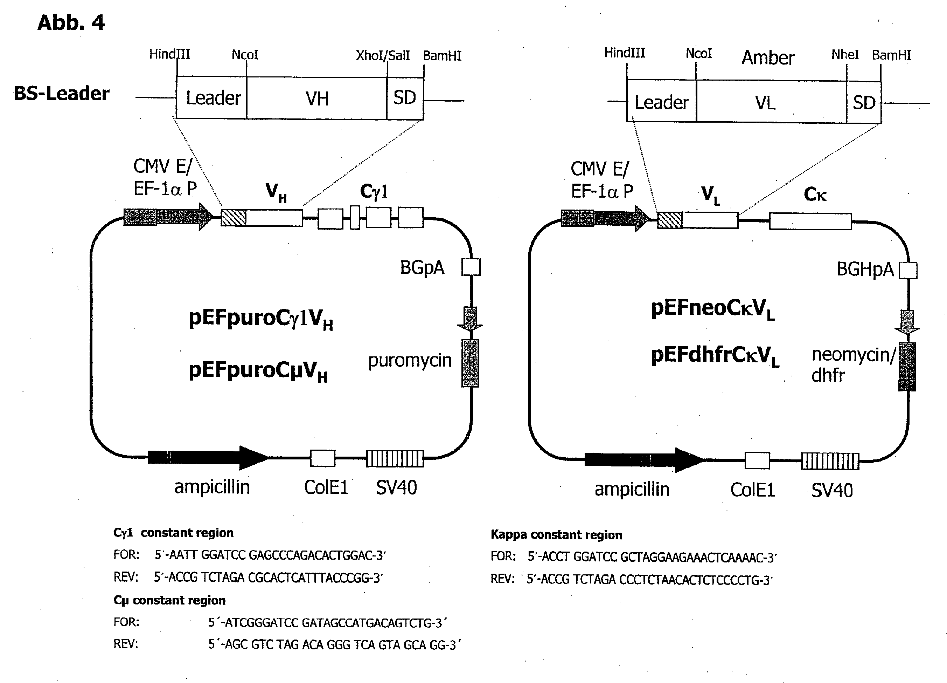

Das NcoI/XhoI DNA Fragment aus dem scFv Vektor, das für die V_{H} kodiert (Abb. 4), wurde in den NcoI/SalI geschnittenen BS-Leader Vektor kloniert. Der BS-Leader Vektor enthält eine Klonierungskassette zur Einführung der T-Zellrezeptor Signalpeptidsequenz an das 6'-Ende sowie einer Splice-Donor-Sequenz an das 3'-Ende der Sequenzen dervariablen Domänen (Abb. 4). Die V_{L}-Sequenz des entsprechenden Antikörpers wurde mit spezifischen Primern zur Einführung der NcoI-Schnittstelle am 5'-Ende und der NheI-Schnittsteile am 3'-Ende in der PCR unter Verwendung der scFv Sequenz als Templat amplifiziert und nach NcoI/NheI Verdau in den gleich verdauten BS-Leader Vektor kloniert. Danach wurde jeweils das HindIII/BamHI Fragment aus dem BS-Leader Vektor in den entsprechenden eukaryontischen Expressionsvektor kloniert. Diese Vektoren (pEFpuroCγ1V_{H}, pEFpuroCµV_{H} und pEFneoCκV_{L} enthalten den EF-1a-Promotor und den HCMV-Enhancer, das SV40-origin, das BGH-Polyadenylierungssignal, das Puromycin-Resistenzgen im Vektor für die schwere Kette und das Neomycin-Resistenzgen oder das Dehydrofolatreduktase-Gen im Vektor für die leichte Kette sowie die genomischen Sequenzen der humanen konstanten γ1 Region oder µ Region für die schwere Kette bzw. der humanen konstanten κ Region für die leichte Kette (Primer zur Amplifizierung aus genomischer humaner DNA und Vektorkarte siehe Abb. 4).

### 5. Eukaryontische Expression Core-1 spezifischer chimärer IgM und IgM Antikörper in CHO Zellen und deren Aufreinigung

Zur Expression der chimären Antikörper cigG-Karo4 bestehend aus den Sequenzen SEQ ID NO. 111 und 113 und cIgM-Karo4 bestehend aus den Sequenzen SEQ ID NO. 112 und 113 wurden CHOdhfr- Zelten (ATCC-Nr. CRL-9096) mit einem Gemisch der Vektoren für die schwere und die leichte Kette (1:3) durch Elektroporation (10⁶ Zellen/ml, 500 V, 60 µs) cotranstiziert und in Selektionsmedium (CHO-S-SFM II Medium (Life Technologies), HT Supplement (Biochrom), 400 µg/ml G418, 5 µg/ml Puromycin) 2 Wochen kultiviert. Nach Einzeizeliklonierung in einer 96-Loch-Platte wurden die Überstände im ELISA (Asialoglykophorin als Antigen, anti human Fcγ1- POD gekoppeltbzw. anti human Fc5µ- POD gekoppelt (Dianova) als Sekundärantikörper) getestet und der Klon mit der höchsten Antikörperproduktionsrate selektioniert (ca. 0,5 µg/10⁶ Zellen/24 h).

Zur Antikörperproduktion wurden die stabil transfizierten, den chimären IgG bzw. IgM sekretierenden CHO Zellen in Spinnerflaschen in CHO-SSFM II Medium, ergänzt durch HT Supplement, kultiviert, bis eine Zelldichte von ca 1 x 10⁶ Zellen/ml erreicht war. Nach Abtrennung der Zellen vom Zelikulturüberstand durch Zentrifugation (400xg, 15 min) wurde der chimäre Antikörper unter Verwendung einer Protein A - Säule (HiTrap rProtein A FF, Amersham Pharmacia Biotech) für den chimären IgG bzw. eineranti human Fc5µ- Antikörper Affinitätssäule aufgereinigt, Die durch pH-Sprung eluierte gereinigte Antikörperfraktion wurde unter Verwendung von Centriprep-Zentrifugenröhrchen (cut off 50 kDe, Millipore) in PBS umgepuffert und aufkonzentriert.

### 6. Angleichung der Sequenz der Core-1 spezifischen Antikörpersequenzen an humane Keimbahnsequenzen

Für die Angleichung der Core-1 bindenden Antikörpersequenzen an humane Sequenzen wurde in der Datenbank humaner Keimbahnsequenzen nach homologen Sequenzen gesucht und unter Verwendung der humanen Consensus-Sequenzen und den Erkenntnissen der kanonischen Struktur humaner Antikörper wurden humanisierte Core-1-bindende Sequenzen entwickelt Für die variable schwere Kette diente die humane Keimbahnsequenz VH-46 als Vorlage, für die variable leichte Kette die Sequenz A18.

Die humanisierten V_{H} bzw. V_{L} Sequenzen SEQ ID NO. 56 bis 79 bzw. 85 bis 94 wurden mit Hilfe der gene assembly PCR (singleoverlap extension PCR) hergestellt. Die PCR Reaktion erfolgte nach folgendem Schema: erste Denaturierung 94°C für 2min, dann 30 Zyklen Denaturierung bei 94°C für 45 sec, Annealing bei 55°C für 45 sec und Elongation bei 73°C für 1,5 min und am Ende einen Elongationsschritt bei 73°C für 7 min.

Die so hergestellten V_{H} und V_{L} Ketten wurden mit den Enzymen NcoI und XhoI bzw. NotI und XhoI geschnitten und zur Seqeunzierung in einen Klonierungsvektor (pLitmus 28 bzw. pBluescript KS) kloniert. Die richtigen V_{H} und V_{L} Ketten wurden anschließend erneut amplifiziert, um am 3'-Ende der V_{H} und am 5'-Ende der V_{L} eine Bbsl Schnittstelle einzufügen, um darüber die V_{H} und die V_{L} mit nur einem Alanin als Linker zu verknüpfen. Nach der Ligation wurden die kompletten scFv (die Ugationsprodukte) unter Verwendung derflanklerenden Primeramplifiziert und in einen bakteriellen. Expressionsvektor kloniert.

### 7. Spezifitätsanalyse der Core-1 spezifischen Erkennungsmoleküle im ELISA

Als Antigene wurden verschiedene Kohlenhydrat-PAA-Konjugate (Synthesome) und Glykoproteine verwendet: Asialoglykophorin (AGP), Glykophorin (GP) und Asialofetuine (Sigma); die PAA (Poly(N-(2-hydroxyethyl) Acrylamid)-Konjugate:Galβ1-3GaNAcα1-OC₃H₆NH-PAA und Galβ1-BGalNAcα1-*p*-OC₆H₄NH-PAA als Core-1 (alpha-Anomer) Konjugate mit unterschiedlichen Unkerlängen, Galβ1-3GalNAcβ1-OC₃H₆NH-PAA als beta-Anomer des Core-1, Galα1-3GalNAcα1-OC₃H₆NH-PAA und Galα1-3GalNAcβ1-OC₃H₆NH-PAA als weitere Stereoanomere des Core-1, die Core-2 Struktur Galβ1-3(GlcNAcβ1-6)GalNAcα1-OC₃H₆NH-PAA und die Derivate GalNAcα1-OC₃H₆NH-PAA, Neu5Acα2-3Galβ1-3GalNAcα1-OC₃H₆NH-PAA, Galβ1-3 (Neu5Acα2-6) GalNAcα1-OC₃H₆NH-PAA-, GlcNAcβ1-2Galβ1-3GalNAcα1-OC₃H₆NH-PAA, GlcNAca1-3Galβ1-3GalNAcα1-OC₃H₆NH-PAA, GalNAcα1-SGalβ1-OC₃H₆NH-PAA und 3'-O-Su-Galβ1-3GalNAcα1-OC₃H₆NH-PAA.

Aus den jeweiligen Stammlösungen (1mg in 1ml Bi-dest.H₂O), die portioniert bei -20°C aufbewahrt werden, wurde eine Verdünnung von 5 µg/ml in PBS hergestellt. Davon wurden 50µl/well in eine Mikrotiterplatte (NUNCLON-TC Microwell 96 F) pipettiert und die Testplatte 1 h bei 37°C und Ober Nacht bei 4°C inkubiert. Am nächsten Tag wurde die Testplatte mit PBS/0,2% Tween 3x gewaschen. Anschließend wurden mit 2% BSA in PBS unspezifische Bindungsstellen blockiert und 50 µl des ersten Antikörpers aufgetragen (chimärer IgG bzw. IgM: gereinigt 0,1 µg/ml in PBS/0,1% BSA oder unverdünnter Kulturüberstand produzierender CHOdhfr- Zellen; Multibodies: 10 µg/ml in PBS/0,1% BSA). Nach drei Waschschritten mit PBS/0,2% Tween wurden zum Nachweis der spezifisch gebundenen Antikörperkonstrukte die entsprechenden Zweit-Antikörper, Peroxidase-gekoppelt, eingesetzt (ein anti-Maus bzw. anti-human Fcγ1 bzw. µ -Antikörper für die ganzen Antikörper, ein anti-His Tag-Antikörper für Multibodies). Zum Nachweis des gebundenen sekundären Antikörpers erfolgte eine Farbreaktion mit TMB (3,3',5,5'-Tetramethylbenzidine). Nach 15 Minuten wurde die Reaktion durch Zugabe von 2,5N H₂SO₄ abgestoppt Die Messung erfolgte mit einem Mikrotiterplattenphotometer mit Filter 450nm im Dual-mode gegen einen Referenzfilter 630nm.

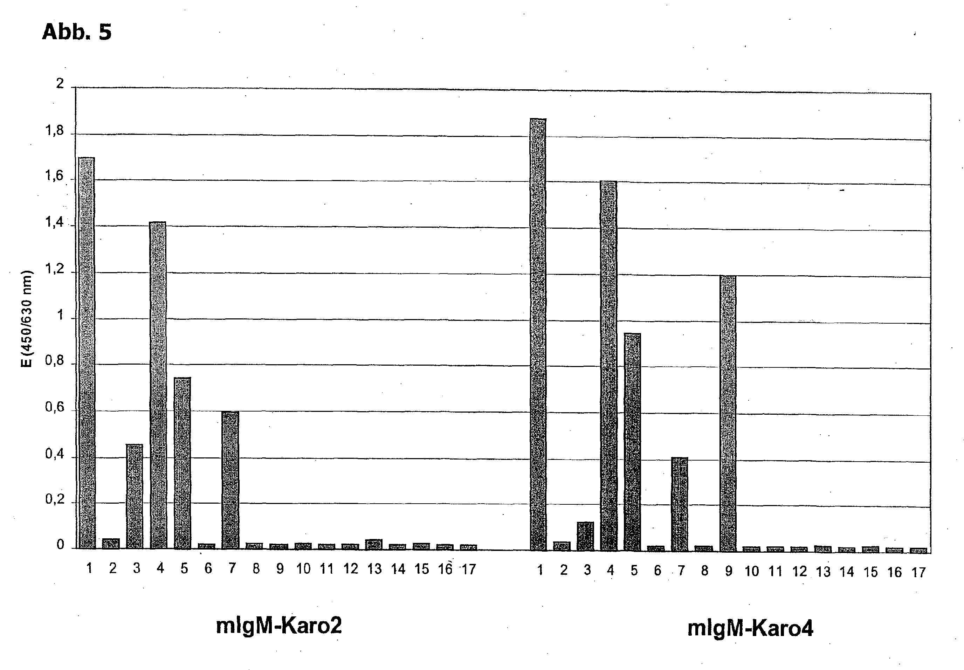

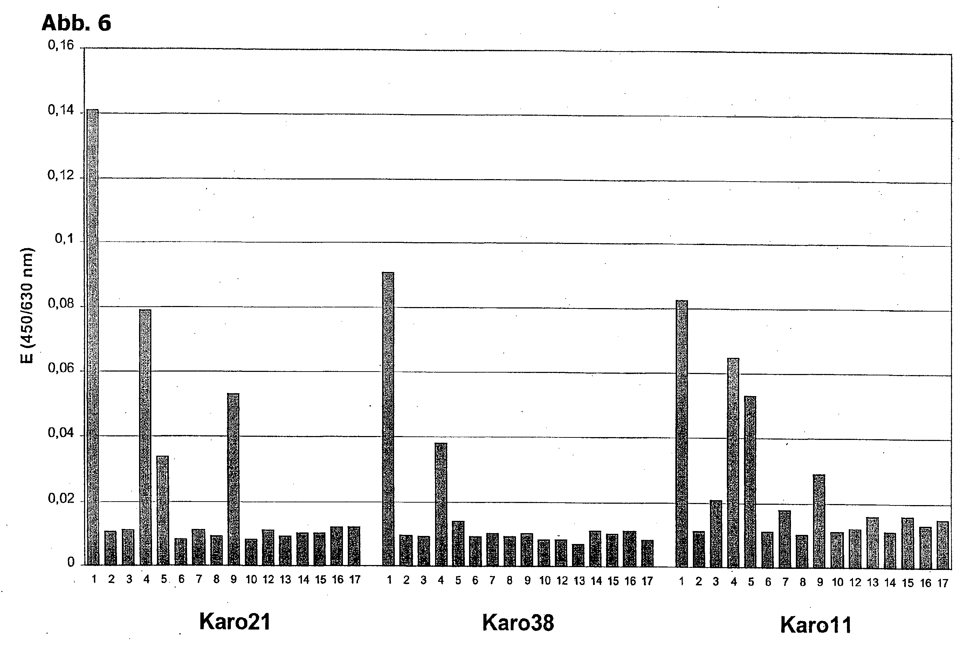

Repräsentative Ergebnisse sind in den Abbildungen 5 und 6 dargestellt. In Abbildung 5 sind zwei Erkennungsmoleküle mit variierenden Loop-Sequenzen im IgM-Format verglichen. Die Antikörperkonstrukte mIgM-Karo2 (SEQ ID NO. 107 und SEQ ID NO. 109) und mlgM-Karo4 (SEQ ID NO. 108 und SEQ ID NO.110) binden hochspezifisch an das Antigen Core-1, bevorzugt an das alpha-Anomer Galβ1-3GalNAcα und schwächer an das beta-Anomer Galβ1-3GalNAcβ. Die Erkennungsmoleküle können auch nurdas alpha-AmomerGalβ1-3GalNAcα oder beide Anomere Galβ2-3GalNAcα und Galβ1-3GalNAcβ in gleicher Weise binden. Zusätzlich bindet mIgM-Karo4 die Core-2 Struktur Galβ1-3 (GlcNAcβ1-6)GalNAcα. Alle anderen getesteten Kohlenhydratstrukturen, auch strukturell stark verwandte Strukturen werden durch die hier beanspruchten Bindungsproteine nicht erkannt Als Core-1 tragendes Glykoprotein zeigt AGP ein starkes Signal mit beiden Varianten, wobei das ebenfalls Core-1 tragende Glykoprotein Asialofetuln deutlich stärker mit der Karo2-Variante reagiert, was sehr wahrscheinlich mit der unterschiedlichen Core-1 Dichte in beiden Proteinen zusammenhängt Abbildung 6 zeigt das Spezifitätsmuster der beispielhaft ausgewählten humanisierten Erkennungsmoleküle Karo11 (SEQ ID NO. 56 und SEQ ID NO. 90), Karo21 (SEQ ID NO. 59 und SEQ ID NO. 90) und Karo38 (SEQ ID NO. 69 und SEQ ID NO. 90) mit variierenden Gerüstsequenzen im scFv-Format mit einer Aminosäure als Linker. Auch hier zeigt sich das gleiche Spezifitätsmuster, wie in der Definition der Core-1 spezifischen Bindung im Sinne der Erfindung beschrieben (siehe oben).

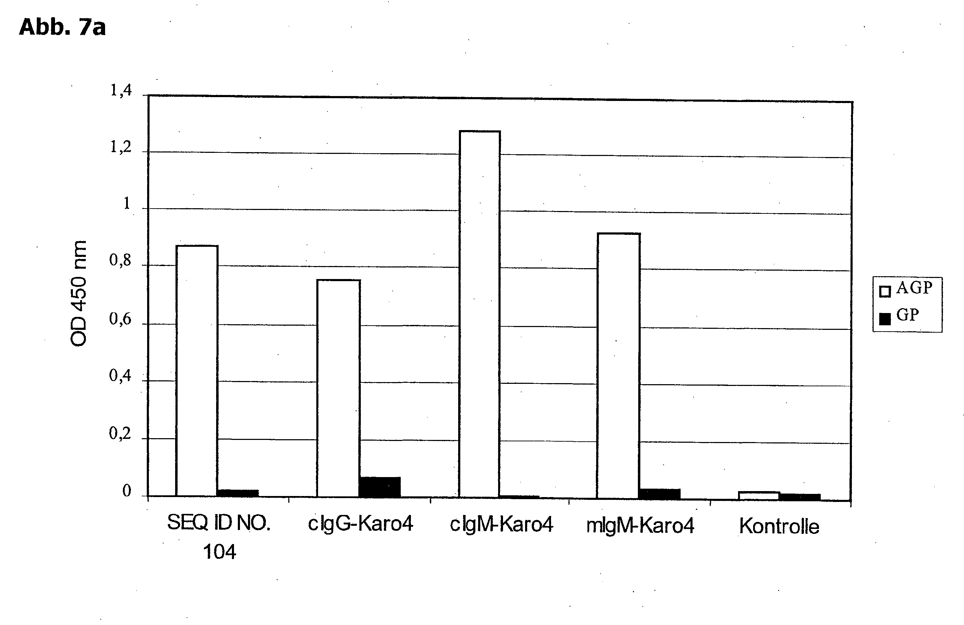

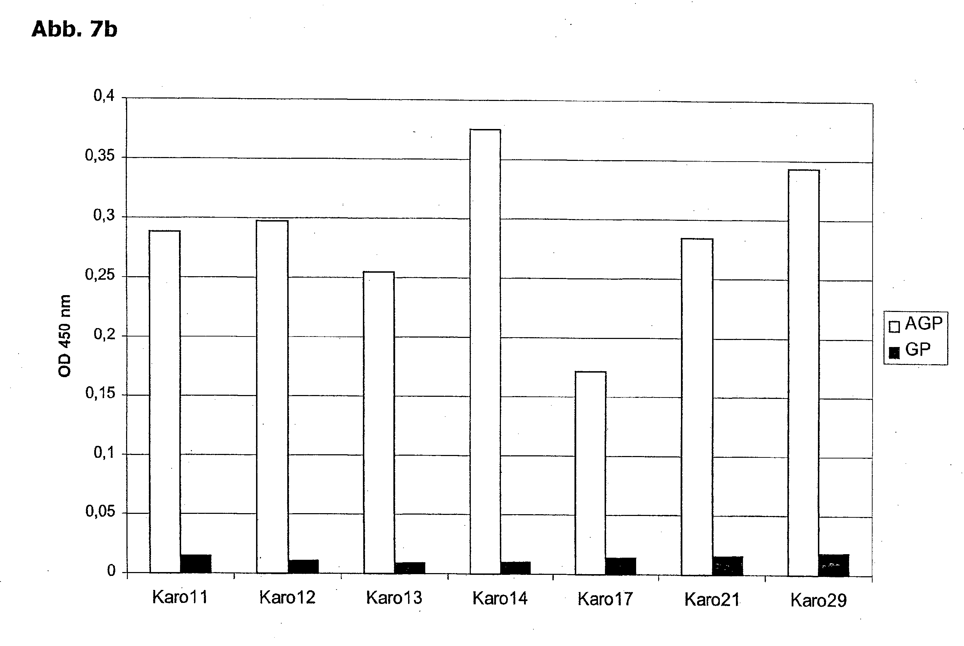

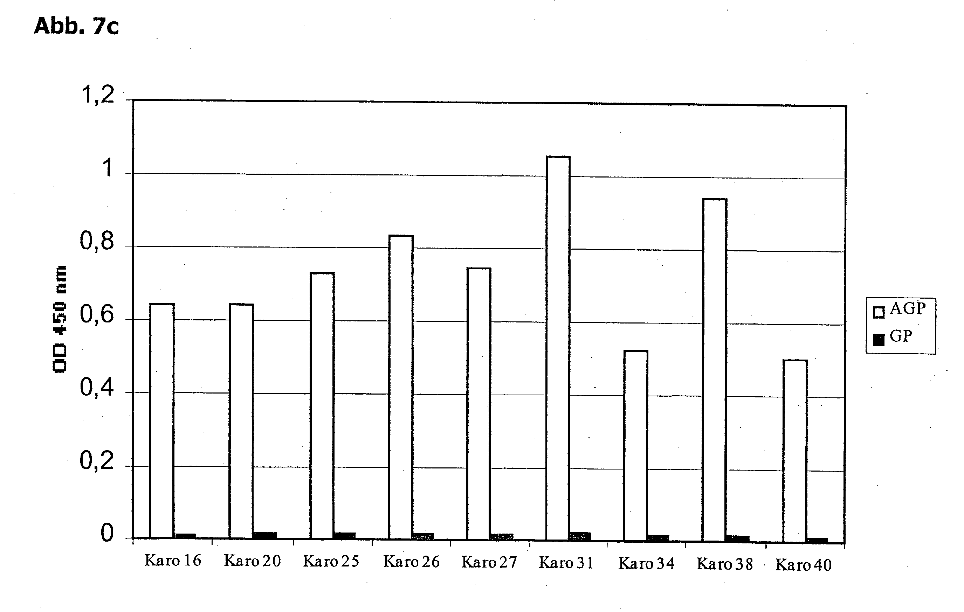

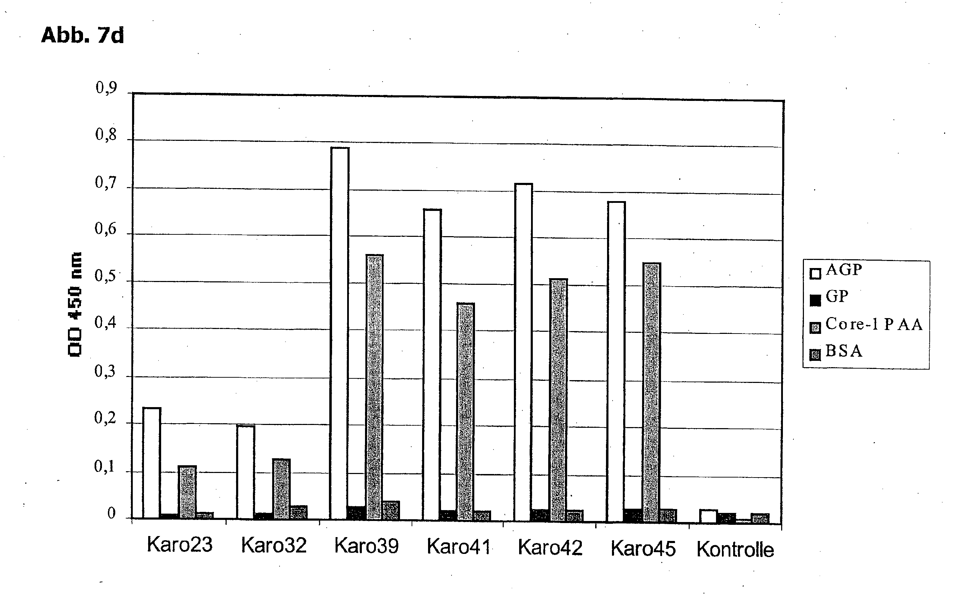

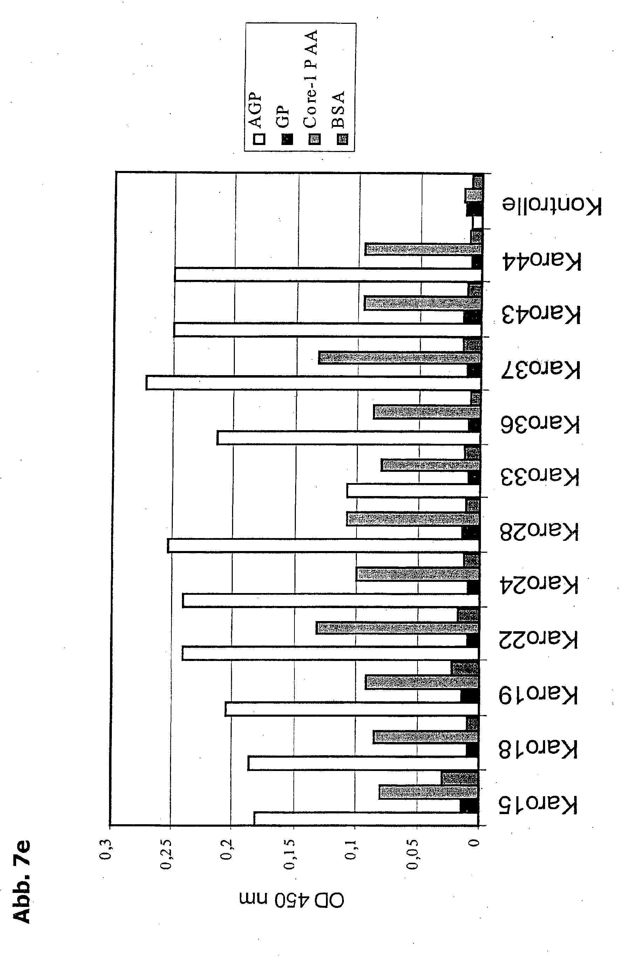

Die spezifische Bindung der verschiedenen bevorzugten Formate und Kombinationen im ELISA, beispielhaft an AGP, GP und/oder Galβ1-3GalNAcα1-OC₃H₆NH-PAA, ist in den Abbildungen 7 a bis e dargestellt.

### 8. Immunhistologische und Immmunzytologische Färbungen

Für die immunhistologischen Färbungen wurden Gefrierschnitte entsprechender Gewebeproben luftgetrocknet und mit 10% Formaldehyd in PBS 15 min fixiert. Zur Reduktion der endogenen Peroxidase-Aktivität wurden die Schnitte mit 3% Wasserstoffperoxid in PBS behandelt und nach Blockierung unspezifischer Bindungsstellen mit präabsorbiertem Keninchenserum an Neuraminidase-behandelten Erythrozyten mit einem Core-1 spezifischen Primärantikörper inkubiert. Anschließend wurden die Präparate mit einem entsprechenden Sekundärantikörper (anti-Maus bzw. anti-human IgG oder IgM, POD gekoppelt) inkubiert. Die Farbreaktion erfolgte unter Verwendung des Peroxidas-Substrats Diaminobenzidin und die Gegenfärbung mit Hämatoxylin.

Das beispielhafte Erkennungsmolekül mlgM-Karo4 reagiert nur mit sehr wenigen Strukturen im Normalgewebe. Diese befinden sich aber in für einen Antikörper nicht zugänglichen Bereichen (Tabelle 3).

**Tabelle 3: Reaktion von humanem Normalgewebe mit dem Core-1 spezifischen AntikörpermIgM-Karo4**

| Gewebetyp | | Reaktivität |
|---|---|---|
| Epidermis - Basalmembran | | negativ |
| Magen | | |
| | Foveola Epithelium | negativ |
| | Furndusdrüsen | negativ |
| | Korpusdrüsen | negativ |
| Colon Mucosa | | negativ |
| Milz | | |
| | Trabeculae lienis | negativ |
| | Retikularzellen | negativ |
| | Lymphozyten | negativ |
| Endothellum | | negativ |
| Prostata | | negativ |
| Leber | | |
| | Hepatozyten | negativ |
| | Kupffer-Zellen | negativ |
| | Gallenwege | negativ |
| Lymphknoten | | |
| | Lymphozyten | negativ |
| | Retikularzellen | negativ |
| Gallenblase | | negativ |
| Nebenniere | | |
| | Nebennierenrinde | negativ |
| | Nebennierenmark | negativ |
| Blase | | negativ |
| Herz | | negativ |
| Bauchspeicheldrüse | | |
| | Drüsengänge | positiv |
| | Azinl | negativ |
| | Langerhans'sche inseln | negativ |

Die Erkennungsmoleküle reagieren positiv mit einer Vielzahl von Karzinomen. Die Daten in Tabelle 4zeigen, dass die Core-1 spezifischen Erkennungsmoleküle einen großen Prozentsatz an Tumorpatienten einer Indikation erkennen, der von indikation zu indikation unterschiedlich ist.

**Tabelle 4: Reaktion von humanem Tumorgewebe mit dem Core-1 spezifischen Antikörper mIgM-Karo4**

| | **Gewebetyp** | **Reaktivität** |
|---|---|---|
| Colonkarzinom | | |
| | primäres Karzinom | 31/52 |
| | Lebermetastasen | 20/22 |
| Lungenkarzinom | | |
| | GroBzeiliges | 3/8 |
| | Bronchoalveolar | 1/1 |
| | Adenokarzinom | 6/6 |
| Blasenkarzinom | | 5/9 |
| Magenkarzinom | | |
| | Intestinaler Typ | 8/8 |
| | Diffuseer Typ | 3/3 |
| Prostatakarzinom | | 9/9 |
| Mammakarzinom | | |
| | Intraductal/ductal | 8/10 |
| | Schwach differenziert | 2/5 |
| | Muzinöses | 1/1 |
| Schilddrüsenkarzinom | | 0/10 |
| Nierenkarzinom | | |
| | Klarzelliges | 4/9 |
| | Transitionalzeiliges | 2/5 |
| Zervikalkarzinom | | 1/2 |
| Ovarialkarzinom | | |
| | Adenokarzinom | 2/2 |
| | Endometriolde | 2/2 |
| | Teratom | 2/2 |
| Glioblastom | | 0/3 |

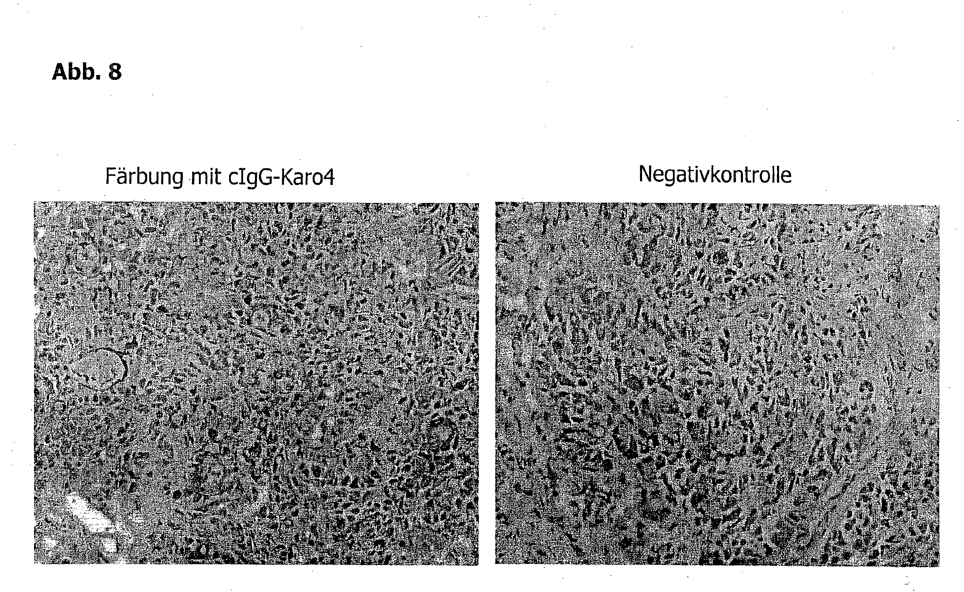

Für die Entwicklung eines Maus-Tumormodells wurden verschiedene Xenotransplantate untersucht. Bei den Xenotransplantaten handelt es sich um humanes Kolonkarzinomgewebe, das auf Nacktmäusen mehrfach passagiert wurde. Abbildung 8 zeigt beispielhaft eine immunhistochemische Färbung eines Xenotransplantat-Präparats mit dem Core-1 spezifischen Antikörper cigG-Karo4.

Für die Immunzytologischen Färbungen wurde die Immunfluoreszenz eingesetzt Dafür wurden die entsprechenden Zellen auf Objektträger angetrocknet und mit 5% Formaldehyd 10 min fixiert. Nach Blockierung unspezifischer Bindungsstellen mit BSA (1 % in PBS) wurden die Zellen mit dem Primärantikörper inkubiert Anschließend wurde 3 mal mit PBS gewaschen und mit dem entsprechenden fluoreszenz-markierten Sekundärantikörper (anti-Maus oder antihuman IgG bzw. IgM für ganze Antikörper; anti-Myc-Tag oder anti-His-Tag Antikörper für die single chain Antikörperfragmente) inkubiert. Nach mehrmaligem Waschen mit PBS wurden die Zellen in Mowiol eingebettet.

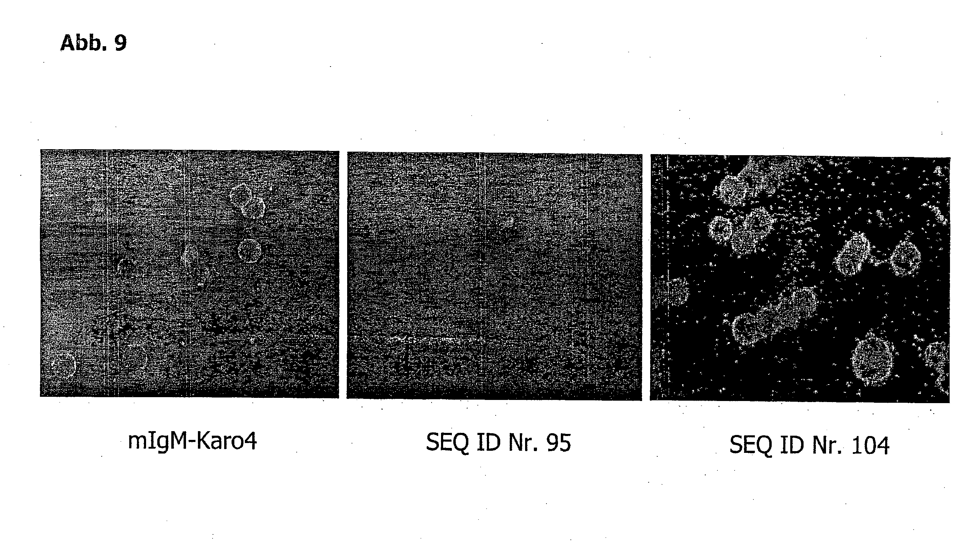

Es wurden verschiedene Zeillinien mit Core-1 spezifischen Erkennungsmolekülen in der immunfluoreszenz getestet Eine Reihe von Tumorzeillinien und auch einigen Leukämie-Zeillinien reagieren positiv (Tab. 6 und Abb. 9).

**Tabelle 5: Reaktivität verschiedener Zeillinien mit den Core-1 spezifischen Antikörpern migM-Karol bzw. migM-Karo4**

| **Zeillinien** | **Reaktivität** |
|---|---|
| KG-1 | positiv |
| ZR-75-1 | positiv |
| T47D | (positiv) |
| | wenige Zellen |
| U268 | negativ |
| LN78 | positiv |
| HT29 | positiv |
| HCT116 | negativ |
| HepG2 | negativ |
| K562 | negativ |
| NM-D4 | positiv |

Abbildung 9 zeigt beispielhaft eine Fluoreszenzmarkierung von KG-1 Zellen, einer akuten myeloischen Leukämie-Zeillinie, mit verschiedenen Antikorperkonstrukten, einem murinen IgM und zwei scFv-Antikörpem mit unterschiedlicher Linkerlänge (SEQ ID NO. 95 mit 18 Aminosäuren und SEQ ID NO. 104 mit einer Aminosäure als Unker). Alle drei Konstrukte zeigen eine spezifische Färbung der Tumorzeillinie, wobei das monovalente Antikörperfragment SEQ ID NO. 95 das schwächste Signal zeigt.

### 9. Chelatisierung und radioaktive Markierung von Antikörpern und Antikörperfragmenten

Durch Konjugation wurde an den Antikörper clgG-Karo4 bzw. den Multibody mit der Sequenz SEQ ID NO.104 ein Chelator kovalent gebunden, der die Bindung eines Radiometalls ermöglicht. Als Chelatoren kamen die kommerziellen Produkte der Firma Macrocyclics (Dallas, USA), p-isothiocyanatobenzyldiethylenetriaminepentaacetic acid (p-SCN-Bz-DTPA) und p-isothiocyanatobenzyl-1,4,7,10-tetraazacyclododecane-1,4,7,10-tetraacetic acid (p-SCN-Bz-DO-TA) zum Einsatz. Beide Chelatoren eignen sich zur Kopplung an Antikörper zu deren Radioaktivmarkierung [Brechbiel et al., 1986; Kozak et al., 1289, Stimmel et al., 1995].

Die Konjugation erfolgte durch Reaktion der isothiocyanatgruppe des Chelators mit einerfreien ε-Aminogruppe der Aminosäure Lysin am Antikörper. Es entsteht eine kovalente N-C-Bindung zwischen Chelator und Antikörper.

Der gereinigte Antikörper bzw. das gereinigte Antikörperfragment muß zunächst in Kopplungspuffer pH 8,7 umgepuffert werden. Hierzu wurde eine Ultrafiltration in einer Fiftrationshüise (Centriprep YM50 (Amicon)) durchgeführt. Dies erfolgte durch mehrmalige Verdünnung mit 10-fachem Volumen und Filtration durch eine Membran mit definierter Porengröße durch Zentrifugation. Dadurch wurde PBS durch den alkalischen Kopplungspuffer (0,05 M Natrium-Carbonat, 0,15 M Netriumchlorid, pH 8,7) ersetzt

Die Chelatisierung wurde mit den bifunktionellen Chelatoren p-SCN-Bz-DTPA- bzw. p-SCN-Bz-DOTA durchgeführt. Zur Chelatisierungsreaktion wurden Protein (1-10 mg/ml) in Kopplungspuffer und eine Lösung des Chelators von 1 mg/ml in 2% DMSO/Wasser so gemischt, dass ein molarer Überschuß des Chelators gewährleistet war. Es folgte eine inkubation der Mischung von 1h bal 37°C. Anschließend wurde nicht gebundener Chelator durch Ultrafiltration im gleichen Gefäß (Centriprep YM50 (Amicon)) abgetrennt und wie oben beschrieben in den zur Radioaktivmarklerung notwendigen Beladungspuffer auf pH 4,2 umgepuffert (0,15 M Natriumacetat, 0,15 M Notriumclorid, pH 4,2). Die Proteinkonzentration Während und nach diesem Schritt wurde wieder auf 1-10mg/ml mit Hilfe einer UV-Messung bei 280nm eingestellt.

Es waren Bedingungen für die Chelatisierungsreaktion zu finden, die eine Radiomarkierung des Antikörpers erlauben, ohne dessen Bioaktivität wesentlich zu mindern.

Der chelatisierte Antikörper wurde mit einem Radiometall beladen, wodurch der Radio-Antikörper erzeugt wurde. Zur Beladung wurden die isotope ¹¹¹Indium und ⁹⁰Yttrium verwendet. Beide haben chemisch und physikochemisch vergleichbare Eigenschaften und werden durch den Chelator als dreiwertige Ionen gebunden (¹¹¹In³⁺, ⁸⁰Y³⁺). Der mit ¹¹¹indium markierte Antikörper ist ein γ-Strahler und wird in der Klinik zur individuellen Dosisfindung für den Patienten verwendet, während ⁹⁰Yttrium ein β-Strahier ist, der therapeutisch zum Einsatz kommt Die Halbwertzeiten betragen für ¹¹¹In 67 Stunden und für ⁹⁰Y 84 Stunden.

Zur Beladung wurde ¹¹¹Indiumchlorid der Firma NEN (Perkin Elmer, Belgien) verwendet. Die Lieferung des Radiometalls erfolgt in salzsaurer Lösung. Diese ¹¹¹InCl₃ Lösung wurde zunächst kurzzeitig auf eine HCl-Konzentration von 1 M gebracht Anschließend wurde mit 0,05M HCl auf eine spezifische Aktivität von 80-320mCl/ml verdünnt und davon ein Aliquot zum Einbau in den chelatisierten Antikörper verwendet, wobei das zugegebene Volumen HClsaurer ¹¹¹InCl₃-Lösung gleich dem Volumen der vorgelegten Antikörperlösung im Kopplungspuffer pH 4,2 sein sollte, um die pH-Stabilität zu gewährleisten. Inkubationsdauer war 1 h bei 37°C mit gelegentlicher vorsichtigem Mischen.

Im Anschluß daran wurde der Filtereinsatz wieder in die Filtrationshülsa eingesetzt und wie oben beschrieben in Phosphatpuffer pH 7,2 mit physiologischem Gehalt an Kochsalz umgepuffert. Dabei erfolgte eine Trennung von hochmolekularem radioaktiv markiertem Antikörper und ungebundenem ¹¹¹InCl₃. Die Quantifizierung des Einbaus von ¹¹¹In in den chelatisierten Antikörper erfolgte dünnschichtchromatographisch. Die Einbaurate des Radiometalis lag bei 70-99% der eingesetzten Radioaktivität.

### 10. Nachweis des Core-1 positiven, sekretorischen MUC1 im Sandwich-ELISA

Core-1 positives, sekretorisches MUC1 kann im Sandwich-ELISA nachgewiesen werden. Debel dient ein MUC1 spezifischer Antikörper als Fänger-Antikörper von MUC1 und ein Core-1 spezifischer Antikörper zum Nachweis des Core-1 Antigens. Ein dritter Enzym- oder Fluoreszenz-gekoppelter Antikörper muss zur Detektion des Zwettantikörpers eingesetzt werden.

Als Beispiel wurden die Überstände zweier Tumorzeillinien analysiert (K562 und T470). Die Ergebnisse sind in Tabelle 6 dargestellt. 10⁵ Zellen pro ml Zellkulturmedium wurden ausgesät, 4 Tage ohne Mediumwechsel kultiviert, anschließend ein Aliquot abgenommen und der Zellkulturüberstand durch Zentrifugation vom Zellpellet getrennt. 50 µl dieser Überstände wurden unverdünnt im ELISA eingesetzt. Der anti-MUCI-anti-Core-1-Sandwich-ELISA wurde durchgeführt, indem die Mikrotiterplatte mit dem Fänger-Antikörper (1 µg/ml) in PBS über Nacht bei 4 °C beschichtet wurde. - Es wurden drei verschiedenen Konzentrationen des Antikörpers für die Beschichtung getestet (1 µg/ml, 2 µg/ml und 4 µg/ml.. Die Beschichtung mit 1 µg/ml erwies sich im Sandwich ELISA als am empfindlichsten.- Anschließend wurden die beschichteten Platten zweimalmit PBS gewaschen und 1,5 h in 5% BSA, 0,05%Tween 20 in PBS bel Raumtemperatur blockiert. Der Biockierungspuffer wurde entfernt, die Platten erneut einmal mit 0,1% Tween 20 in PBS (Waschpuffer) gewaschen, die Proben dazugegeben und 1,5 h bei Raumtemperatur inkubiert. Als Negativkontrollen wurden das Zellkulturmedium oder 2% BSA in Waschpuffer (Verdünnungspuffer für Zweitantikörper) verwendet Eine Positivkontrolle stand nicht zur Verfügung. Nach dreimaligem Waschen erfolgte die Neuraminidase Behandlung in den dafür vorgesehenen Wells. Zu diesem Zweck wurde die Neuraminidase-Lösung (DADE Behring, Deutschland) in Immidazolpuffer (0,68 g Immidazol, 0,19 g CaCl₂ und 0,4 g NaCl In 100 ml H₂O, pH 6,8) 1:5 verdünnt und 50 µl/well 30 min bei 37 °C inkubiert. Als Kontrolle wurde der Imidazolpuffer ohne Neuraminidase-Lösung in einem entsprechenden Well inkubiert. Anschließend wurden die Wells dreimal gewaschen und der migM-Karo4 Antikörper zum Nachweis des Core-1 Antigens in einer 1:500 Verdünnung in 2% BSA in Waschpuffer dazugegeben und eine weitere Stunde bei Raumtemperatur inkubiert. Nach erneutem dreimaligen Waschen folgte die Zugabe eines Peroxidasegekoppelten anti Maus IgM(µ) Antikörpers (Dianova) 1:5000 verdünnt in 2% BSA in Waschpuffer und eine Inkubation von 1 h bei Raumtemperatur. Abschließend wurden die Platten zweimal in Waschpuffer und einmal in PBS gewaschen. Die Färbereaktion erfolgte in 25 mM Zitronensäure, Phosphatpuffer pH 5,0 mit 0,04% H₂O₂ und 0,4 mg/ml o-Phenylendiamin (Sigma) im Dunkeln bei Raumtemperatur. Die Farbreaktion wurde durch Zugabe von 2,5 N Schwefelsäure (Endkonzentration 0,07 N) gestoppt und im ELISA-Reader bei 492 nm mit einem Referenzfilter von 620 nm vermessen.

**Tabelle 6: Analyse von Core-1 positivem MUC1 in Kulturüberständen zweler zeillinien mit und ohne Neuraminidase Behandlung im Sandwich ELISA**

| Zeillinie | Signal | |
|---|---|---|
| | - NeuAcdase | +NeuAcdase |
| K562 | - | + |
| T47D | + | +++ |

### 11. Effektive Bindung von radioaktiv-markierten Core-1 spezifischen Erkennungsmolekülen an Tumorzellen

Die Core-1 positive Tumorzeillinie NM-D4 [DSMZ deposit NO. DSM ACC2605] (vergleiche Tabelle 5) wurden zur Testung der Bindungsfähigkeit der radiomarkierten Erkennungsmoleküle an Core-1 positive Tumorzellen herangezogen. Dabei wurde jeweils in Dublettbestimmungen eine bestimmte Anzahl Zellen in einem 1,5 ml Gefäß vorgelegt und mit steigenden Mengen Antikörper inkubiert. Nach dem Waschen wurde anhand der Zählrate bestimmt, wieviel Antikörper gebunden hat.

Pro Ansatz werden 2x10⁸ Zellen benötigt. Nach Vorinkubation der Zellen für eine Stunde auf Eis wurde die benötigte Menge Zellen in Reaktionsgefäße vorgelegt, zentrifugiert (5min 1000xg, 25°C) und der Überstand abgenommen. Dann wurde mit PBS/0,1%Tween20/1%BSA aufgefüllt bis zum Volumen 200µl abzüglich der noch zuzugebenen Menge Erkennungsmclekül. Anschließend wurde das entsprechende ¹¹¹In-markierte Erkennungsmolekül (siehe Belspiel 9) zum Endvolumen 200 µl zugegeben (ca. 0,5 bis 20 µg, je nach Erkennungsmolekül) und die Ansätze für eine Stunde bei 4-8°C inkubiert. Nach Zentrifugation (4min, 1000xg, 25°C) wurde der Überstand abgenommen und das Zellpellet in 400µl PBST/1 %BSA vorsichtig resuspendiert. Nach nochmaligem Waschen wurde das Zellpellet im Gefäß am gamma-Counter gemessen. Von den Ausgangslösungen definierter Konzentration wurden die spezifischen Zählraten bestimmt, der Wert in cpm/ng wurde der Relativierung der Messwerte von gebundenem Antikörper- zugrunde gelegt. Die freie Bindung ergibt sich aus der Differenz aus Gesamtmenge und gebundener Menge Antikörper. Diese Werte wurden als Verhältnis gebunden/nichtgebunden gegen die Menge gebunden in ein Diagramm aufgetragen und im linearen Bereich der Kurve die Steigung ermittelt und der Abzissen-Schnittpunkt bestimmt (Scatchard Analyse). Der Abzissen-Schnittpunkt bezeichnet die Anzahl der Bindungsplätze/Zelle. Aus der Steigung der Geraden ergibt sich die Assoziationskonstante Kass in [M⁻¹].

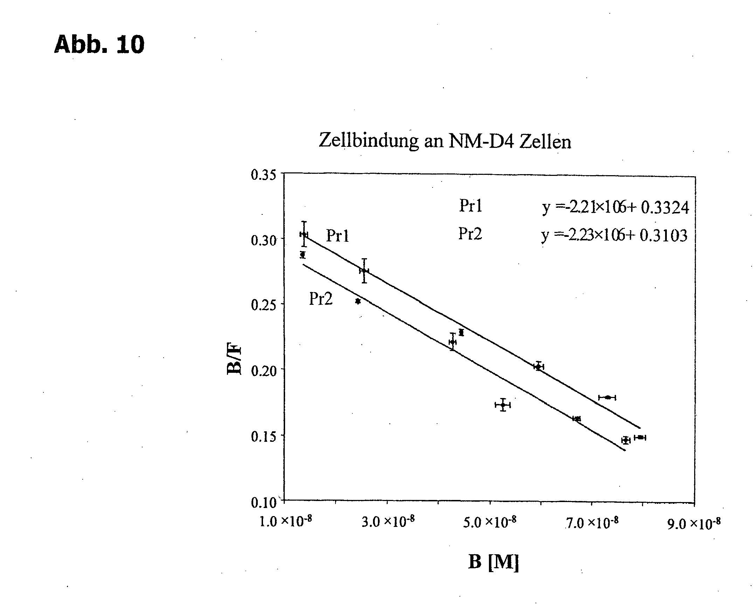

In Abbildung 10 ist beispielhaft die Scatchard-Analyse der Bindung des radioaktiv-markierten Erkennungsmoteküls im scFv-Format mit der Sequenz SEQ ID NO. 104 mit einer Aminosäure als Linker an NM-D4 Zellen dargestellt (zwei verschiedene Präparationen).

In Tabelle 7 sind die Assozistionskonstanten und die Anzahl der Zellbindungsplätze verschiedener Core-1 spezifischer Multibodies an NM-D4 Zellen zusammengefaßt.

**Tabelle 7: Zellbindungstest und Scatchard-Analyse mit ¹¹¹in-markierten Erkennungsmolekülen an NM-D4 Zellen.**

| **Antikörper** | **Kass [M-1]** | **Anzahl Bindungsplätze/Zelle** |
|---|---|---|
| SEQ ID NO. 105 | 1,1x10⁷ | 4,8x10⁶ |
| SEQ ID NO. 104 | 2,1x10⁶ | 8,1x10⁶ |
| SEQ ID NO. 103 | 1,2x10⁸ | 9,2x10⁸ |

### 12. Anreicherung der radioaktiv-markierten Core-1 spezifischen Erkennungsmoleküle an Core-1 positiven Tumoren in einem in vivo Tumormodell

Als Tumormodell wurden ZR-75-1 Zellen subkutan in Nacktmäuse (Ncr: nu/nu, welblich) injiziert Nach ca. 3-4 Wochen ist der Tumor unter der Haut tastbar. Den Tumor-tragenden Mäusen (pro Zeitpunkt n=4) wurden jeweils 5 µg ¹¹¹In-markierter Multibody (SEQ ID NO. 104 bzw. SEQ ID NO. 105) in 200 µl i.v. in die Schwanzvene verabreicht. Nach 24h wurden die Mäuse getötet und die Verteilung der Radioaktivität im Tumor, im Serum und den Organen ermittelt. Tabelle 8 zeigt die spezifische hohe Anreicherung der Multibodies im Tumor (in %ID/g Tumor bezogen auf die Injizierte Dosis und das Tumorgeweicht) im Vergleich zum Serum und den Organen.

**Tabelle 8: Biodistribution ¹¹¹In-markierter Erkennungsmoleküle in Tumor-tragenden Mäusen.**

| | **SEQ ID NO. 104** | **SEC ID NO. 105** |
|---|---|---|
| Serum (%ID/ml) | 1,4 ± 0,16 | 1,0 ± 0,24 |
| Tumor (%ID/g) | 10,8 ± 2,88 | 8,1 ± 1,45 |
| Leber (%ID/g) | 3,7 ± 0,15 | 5,3 ± 0,92 |
| Lunge (%ID/g) | 1,7 ± 0,11 | 1,9 ± 0,19 |
| Herz (%ID/g) | 1,5 ± 0,06 | 1,9 ± 0,19 |
| Milz (%ID/g) | 5,4 ± 0,75 | 6,7 ± 1,07 |
| Gehlm (%ID/g) | 0,1 ± 0,01 | 0,1 ± 0,00 |
| Knochenmark (%ID/g) | 1,0 ± 0,16 | 1,7 ± 0,90 |

### 13. Theraplestudie zur Reduktion von Core-1 positiver Tumoren mit radioaktiv-markierten Core-1 spezifischen Erkennungsmolekülen in einem in vivo Tumormodell

Die Theraplestudien wurden unter Verwendung des gleichen etablierten ZR-75-1-Tumormodells durchgeführt, wie für die Biodistributionstudien beschrieben (siehe Beispiel 12). Die chelatisierten Erkennungsmoleküle (siehe Beispiel 9) wurden hierfür mit ⁹⁰Yttrium (ein β-strahler zur Zerstörung der Tumorzellen) beladen (pH 4,5, 37°C, 30 min; vergleiche Einbau von ¹¹¹indium) und in der Dünnschichtchromatographie die Stabilität kontrolliert. Den Tumor-tragenden Mäusen (ca. drei Wochen nach der subkutanen Injektion der ZR-75-1 Zellen) wurden 200 µl i.v, in die Schwanzvene verabreicht. Die Injektionslösung enthielt den ⁸⁰Y-markierten Multibody (bis maximal 100 µCl pro Dosis) in Ca/Mg-PBS mit 0,2 bis 4% fötales Kälberserum zum Schutz vor Radiolyse. Kontrollgruppen erhielten die gleiche injektion ohne radioaktivmarkiertem Erkennungsmolekül. Körpergewicht und Tumorgröße wurden zweimal wöchentlich gemessen und verglichen. Das relative Tumorwachstum wurde unter Berücksichtigung der Jeweilligen Tumorgröße zu Beginn der Behandlung bestimmt. Eine zweite Injektion wurde drei Wochen nach der ersten Behandlung injiziert. Durch geeignete Behandlung konnte das Tumorwachstum signifikant zur Kontrollgruppe reduziert werden.

### Legende zur den Abbildungen

- **Abb.1a:**: **Sequenzen der Linker in den verschiedenen Multibody single chain Antikörperfragmenten.**
- **Abb. 1b:**: **Klonierungsschema zur Herstellung von single chain Antikörperfragmenten mit unterschiedlicher Linkerlänge.**
- **Abb. 2:**: **Vektor zur Klonierung und bakterlellen Expression von single chain Antikörperfragmenten.**
- **Abb. 3:**: **Analyse von Multibodies im scFv Format mit unterschledlicher Linkerlänge im ELISA.**
Multibodies mit den Aminosäuresequenzen SEQ ID NO. 95, 96, 97, 98, 99, 100, 101, 103, 104 und 105 wurden wie oben beschrieben in E.coli exprimiert und die Periplasmafraktionen gewonnen. Als Antigen für den ELISA Test wurde Aslaloglykophorin, ein Core-1 tragendes Glykoprotein, eingesetzt. Eine schrittweite Linkerverkürzung führt zu einer Erhöhung der Bindung an Asialoglykophorin. Die besten Bindungseigenschaften zeigen die Varianten mit der SEQ ID NO. 104 und 105. Diese multivalenten Konstrukte im Dia/Triabody Format sind bevorzugte Ausführungsformen der Erfindung.
- **Abb.4:**: **Vektorsystem zur Klonierung und eukaryontischen Expression von chimäre Antikörpern im IgG₁ oder IgM-Format.**
- **Abb. 5 und 6:**: **Spezifltätsanalyse im ELISA.**

Als Antigene wurden verschiedene Glykoproteine und Kohlenhydrat-PAA-Konjugate verwendet Asialoglykophorin [1]; Glykophorin [2]; Asialofetuine [3]; *Gal*ß1-3GalNAcαl-OC₃*H*₆NH-PAA **[4]**; Galß1-3GalNAcα1-*p*-OC₈H₄NH-PAA **[5];** Galα1-3GalNAcα1-OC₃H₆NH-PAA [6]; Galβ1-3GalNAcß1-OC₃H₆NH-PAA **[7]**; Galα1-3GalNAcB1-OC₃H₆NH-PAA **[8];** Galß1-3(GlcNAcß1-6)GalNAcα1-OC₃H₆NH-PAA **[9];** GalNAcα1-OC₃H₆NH-PAA **[10];** Neu5Acα2-3Galß-3GalNAcα1-OC₃H₆NH-PAA **[11]**; Galß1-3 (Neu5Acα2-6)GalNAcα1-OC₃H₆NH-PAA **[12]**; GlcNAcß1-2Galß1-3GalNAcα1-OC₃H₈NH-PAA **[13]**; GlcNAca1-3GalNAcα1-3GalNAcα1-OC₃H₆NH-PAA **[14]**; GalNAcα1-3Galß1-OC₃H₆NH-PAA **[15]**; und 3'-O-Su-Galß1-3GalNAcα1-OC₃H₆NH-PAA **[16]**. Als Kontrolle wurde BSA [17] verwendet. In **Abbildung 5** wurden zwei Antikörper im IgM-Format mit unterschledlicher CDR-Sequenz-Zusammensetzung eingesetzt. **Abbildung 6** zeigt das Spezifitätsmuster von drei humanisterten Erkennungsmolekülen im scFv-Format mit variierenden Gerüstsequnezen.
- **Abb. 7:**: **Spezifische Bindung verschiedener bevorzugter Formate und Kombinationen der Erkennungsmoleküle im ELISA, beispielhaft an den Antigenen AGP, GP und/oder Core-1-PAA (Galß1-3GalNAcα1-OC₃H₆NH-PAA).**
- **Abb. 8:**: **immunhistochemische Färbung von Xenotransplantat-Präparaten.**

Humanes Colonkarzinomgewebe wurde auf Nacktmäuse transplantiert und nach Erreichen einer bestimmten Größe passagiert. Das Tumorgewebe wurde eingebettet und geschnitten und für immunhistochemische Färbungen eingesetzt. In a) wurde das Gewebes mit clgG-Karo4 als Primärantikörper und einem anti-human Fcγ Antikörper- POD gekoppelt als Sekundärantikörper markiert. Die braune Färbung kennzeichnet die Core-1-positiven Strukturen.
- **Abb. 9:**: **Fluoreszenzmarkierung von Zellen der Tumorzeillinie KG-1 mit verschledenen Core-1 spezifischen Erkennungsmolekülen.**
- **Abb.10:**: **Scatchard-Diagramm zur Analyse der Zellbindung radioaktive markierter Core-1 spezifischer Erkennungemoleküle.** Beispielhaft sind hier die Bindungsdaten des Multibodies SEQ ID NO. 104 mit einer Linkerlänge von einer Aminosäure dargestellt (Pr1 und Pr2 entsprechen zwei verschiedenen Präparationen). B: an die Zellen gebundener Anteil [M], F: freie Bindung als Differenz aus Gesamtmenge und gebundener Menge Antikörper [M]. Oben ist die entsprechende Geradengleichung angegeben, wobei der Geradenanstieg die Assoziationskonstante wiedergibt.

### Sequenzen

| **CDR Sequenzen** | |
|---|---|
| SEQ ID NO. 1 | NYWLG |
| | |
| SEQ ID NO. 2 | DIYPGGGYTNYNEKFKG |
| SEQ ID NO. 3 | DIYPGGSYTNYNEKFKG |
| | |
| SEQ ID NO. 4 | YDAAGPWFAY |
| SEQ ID NO. 5 | YDAAGPGFAY |
| SEQ ID NO. 6 | YDNHYFDY |
| | |
| SEQ ID NO. 7 | RSSQSIVHSNGNTYLE |
| SEQ ID NO. 8 | RSSQSLLHSNGNTYLH |
| SEQ ID NO. 9 | KSSQSLLHSDGKTYLY |
| | |
| SEQ ID NO. 10 | KVSNRFS |
| SEQ ID NO. 11 | EVSSRFS |
| | |
| SEQ ID NO. 12 | FQGSHVPYT |
| SEQ ID NO. 13 | SQSTHVPYT |
| | |

| **CDR Sequenzen (canonical structure variants)** | |
|---|---|
| SEQ ID NO. 14 | NYWIG |
| SEQ ID NO. 15 | NYWMG |
| SEQ CD NO. 16 | NYWWG |
| SEQ ID NO. 17 | NYWVG |
| | |
| SEQ ID NO. 18 | DIYPGGDYTNYNEKFKG |
| SEQ ID NO. 19 | DIYPGGNYTNYNEKFKG |
| SEQ ID NO. 20 | DIYTGGGYTNYNEKFKG |
| SEQ ID NO. 21 | DIYTGGDYTNYNEKFKG |
| SEQ ID NO. 22 | DIYTGGNYTNYNEKFKG |
| SEQ ID NO. 23 | DIYTGGSYTNYNEKFKG |
| SEQ ID NO. 24 | DIYAGGGYTNYNEKFKG |
| SEQ ID NO. 25 | DIYAGGDYTNYNEKFKG |
| SEQ ID NO. 26 | DIYAGGNYTNYNEKFKG |
| SEQ ID NO. 27 | DIYAGGSYTNYNEKFKG |
| | |
| SEQ ID NO. 28 | RPSQSIVHSNGNTYLE |
| SEQ ID NO. 29 | RSSQSLVHSNGNTYLE |
| SEQ ID NO. 30 | RSSQSIVHSNGNTYFE |
| SEQ ID NO. 31 | RPSQSLVHSNGNTYLE |
| SEQ ID NO. 32 | RPSQSIVHSNGNTYFE |
| SEO ID NO. 33 | RSSQSLVHSNGNTYFE |
| SEQ ID NO. 34 | RPSQSLLHSNGNTYLH |
| SEQ ID NO. 35 | RSSQSILHSNGNTYLH |
| SEQ ID NO. 36 | RSSQSLLHSNGNTYFH |
| SEQ ID NO. 37 | RPSQSILHSNGNTYLH |
| SEQ ID NO. 38 | RPSQSLLHSNGNTYFH |
| SEQ ID NO. 39 | RSSQSILHSNGNTYFH |
| SEQ ID NO. 40 | KPSQSLLHSDGKTYLY |
| SEQ ID NO. 41 | KSSQSILHSDGKTYLY |
| SEQ ID NO. 42 | KSSQSLLHSDGKTYFY |
| SEQ ID NO. 43 | KPSQSILHSDGKTYLY |
| SEQ ID NO. 44 | KPSQSLLHSDGKTYFY |
| SEQ ID NO. 45 | KSSQSILHSDGKTYFY |

### variable schwere Ketten VH

SEQ ID NO. 46
SEQ ID NO. 47
SEQ ID NO. 48
SEQ ID NO. 49
SEQ ID NO. 50
SEQ ID NO. 51
SEQ ID NO. 52
SEQ ID NO. 53
SEQ ID NO. 54
SEQ ID NO. 55
SEQ ID NO. 56
SEQ ID NO. 57
SEQ ID NO. 58
SEQ ID NO. 59
SEQ ID NO. 60
SEQ ID NO: 61
SEQ ID NO. 62
SEQ ID NO. 63
SEQ ID NO. 64
SEQ ID NO. 65
SEQ ID NO. 66
SEQ ID NO. 67
SEQ ID NO. 68
SEQ ID NO. 69
SEQ ID NO. 70
SEQ ID NO. 71
SEQ ID NO. 72
SEQ ID NO. 73
SEQ ID NO. 74
SEQ ID NO. 75
SEQ ID NO. 76
SEQ ID NO. 77
SEQ ID NO. 78
SEQ ID NO. 79

### variable leichte Ketten

SEQ ID NO. 80
SEQ ID NO. 81
SEQ ID NO. 82
SEQ ID NO. 83
SEQ ID NO. 84
SEQ ID NO. 85
SEQ ID NO. 86
SEQ ID NO. 87
SEQ ID NO. 88
SEQ ID NO. 89
SEQ ID NO. 90
SEQ ID NO. 91
SEQ ID NO. 92
SEQ ID NO. 93
SEQ ID NO. 94

### VH/VL Paarungen

| Mausantikörper | |
|---|---|
| Karo1 | SEQ ID NO. 46 |
| | SEQ ID NO. 80 |
| | |
| Karo2 | SEQ ID NO. 47 |
| | SEQ ID NO. 81 |
| | |
| Karo3 | SEQ ID NO. 48 |
| | SEQ ID NO. 80 |
| | |
| Karo4 | SEQ ID NO. 50 |
| | SEQ ID NO. 80 |
| | |
| Karo5 | SEQ ID NO. 53 |
| | SEQ ID NO. 82 |
| | |
| Karo6 | SEQ ID NO. 52 |
| | SEQ ID NO. 63 |
| | |
| Karo7 | SEQ ID NO. 55 |
| | SEQ ID NO. 83 |
| | |
| Karo8 | SEQ ID NO. 54 |
| | SEQ ID NO. 80 |
| | |
| Karo9 | SEQ ID NO. 51 |
| | SEQ ID NO. 83 |
| | |
| Karo10 | SEQ ID NO. 49 |
| | SEQ ID NO. 80 |

| humanisierte Sequenzen | |
|---|---|
| Karo11 | SEQ ID NO. 56 |
| | SEQ ID NO. 90 |
| | |
| Karo12 | SEQ ID NO. 57 |
| | SEQ ID NO. 90 |
| | |
| Karo13 | SEQ ID NO. 57 |
| | SEQ ID NO. 86 |
| | |
| Karo14 | SEQ ID NO. 58 |
| | SEQ ID NO. 87 |
| | |
| Karo15 | SEQ ID NO. 56 |
| | SEQ ID NO. 91 |
| | |
| Karo16 | SEQ ID NO. 59 |
| | SEQ ID NO. 91 |
| | |
| Karo17 | SEQ ID NO. 60 |
| | SEQ ID NO. 87 |
| | |
| Karo18 | SEQ ID NO. 61 |
| | SEQ ID NO. 90 |
| | |
| Karo19 | SEQ ID NO. 56 |
| | SEQ ID NO. 88 |
| | |
| Karo20 | SEQ ID NO. 56 |
| | SEQ ID NO. 85 |
| | |
| Karo21 | SEQ ID NO. 59 |
| | SEQ ID NO. 90 |
| Karo22 | SEQ ID NO. 62 |
| | SEQ ID NO. 90 |
| | |
| Karo23 | SEQ ID NO. 59 |
| | SEQ ID NO. 86 |
| Karo24 | SEQ ID NO. 74 |
| | SEQ ID NO. 92 |
| | |
| Karo25 | SEQ ID NO. 63 |
| | SEQ ID NO. 87 |
| | |
| Karo26 | SEQ ID NO. 74 |
| | SEQ ID NO. 87 |
| | |
| Karo27 | SEQ ID NO. 74 |
| | SEQ ID NO. 89 |
| | |
| Karo28 | SEQ ID NO. 74 |
| | SEQ ID NO. 85 |
| | |
| Karo29 | SEQ ID NO. 64 |
| | SEQ ID NO. 86 |
| | |
| Karo30 | SEQ ID NO. 74 |
| | SEQ ID NO. 86 |
| | |
| Karo31 | SEQ ID NO. 63 |
| | SEQ ID NO. 86 |
| | |
| Karo32 | SEQ ID NO. 65 |
| | SEQ ID NO. 85 |
| Karo33 | SEQ ID NO. 65 |
| | SEQ ID NO. 86 |
| | |
| Karo34 | SEQ ID NO. 66 |
| | SEQ ID NO. 85 |
| | |
| Karo35 | SEQ ID NO. 67 |
| | SEQ ID NO. 87 |
| | |
| Karo36 | SEQ ID NO. 68 |
| | SEQ ID NO. 86 |
| | |
| Karo37 | SEQ ID NO. 72 |
| | SEQ ID NO. 88 |
| | |
| Karo38 | SEQ ID NO. 69 |
| | SEQ ID NO. 90 |
| | |
| Karo39 | SEQ ID NO. 70 |
| | SEQ ID NO. 90 |
| | |
| Karo40 | SEQ ID NO. 69 |
| | SEQ ID NO. 92 |
| Karo41 | SEQ ID NO. 73 |
| | SEQ ID NO. 86 |
| | |
| Karo42 | SEQ ID NO. 69 |
| | SEQ ID NO. 89 |
| | |
| Karo43 | SEQ ID NO. 71 |
| | SEQ ID NO. 92 |
| | |
| Karo44 | SEQ ID NO. 56 |
| | SEQ ID NO. 86 |
| | |
| Karo45 | SEQ ID NO. 65 |
| | SEQ ID NO. 92 |

### verschiedene single chain Fv Formate

SEQ ID NO. 95
SEQ ID NO. 96
SEQ ID NO. 97
SEQ ID NO. 98
SEQ ID NO. 99
SEQ ID NO. 100
SEQ ID NO. 101
SEQ ID NO. 102
SEQ ID NO. 103
SEQ ID NO. 104
SEQ ID NO. 105
SEQ ID NO. 106

### Murine Antikörper

SEQ ID NO. 107
SEQ ID NO. 108
SEQ ID NO. 109
SEQ ID NO. 110

| | |
|---|---|
| mlgM-Karo2 | SEQ ID NO. 109 |
| | SEQ ID NO. 107 |
| mIgM-Karo4 | SEQ ID NO. 110 |
| | SEQ ID NO. 108 |

### Chimäre Antikörper (Maus/Mensch)

SEQ ID NO. 111
SEQ ID NO. 112
SEQ ID NO. 113

### chimäre Antikörper

| | |
|---|---|
| clgG-Karo4 | SEQ ID NO. 111 |
| | SEQ ID NO. 113 |
| clgM-Karo4 | SEQ ID NO. 112 |
| | SEQ ID NO. 113 |

## Patentansprüche

1. Verfahren zur Identifizierung und/oder Isolierung und/oder Gewinnung eines Core-1 tragenden Moleküls, einer Core-1 tragenden Zelle, eines Core-1 tragenden Virus, eines Core-1 tragenden Bakteriums, oder von Core-1 tragenden Teilen von Zellen durch Bindung an ein Core-1 spezifisches Erkennungsmolekül, wobei das Erkennungsmolekül eine Aminosäuresequenz umfasst, die
(i) die Aminosäuresequenz SEQ ID NO. 1 oder eine zu SEQ ID NO. 1 mindestens 80% homologe Aminosäuresequenz, und
(ii) die Aminosäuresequenz SEQ ID NO. 2 oder 3 oder eine zu SEQ ID NO. 2 oder 3 mindestens 80% homologe Aminosäuresequenz, und
(iii) die Aminosäuresequenz SEQ ID NO. 4, 5 oder 6 oder eine zu SEQ ID NO. 4, 5 oder 6 mindestens 80% homologe Aminosäuresequenz
enthält und das Antigen Core-1 spezifisch bindet.

2. Verfahren gemäß Anspruch 1, wobei ein Core-1 tragendes Bakterium identifiziert, isoliert und/oder gewonnen wird.

3. Verfahren gemäß Anspruch 1, wobei das Core-1 tragende Molekül ausgewählt ist aus der Gruppe bestehend aus Glykoproteinen, Glykopeptiden und Glykolipiden, und der Core-1 tragende Zellteil ausgewählt ist aus der Gruppe bestehend aus Exosomen und Liposomen.

4. Verfahren gemäß einem der Ansprüche 1 bis 3, wobei das Core-1 tragende Molekül, die Core-1 tragende Zelle, der Core-1 tragende Virus, das Core-1 tragende Bakterium, oder der Core-1 tragende Zellteil auch Core-2 Strukturen trägt.

5. Verfahren gemäß einem der Ansprüche 1 bis 4, wobei das Erkennungsmolekül weiterhin eine Aminosäuresequenz umfasst, die
(i) die Aminosäuresequenz SEQ ID NO. 7 oder 8 oder 9 oder eine zu SEQ ID NO. 7, 8 oder 9 mindestens 80% homologe Aminosäuresequenz, und
(ii) die Aminosäuresequenz SEQ ID NO. 10 oder 11 oder eine zu SEQ ID NO. 10 oder 11 mindestens 80% homologe Aminosäuresequenz, und
(iii) die Aminosäuresequenz SEQ ID NO. 12 oder 13 oder eine zu SEQ ID NO. 12 oder 13 mindestens 80% homologe Aminosäuresequenz,
enthält und das Antigen Core-1 spezifisch bindet.

6. Verfahren gemäß einem der Ansprüche 1 bis 5, wobei die Sequenz SEQ ID NO. 1 durch eine äquivalente kanonische Strukturvariante gemäß SEQ ID NO. 14 bis 17 ersetzt ist und/oder mindestens eine Sequenz der Sequenzen SEQ ID NO. 2 oder 3 durch eine äquivalente kanonische Strukturvariante gemäß SEQ ID NO. 18 bis 27 ersetzt ist und/oder mindestens eine Sequenz gemäß SEQ ID NO. 7 bis 9 durch eine äquivalente kanonische Strukturvariante gemäß SEQ ID NO. 28 bis 45 ersetzt ist und das Erkennungsmolekül das Antigen Core-1 spezifisch bindet.

7. Verfahren gemäß einem der Ansprüche 1 bis 6, wobei das Erkennungsmolekül weiterhin Gerüstsequenzen, die die Aminosäuresequenzen voneinander trennen, einschließen und/oder flankieren, wobei die Gerüstsequenzen vorzugsweise ausgewählt sind aus der Gruppe umfassend die Immunglobulin-Superfamilie, Protease-Inhibitoren, Lektine, Helix-Bündel-Proteine und/oder Lipocaline, wobei die Gerüstsequenzen bevorzugt Antikörpergerüstsequenzen sind, wobei die Antikörpergerüstsequenzen für das Erkennungsmolekül nach Anspruch 1 vorzugsweise Sequenzen der variablen schweren Kette VH und die Antikörpergerüstsequenzen für die zusätzlichen Sequenzen des Erkennungsmoleküls nach Anspruch 5 vorzugsweise Sequenzen der variablen leichten Kette VL sind.

8. Verfahren gemäß einem der Ansprüche 1 bis 7, wobei das Erkennungsmolekül eine Kombination der Sequenzen SEQ ID NO. 46 und 80, oder SEQ ID NO. 47 und 81, oder SEQ ID NO. 48 und 80, oder SEQ ID NO. 50 und 80, oder SEQ ID NO. 53 und 82, oder SEQ ID NO. 52 und 83, oder SEQ ID NO. 55 und 83, oder SEQ ID NO. 54 und 80, oder SEQ ID NO. 51 und 83, oder SEQ ID NO. 49 und 80, oder SEQ ID NO. 56 und 90, oder SEQ ID NO. 57 und 90, oder SEQ ID NO. 57 und 86, oder SEQ ID NO. 58 und 87, oder SEQ ID NO. 56 und 91, oder SEQ ID NO. 59 und 91, oder SEQ ID NO. 60 und 87, oder SEQ ID NO. 61 und 90, oder SEQ ID NO. 56 und 88, oder SEQ ID NO. 56 und 85, oder SEQ ID NO. 59 und 90, oder SEQ ID NO. 62 und 90, oder SEQ ID NO. 59 und 86, oder SEQ ID NO. 74 und 92, oder SEQ ID NO. 63 und 87, oder SEQ ID NO. 74 und 87, oder SEQ ID NO. 74 und 89, oder SEQ ID NO. 74 und 85, oder SEQ ID NO. 64 und 86, oder SEQ ID NO. 74 und 86, oder SEQ ID NO. 63 und 86, oder SEQ ID NO. 65 und 85, oder SEQ ID NO. 65 und 86, oder SEQ ID NO. 66 und 85, oder SEQ ID NO. 67 und 87, oder SEQ ID NO. 68 und 86, oder SEQ ID NO. 72 und 88, oder SEQ ID NO. 69 und 90, oder SEQ ID NO. 70 und 90, oder SEQ ID NO. 69 und 92, oder SEQ ID NO. 73 und 86, oder SEQ ID NO. 69 und 89, oder SEQ ID NO. 71 und 92, oder SEQ ID NO. 56 und 86, oder SEQ ID NO. 65 und 92 umfasst.

9. Verfahren gemäß einem der Ansprüche 1 bis 8, wobei das Erkennungsmolekül eine variable schwere Kette VH und eine variable leichte Kette VL umfasst, wobei die variable schwere Kette VH und die variable leichte Kette VL auf verschiedenen Polypeptidketten liegen oder wobei die variable schwere Kette VH und die variable leichte Kette VL miteinander in einem Fusionsprotein direkt verbunden sind.

10. Verfahren gemäß einem der Ansprüche 1 bis 9, wobei das Erkennungsmolekül zusätzliche His-Tag, myc-Tag, Lysin-reiche Sequenzen und/oder Multimerisierungssequenzen umfasst.

11. Verfahren gemäß einem der Ansprüche 1 bis 10, wobei das Erkennungsmolekül von einem Immunglobulin abgeleitet ist.

12. Verfahren gemäß einem der Ansprüche 1 bis 11, wobei das Erkennungsmolekül durch ein oder mehrere der folgenden Merkmale gekennzeichnet ist:
(i) es ist ein single chain Antikörperfragment, ein Multibody, ein Fab-Fragment, ein Fusionsprotein aus einem Antikörperfragment mit Peptiden oder Proteinen und/oder ein Immunglobulin der Isotypen IgG, IgM, IgA, IgE, IgD und/oder deren Subklassen;
(ii) es ist ein muriner, chimärisierter, humanisierter, humaner, partiell humaner Antikörper oder Antikörperfragment;
(iii) es ist ein IgM ohne J Kette.

13. Verfahren gemäß einem der Ansprüche 1 bis 12, wobei das Erkennungsmolekül eine Sequenz gemäß SEQ ID NO. 95 bis 113 umfasst.

## Claims

1. Method for identification and/or isolation and/or obtaining a molecule carrying Core-1, a cell carrying Core-1, a virus carrying Core-1, a bacterium carrying Core-1, or cell parts carrying Core-1 by binding to a Core-1 specific recognition molecule, wherein the recognition molecule comprises an amino acid sequence which contains
(i) the amino acid sequence of SEQ ID NO: 1, or an amino acid sequence which is at least 80% homologous to SEQ ID NO: 1; and
(ii) the amino acid sequence of SEQ ID NO: 2 or 3, or an amino acid sequence which is at least 80% homologous to SEQ ID NO: 2 or 3; and
(iii) the amino acid sequence of SEQ ID NO: 4, 5 or 6, or an amino acid sequence which is at least 80% homologous to SEQ ID NO: 4, 5 or 6; and
which specifically binds the antigen Core-1.

2. The method according to claim 1, wherein a bacterium carrying Core-1 is identified, isolated and/or obtained.

3. The method according to claim 1, wherein the molecule carrying Core-1 is selected from the group consisting of glycoproteins, glycopeptides and glycolipids, and wherein the cell part carrying Core-1 is selected from the group consisting of exosomes and liposomes.

4. The method according to any one of claims 1 to 3, wherein the molecule carrying Core-1, the cell carrying Core-1, the virus carrying Core-1, the bacterium carrying Core-1, or the cell part carrying Core-1 also carries Core-2 structures.

5. The method according to any one of claims 1 to 4, wherein the recognition molecule further comprises an amino acid sequence which contains
(i) the amino acid sequence of SEQ ID NO: 7 or 8 or 9, or an amino acid sequence which is at least 80% homologous to SEQ ID NO: 7 or 8 or 9; and
(ii) the amino acid sequence of SEQ ID NO: 10 or 11, or an amino acid sequence which is at least 80% homologous to SEQ ID NO: 10 or 11; and
(iii) the amino acid sequence of SEQ ID NO: 12 or 13, or an amino acid sequence which is at least 80% homologous to SEQ ID NO: 12 or 13; and
which specifically binds the antigen Core-1.

6. The method according to any one of claims 1 to 5, wherein the sequence of SEQ ID NO: 1 is substituted by an equivalent canonical structure variant of SEQ ID NOs: 14 to 17 and/or at least one sequence of sequences SEQ ID NOs: 2 or 3 is substituted by an equivalent canonical structure variant of SEQ ID NOs: 18 to 27 and/or at least one sequence of SEQ ID NOs: 7 to 9 is substituted by an equivalent canonical structure variant of SEQ ID NOs: 28 to 45, and the recognition molecule specifically binds to the antigen Core-1.

7. The method according to any one of claims 1 to 6, wherein the recognition molecule further comprises framework sequences, which separate the amino acid sequences from each other, enclose them and/or flank them, wherein the framework sequences preferably are selected from the group comprising the immunoglobulin superfamily, protease inhibitors, lectins, helix bundle proteins, and/or lipocalines, wherein the framework sequences preferably are antibody framework sequences, wherein the antibody framework sequences for the recognition molecule according to claim 1 preferably are sequences of the variable heavy chain VH and the antibody framework sequences for the additional sequences of the recognition molecule according to claim 5 preferably are sequences of the variable light chain VL.

8. The method according to any one of claims 1 to 7, wherein the recognition molecule comprises a combination of the sequences SEQ ID NO. 46 and 80, or SEQ ID NO. 47 and 81, or SEQ ID NO. 48 and 80, or SEQ ID NO. 50 and 80, or SEQ ID NO. 53 and 82, or SEQ ID NO. 52 and 83, or SEQ ID NO. 55 and 83, or SEQ ID NO. 54 and 80, or SEQ ID NO. 51 and 83, or SEQ ID NO. 49 and 80, or SEQ ID NO. 56 and 90, or SEQ ID NO. 57 and 90, or SEQ ID NO. 57 and 86, or SEQ ID NO. 58 and 87, or SEQ ID NO. 56 and 91, or SEQ ID NO. 59 and 91, or SEQ ID NO. 60 and 87, or SEQ ID NO. 61 and 90, or SEQ ID NO. 56 and 88, or SEQ ID NO. 56 and 85, or SEQ ID NO. 59 and 90, or SEQ ID NO. 62 and 90, or SEQ ID NO. 59 and 86, or SEQ ID NO. 74 and 92, or SEQ ID NO. 63 and 87, or SEQ ID NO. 74 and 87, or SEQ ID NO. 74 and 89, or SEQ ID NO. 74 and 85, or SEQ ID NO. 64 and 86, or SEQ ID NO. 74 and 86, or SEQ ID NO. 63 and 86, or SEQ ID NO. 65 and 85, or SEQ ID NO. 65 and 86, or SEQ ID NO. 66 and 85, or SEQ ID NO. 67 and 87, or SEQ ID NO. 68 and 86, or SEQ ID NO. 72 and 88, or SEQ ID NO. 69 and 90, or SEQ ID NO. 70 and 90, or SEQ ID NO. 69 and 92, or SEQ ID NO. 73 and 86, or SEQ ID NO. 69 and 89, or SEQ ID NO. 71 and 92, or SEQ ID NO. 56 and 86, or SEQ ID NO. 65 and 92.

9. The method according to any one of claims 1 to 8, wherein the recognition molecule comprises a variable heavy chain VH and a variable light chain VL, wherein the variable heavy chain VH and the variable light chain VL are on different polypeptide chains or wherein the variable heavy chain VH and the variable light chain VL are directly linked with each other in a fusion protein.

10. The method according to any one of claims 1 to 9, wherein the recognition molecule further comprises His-tag, myc-tag, lysine-rich sequences and/or multimerization sequences.

11. The method according to any one of claims 1 to 10, wherein the recognition molecule is derived from an immunoglobulin.

12. The method according to any one of claims 1 to 11, wherein the recognition molecule is **characterized by** one or more of the following features:.
(i) it is a single-chain antibody fragment, a multibody, a Fab fragment, a fusion protein of an antibody fragment with peptides or proteins and/or an immunoglobulin of the IgG, IgM, IgA, IgE, IgD isotypes and/or subclasses thereof;
(ii) it is a murine, chimeric, humanized, human, partially human antibody or antibody fragment;
(iii) it is an IgM without J chain.

13. The method according to any one of claims 1 to 12, wherein the recognition molecule comprises a sequence according to SEQ ID NO. 95 to 113.

## Revendications

1. Procédé d'identification et/ou d'isolation et/ou d'obtention d'une molécule porteuse de Core-1, d'une cellule porteuse de Core-1, d'un virus porteur de Core-1, d'une bactérie porteuse de Core-1 ou de parties de cellules porteuses de Core-1 par fixation à une molécule de reconnaissance spécifique à Core-1, dans lequel la molécule de reconnaissance comprend une séquence d'acides aminés qui contient
(i) la séquence d'acides aminés SEQ ID N° 1 ou une séquence d'acides aminés homologue à SEQ ID N° 1 à au moins 80 %, et
(ii) la séquence d'acides aminés SEQ ID N° 2 ou 3 ou une séquence d'acides aminés homologue à SEQ ID N°2 ou 3 à au moins 80 %, et
(iii) la séquence d'acides aminés SEQ ID N° 4, 5 ou 6 ou une séquence d'acides aminés homologue à SEQ ID N°4, 5 ou 6 à au moins 80 %,
et fixe spécifiquement l'antigène Core-1.

2. Procédé selon la revendication 1, dans lequel une bactérie porteuse de Core-1 est identifiée, isolée et/ou obtenue.

3. Procédé selon la revendication 1, dans lequel la molécule porteuse de Core-1 est sélectionnée parmi le groupe constitué de glycoprotéines, glycopeptides et glycolipides, et la partie de cellule porteuse de Core-1 est sélectionnée parmi le groupe constitué d'exosomes et de liposomes.

4. Procédé selon une des revendications 1 à 3, dans lequel la molécule porteuse de Core-1, la cellule porteuse de Core-1, le virus porteur de Core-1, la bactérie porteuse de Core-1 ou la partie de cellule porteuse de Core-1 porte également des structures Core-2.

5. Procédé selon une des revendications 1 à 4, dans lequel la molécule de reconnaissance comprend en outre une séquence d'acides aminés qui contient
(i) la séquence d'acides aminés SEQ ID N° 7 ou 8 ou 9 ou une séquence d'acides aminés homologue à SEQ ID N°7, 8 ou 9 à au moins 80 %, et
(ii) la séquence d'acides aminés SEQ ID N° 10 ou 11 ou une séquence d'acides aminés homologue à SEQ ID N° 10 ou 11 à au moins 80 %, et
(iii) la séquence d'acides aminés SEQ ID N° 12 ou 13 ou une séquence d'acides aminés homologue à SEQ ID N° 12 ou 13 à au moins 80 %,
et fixe spécifiquement l'antigène Core-1.

6. Procédé selon une des revendications 1 à 5, dans lequel la séquence SEQ ID N° 1 est remplacée par une variante structurelle canonique équivalente selon SEQ ID N° 14 à 17 et/ou au moins une séquence des séquences SEQ ID N° 2 ou 3 est remplacée par une variante structurelle canonique équivalente selon SEQ ID N° 18 à 27 et/ou au moins une séquence selon SEQ ID N° 7 à 9 est remplacée par une variante structurelle canonique équivalente selon SEQ ID N° 28 à 45 et la molécule de reconnaissance fixe spécifiquement l'antigène Core-1.

7. Procédé selon une des revendications 1 à 6, dans lequel la molécule de reconnaissance en outre des séquences de squelette qui séparent les unes des autres, incluent et/ou flanquent les séquences d'acides aminés, dans lequel les séquences de squelette sont de préférence sélectionnées parmi le groupe comprenant la superfamille de l'immunoglobuline, des inhibiteurs de protéase, des lectines, des protéines à faisceau d'hélices et/ou des lipocalines, dans lequel les séquences de squelette sont de préférence des séquences de squelette d'anticorps, dans lequel les séquences de squelette d'anticorps pour la molécule de reconnaissance selon la revendication 1 sont de préférence des séquences de la chaîne lourde variable VH et les séquences de squelette d'anticorps pour les séquences supplémentaires de la molécule de reconnaissance selon la revendication 5 sont de préférence des séquences de la chaîne légère variable VL.

8. Procédé selon une des revendications 1 à 7, dans lequel la molécule de reconnaissance comprend une combinaison des séquences SEQ ID N° 46 et 80, ou SEQ ID N° 47 et 81, ou SEQ ID N° 48 et 80, ou SEQ ID N° 50 et 80, ou SEQ ID N° 53 et 82, ou SEQ ID N° 52 et 83, ou SEQ ID N° 55 et 83, ou SEQ ID N°54 et 80, ou SEQ ID N°51 et 83, ou SEQ ID N°49 et 80, ou SEQ ID N°56 et 90, ou SEQ ID N° 57 et 90, ou SEQ ID N° 57 et 86, ou SEQ ID N° 58 et 87, ou SEQ ID N° 56 et 91, ou SEQ ID N° 59 et 91, ou SEQ ID N°60 et 87, ou SEQ ID N°61 et 90, ou SEQ ID N°56 et 88, ou SEQ ID N° 56 et 85, ou SEQ ID N° 59 et 90, ou SEQ ID N° 62 et 90, ou SEQ ID N° 59 et 86, ou SEQ ID N° 74 et 92, ou SEQ ID N° 63 et 87, ou SEQ ID N° 74 et 87, ou SEQ ID N°74 et 89, ou SEQ ID N°74 et 85, ou SEQ ID N°64 et 86, ou SEQ ID N°74 et 86, ou SEQ ID N° 63 et 86, ou SEQ ID N° 65 et 85, ou SEQ ID N° 65 et 86, ou SEQ ID N° 66 et 85, ou SEQ ID N° 67 et 87, ou SEQ ID N° 68 et 86, ou SEQ ID N° 72 et 88, ou SEQ ID N° 69 et 90, ou SEQ ID N° 70 et 90, ou SEQ ID N° 69 et 92, ou SEQ ID N° 73 et 86, ou SEQ ID N°69 et 89, ou SEQ ID N° 71 et 92, ou SEQ ID N°56 et 86, ou SEQ ID N°65 et 92.

9. Procédé selon une des revendications 1 à 8, dans lequel la molécule de reconnaissance comprend une chaîne lourde variable VH et une chaîne légère variable VL, dans lequel la chaîne lourde variable VH et la chaîne légère variable VL se situent sur différentes chaînes polypeptidiques ou dans lequel la chaîne lourde variable VH et la chaîne légère variable VL sont directement reliées ensemble dans une protéine de fusion.

10. Procédé selon une des revendications 1 à 9, dans lequel la molécule de reconnaissance comprend des séquences riches en His-Tag, myc-Tag, lysine supplémentaires et/ou des séquences de multimérisation.

11. Procédé selon une des revendications 1 à 10, dans lequel la molécule de reconnaissance est dérivée d'une immunoglobuline.

12. Procédé selon une des revendications 1 à 11, dans lequel la molécule de reconnaissance est **caractérisée par** une ou plusieurs des caractéristiques suivantes :
(i) elle est un fragment d'anticorps à chaîne unique, un multicorps, un fragment Fab, une protéine de fusion provenant d'un fragment d'anticorps avec des peptides ou protéines et/ou une immunoglobuline des isotypes IgG, IgM, IgA, IgE, IgD et/ou leurs sous-classes ;
(ii) elle est un anticorps ou fragment d'anticorps murin, chimérisé, humanisé, humain, partiellement humain ;
(iii) elle est une IgM sans chaîne J.

13. Procédé selon une des revendications 1 à 12, dans lequel la molécule de reconnaissance comprend une séquence selon SEQ ID N°95 à 113.
